# EUROPEAN PATENT APPLICATION

(11) **EP 4 817 604 A1**
(43) Date of publication of application: **07.10.2026**
(21) Application number: 25222012.4
(22) Date of filing: 09.12.2025
(51) Int. Cl.: A61K 31/08, A61P 3/04, A61P 3/10, A61P 9/00, A61P 11/06, A61P 25/00, A61P 35/00

(54) **MITOCHONDRIAL BIOSYNTHESIS PROMOTER CONTAINING 1,1-DIETHOXYETHANE AS AN ACTIVE INGREDIENT AND ITS USE**

(30) Priority: 02.04.2025 KR 20250042853; 13.06.2025 KR 20250077896; 09.09.2025 KR 20250128126
(71) Applicant: Lux Anima Co., Ltd., Jeollanam-do 58141 (KR)
(72) Inventor: LEE, Seung-Rock, Gwangju (KR); NGUYEN, Huu Thang, Jeollanam-do (KR); CHOI, Jin Myung, Jeollanam-do (KR); LEE, Geunhaeng, Gwangju (KR); YOON, Hyun Joong, Gwangju (KR); TRINH, Hoang Vu, Jeollanam-do (KR); SAH, Dhiraj Kumar, Jeollanam-do (KR); KIM, Min-Kyu, Jeonbuk-do (KR)
(74) Representative: Vossius & Partner Patentanwälte Rechtsanwälte mbB

(57) **Abstract**

The present invention relates to a mitochondrial biosynthesis promoter comprising 1,1-diethoxyethane (1,1-DEE) as an active ingredient and its uses. More specifically, the present invention relates to a mitochondrial biosynthesis promoter comprising 1,1-diethoxyethane as an active ingredient and its uses, for example, as a composition for preventing, treating, or improving diseases requiring mitochondrial biosynthesis, including a pharmaceutical composition, a cosmetic composition, a food composition, or a feed composition.

## Description

### [Cross-Reference to Related Applications]

This application claims priority to Korean Patent Application Nos. 10-2025-0042853 filed on April 2, 2025, 10-2025-0077896 filed on June 13, 2025, and 10-2025-0128126 filed on September 9, 2025, the entire contents of each of which are incorporated herein by reference.

### [Technical Field]

The present invention relates to a mitochondrial biosynthesis promoter comprising 1,1-diethoxyethane (1,1-DEE) as an active ingredient, and uses thereof. More specifically, the present invention relates to a mitochondrial biosynthesis promoter comprising 1,1-diethoxyethane as an active ingredient and uses thereof, for example, as a composition for preventing, treating, or improving a disease requiring the promotion of mitochondrial biosynthesis, including a pharmaceutical composition, a cosmetic composition, a food composition, or a feed composition.

### [Background Art]

5'-Adenosine monophosphate (AMP)-activated protein kinase (AMPK) is an evolutionarily conserved enzyme belonging to the serine (Ser)/threonine (Thr) kinase family that is responsible for maintaining cellular energy homeostasis and plays a crucial role in regulating energy balance [1,2]. AMPK consists of a catalytic α-subunit, a regulatory β-subunit, and a non-catalytic γ-subunit, each of which regulates various physiological functions [3]. The α-subunit activates AMPK through phosphorylation at the Thr172 residue, the β-subunit promotes the binding of AMPK to glycogen, and the γ-subunit binds AMP and ADP, thereby stimulating AMPK phosphorylation [4]. Activation of AMPK promotes catabolic metabolism while inhibiting anabolic metabolism, generating ATP and improving energy balance. Upstream kinases such as liver kinase B1 (LKB1), Ca²⁺ /calmodulin-dependent protein kinase kinase-2 (CAMKK2), and transforming growth factor-β-activated kinase (TAK) play key roles in AMPK activation [5-7]. Drugs such as metformin, 5-aminoimidazole-4-carboxamide ribonucleotide (AICAR), and resveratrol activate AMPK by modulating upstream kinases [8-10]. Energy conditions such as exercise or nutrient deprivation may also activate AMPK. AMPK activation has potential implications for the prevention of obesity, cardiovascular disease (CVD), and cancer [11], and plays a particularly important preventive role in cardiovascular diseases [17-22].

AMPK contributes significantly to energy balance by regulating downstream effectors of catabolic pathways such as fatty acid synthesis and glycolysis. Acetyl-CoA carboxylase (ACC) plays an essential role in fatty acid biosynthesis, and phosphorylation of Ser79 (ACC1) or Ser212 (ACC2) by AMPK inhibits ACC enzymatic activity, thereby enhancing fatty acid oxidation [1]. Fatty acid oxidation in the heart constitutes an important part of energy production. The 5'-adenosine monophosphate (AMP)-activated protein kinase (AMPK)-acetyl-CoA carboxylase (ACC) signaling pathway regulates various physiological processes, including platelet phospholipid metabolism and thrombus formation, cardiac hypertrophy and contractility, and the regulation of ischemia [24-26]. In addition, glycolysis is regulated by 6-phosphofructo-2-kinase/fructose-2,6-bisphosphatase-2 (PFKFB2), which is involved in the formation and degradation of fructose-2,6-bisphosphate (Fru-2,6-P2), thereby modulating the enzymatic activity of phosphofructokinase-1 (PFK1). Phosphorylation of Ser466 and Ser483 of PFKFB2 promotes glycolysis, which is regulated by protein kinase B (Akt) and AMPK. Activation of PFKFB2 plays an important role in cardiac remodeling, suppression of ferroptosis during ischemia/reperfusion (I/R) injury, and improvement of cardiac function under hypoxic conditions [27-29].

Mitochondrial biosynthesis is an important process that allows cells to adapt to energy demands, and 5'-adenosine monophosphate (AMP)-activated protein kinase (AMPK) can promote this process. Deficiency of AMPK may lead to mitochondrial dysfunction, and one of the major downstream regulators of AMPK, peroxisome proliferator-activated receptor gamma coactivator 1-alpha (PGC-1α), plays a critical role in mitochondrial biosynthesis [2]. AMPK activates PGC-1α by stimulating the transcription factor EB and through direct interaction, and promotes mitochondrial biosynthesis by phosphorylating PGC-1α at Thr177 and Ser538 [32,33]. This process makes an important contribution to cardioprotection [34,35].

### Delay of Aging or Extension of Lifespan

All living organisms possess a limited lifespan and naturally undergo a sequence of processes over time-birth, growth, aging, and ultimately death. Aging is one of the complex biological pathways observed in all living organisms. Extensive research has been conducted to elucidate the causes and mechanisms of aging, which are generally explained by two major theories: the "aging clock" theory and the "cellular senescence" theory. The aging clock theory explains aging in terms of the loss of telomere replication at the ends of DNA strands, proposing that the shortening of telomeres-repetitive sequences found at both ends of chromosomes-can lead to cellular and organismal aging. The cellular senescence theory attributes aging primarily to the accumulation of intracellular damage caused by oxidative stress and mitochondrial dysfunction. Among these, the mitochondrial decline theory emphasizes the critical role of mitochondrial function in maintaining healthy cellular metabolism and bioenergetics. Age-related mitochondrial dysfunction includes decreased ATP production, increased reactive oxygen species (ROS) generation, reduced mitochondrial number, and altered mitochondrial permeability.

As aging progresses, the activity of pathways related to lipid metabolism is inhibited. Specifically, lipid degradation decreases, and the uptake of low-density lipoprotein (LDL) in cells is reduced, leading to an increase in the concentration of LDL in the blood, which can contribute to the development of cardiovascular diseases. In addition, aging leads to a decline in cognitive and motor functions and increases the incidence of neurodegenerative diseases such as Alzheimer's disease and Parkinson's disease, which lower the overall quality of life.

In addition to studies focusing on understanding the aging process, numerous studies have been conducted to delay aging and extend lifespan. To date, the most successful approach has been dietary restriction (DR). The effects of dietary restriction were first reported in rats, and lifespan-extending phenotypes resulting from dietary restriction have subsequently been observed in yeast, nematodes, fruit flies, and mice. However, the beneficial effects of such dietary restriction are observed only when the restriction is continuously maintained, making it difficult to apply this approach universally across all living organisms.

To date, extensive research and efforts have been devoted to achieving the dream of longevity and eternal youth; however, the mechanisms underlying aging have not yet been fully elucidated.

### Cardiovascular Diseases

Proper regulation of heart rate is essential for maintaining life, and conditions such as bradycardia (abnormally slow heart rate) and bradyarrhythmia (slow and irregular heart rhythm) can lead to serious health problems. A decrease in heart rate may cause dizziness, fatigue, and syncope, and in severe cases, it can lead to heart failure or cardiac arrest. Accordingly, there is a continuous need for the development of therapeutic agents capable of effectively increasing heart rate.

To date, therapeutic agents developed to increase heart rate primarily act by stimulating the sympathetic nervous system. Representative drugs include beta-adrenergic agonists, antimuscarinic agents, and specific ion channel modulators.

The beta-adrenergic agonist isoproterenol non-selectively activates both β₁- and β₂-adrenergic receptors, thereby increasing heart rate. However, isoproterenol affects not only heart rate but also myocardial contractility and blood pressure, and long-term use may cause side effects such as hypertension, arrhythmia, and increased myocardial oxygen demand.

Antimuscarinic agents (e.g., atropine) increase heart rate by suppressing vagal nerve activity and thereby reducing the influence of the parasympathetic nervous system. However, these drugs may cause side effects such as dry mouth, blurred vision, and urinary retention, and their ability to regulate heart rate is relatively limited

Some ion channel modulators increase heart rate by regulating the flow of calcium or sodium ions within cardiomyocytes. However, since these drugs directly affect the cell membrane potential, precise dose control is required, and there is a potential risk of inducing arrhythmia.

Therefore, there is a continuing need for the discovery of new substances that can increase heart rate rapidly and effectively while minimizing the side effects associated with existing drugs.

### Cancer

Neuroblastoma originates from the neural crest and typically manifests as a malignant tumor of the sympathetic ganglia and adrenal medulla. More than one-third of all neuroblastomas are detected during infancy (under 1 year of age), and approximately 75% are diagnosed before the age of 5. This disease is one of the most common and fatal pediatric cancers, characterized by its highly aggressive nature and poor prognosis in advanced stages. Despite extensive efforts to improve outcomes-including intensive multi-modal therapy, advances in immunotherapy, and the introduction of novel targeted agents into clinical trials-the prognosis for high-risk patients remains unfavorable, with a median 5-year overall survival rate of only about 50-70%. Recent anticancer strategies have focused primarily on mechanisms that induce apoptosis (programmed cell death). However, defects in apoptotic regulatory pathways not only confer growth advantages to tumor cells but also represent a major cause of therapeutic resistance. Indeed, drug resistance remains one of the greatest challenges in the treatment and control of neuroblastoma.

To date, research on neuroblastoma has primarily focused on multidrug resistance (MDR) proteins expressed in neuroblastoma cells and anti-apoptotic mechanisms. However, studies investigating cellular signaling pathways associated with therapeutic resistance have been relatively limited. In fact, one of the key hallmarks of cancer, metabolic reprogramming, is regulated through interactions among various signaling proteins and kinases, and a deeper understanding of the synergistic relationships among these molecules may contribute to the identification of promising therapeutic targets. In particular, AMP-activated protein kinase (AMPK), which plays a central role in regulating metabolism and energy homeostasis in both normal and cancer cells, has attracted attention as a key component of this signaling axis. Numerous previous studies have reported that the AMPK signaling pathway performs a dual role in maintaining homeostasis within cancer cells. Accordingly, therapeutic strategies that specifically target tumor cells while modulating AMPK and related signaling systems are considered promising approaches for suppressing the development of therapeutic resistance and improving patient prognosis.

### Stroke

Stroke is the third leading cause of death worldwide. Survivors of stroke often suffer from disabilities such as motor impairments and cognitive deficits, which significantly interfere with daily activities and reduce quality of life. Moreover, stroke is an independent risk factor for all-cause dementia. From 1990 to 2021, the global burden of stroke has increased substantially - with the incidence rate rising by 70%, stroke-related mortality by 44%, and disability-adjusted life years (DALYs) lost by 32%. Despite advances in medicine, the WSO/Lancet Neurology Commission on Stroke 2 has projected that stroke-related deaths will increase by 50% between 2020 and 2050, representing a tremendous global health burden. Therefore, stroke remains a major global health issue, both in terms of mortality and disability. The discovery of effective substances for stroke prevention, treatment, and post-stroke recovery is of critical importance.

### Dementia

Dementia refers to a group of neurodegenerative disorders that impair learning and memory abilities, thereby interfering with daily life. According to the World Health Organization (WHO), dementia currently ranks as the seventh leading cause of death worldwide and is recognized as one of the primary causes of disability and dependency among the elderly. Each year, approximately 10 million new cases of dementia are diagnosed, making it a major global health issue that affects millions of lives and imposes an enormous social and economic burden. Various risk factors such as advanced age, hypertension, diabetes, obesity, smoking, and excessive alcohol consumption are known to be significantly associated with the onset of dementia. Meanwhile, the impact of low to moderate alcohol consumption on dementia risk remains unclear. Some studies have suggested that certain levels of alcohol intake may exert potential protective effects against dementia; however, further detailed research is required to clarify this relationship.

### Prior Art References

[Non-Patent Document 1] Steinberg, G.R. and D.G. Hardie, New insights into activation and function of the AMPK. Nat Rev Mol Cell Biol, 2023. 24(4): p. 255-272.
[Non-Patent Document 2] Herzig, S. and R.J. Shaw, AMPK: guardian of metabolism and mitochondrial homeostasis. Nat Rev Mol Cell Biol, 2018. 19(2): p. 121-135.
[Non-Patent Document 3] Davies, S.P., et al., Purification of the AMP-activated protein kinase on ATP-gamma-sepharose and analysis of its subunit structure. Eur J Biochem, 1994. 223(2): p. 351-7.
[Non-Patent Document 4] Hardie, D.G., F.A. Ross, and S.A. Hawley, AMPK: a nutrient and energy sensor that maintains energy homeostasis. Nat Rev Mol Cell Biol, 2012. 13(4): p. 251-62.
[Non-Patent Document 5] Woods, A., et al., LKB1 is the upstream kinase in the AMP-activated protein kinase cascade. Curr Biol, 2003. 13(22): p. 2004-8.
[Non-Patent Document 6] Woods, A., et al., Ca2+/calmodulin-dependent protein kinase kinase-beta acts upstream of AMP-activated protein kinase in mammalian cells. Cell Metab, 2005. 2(1): p. 21-33.
[Non-Patent Document 7] Xie, M., et al., A pivotal role for endogenous TGF-beta-activated kinase-1 in the LKB1/AMP-activated protein kinase energy-sensor pathway. Proc Natl Acad Sci U S A, 2006. 103(46): p. 17378-83.
[Non-Patent Document 8] Shaw, R.J., et al., The kinase LKB1 mediates glucose homeostasis in liver and therapeutic effects of metformin. Science, 2005. 310(5754): p. 1642-6.
[Non-Patent Document 9] Chan, A.Y., et al., Resveratrol inhibits cardiac hypertrophy via AMP-activated protein kinase and Akt. J Biol Chem, 2008. 283(35): p. 24194-201.
[Non-Patent Document 10] Corton, J.M., et al., 5-aminoimidazole-4-carboxamide ribonucleoside. A specific method for activating AMP-activated protein kinase in intact cells? Eur J Biochem, 1995. 229(2): p. 558-65.
[Non-Patent Document 11] Jeon, S.M., Regulation and function of AMPK in physiology and diseases. Exp Mol Med, 2016. 48(7): p. e245.
[Non-Patent Document 12] Marcq, P. and P. Schieberle, Characterization of the Key Aroma Compounds in a Commercial Fino and a Commercial Pedro Ximénez Sherry Wine by Application of the Sensomics Approach. J Agric Food Chem, 2021. 69(17): p. 5125-5133.
[Non-Patent Document 13] Moreno, J., et al., Use of a flor velum yeast for modulating colour, ethanol and major aroma compound contents in red wine. Food Chem, 2016. 213: p. 90-97.
[Non-Patent Document 14] Tan, C., et al., The Effects of Ultrasonic and Gamma Irradiation on the Flavor of Potato Wines Investigated by Sensory Omics. Foods, 2023. 12(15).
[Non-Patent Document 15] Dunkel, A., et al., Nature's chemical signatures in human olfaction: a foodborne perspective for future biotechnology. Angew Chem Int Ed Engl, 2014. 53(28): p. 7124-43.
[Non-Patent Document 16] Kawaguchi, D., H. Ogihara, and H. Kurokawa, Upgrading of Ethanol to 1,1-Diethoxyethane by Proton-Exchange Membrane Electrolysis. ChemSusChem, 2021. 14(20): p. 4431-4438.
[Non-Patent Document 17] Li, Y., et al., AMPK phosphorylates and inhibits SREBP activity to attenuate hepatic steatosis and atherosclerosis in diet-induced insulin-resistant mice. Cell Metab, 2011. 13(4): p. 376-388.
[Non-Patent Document 18] Pulipaka, S., et al., Therapeutic efficacies of mitochondria-targeted esculetin and metformin in the improvement of age-associated atherosclerosis via regulating AMPK activation. Geroscience, 2024. 46(2): p. 2391-2408.
[Non-Patent Document 19] Wang, Z., et al., Dexmedetomidine attenuates myocardial ischemia/reperfusion-induced ferroptosis via AMPK/GSK-3β/Nrf2 axis. Biomed Pharmacother, 2022. 154: p. 113572.
[Non-Patent Document 20] Zhang, Y., et al., Melatonin attenuates myocardial ischemia-reperfusion injury via improving mitochondrial fusion/mitophagy and activating the AMPK-OPA1 signaling pathways. J Pineal Res, 2019. 66(2): p. e12542.
[Non-Patent Document 21] Gélinas, R., et al., AMPK activation counteracts cardiac hypertrophy by reducing O-GlcNAcylation. Nat Commun, 2018. 9(1): p. 374.
[Non-Patent Document 22] Guo, R. and J. Ren, Deficiency in AMPK attenuates ethanol-induced cardiac contractile dysfunction through inhibition of autophagosome formation. Cardiovasc Res, 2012. 94 (3): p. 480-91.
[Non-Patent Document 23] Tong, L., Acetyl-coenzyme A carboxylase: crucial metabolic enzyme and attractive target for drug discovery. Cell Mol Life Sci, 2005. 62(16): p. 1784-803.
[Non-Patent Document 24] Lepropre, S., et al., AMPK-ACC signaling modulates platelet phospholipids and potentiates thrombus formation. Blood, 2018. 132(11): p. 1180-1192.
[Non-Patent Document 25] Turdi, S., et al., Deficiency in AMP-activated protein kinase exaggerates high fat diet-induced cardiac hypertrophy and contractile dysfunction. J Mol Cell Cardiol, 2011. 50(4): p. 712-22.
[Non-Patent Document 26] Hopkins, T.A., J.R. Dyck, and G.D. Lopaschuk, AMP-activated protein kinase regulation of fatty acid oxidation in the ischaemic heart. Biochem Soc Trans, 2003. 31(Pt 1): p. 207-12.
[Non-Patent Document 27] Harold, K.M., et al., Loss of Cardiac PFKFB2 Drives Metabolic, Functional, and Electrophysiological Remodeling in the Heart. J Am Heart Assoc, 2024. 13(7): p. e033676.
[Non-Patent Document 28] Fu, C., et al., PFKFB2 Inhibits Ferroptosis in Myocardial Ischemia/Reperfusion Injury Through Adenosine Monophosphate-Activated Protein Kinase Activation. J Cardiovasc Pharmacol, 2023. 82(2): p. 128-137.
[Non-Patent Document 29] Gao, J., et al., HIF-1/AKT Signaling-Activated PFKFB2 Alleviates Cardiac Dysfunction and Cardiomyocyte Apoptosis in Response to Hypoxia. Int Heart J, 2021. 62(2): p. 350-358.
[Non-Patent Document 30] Bergeron, R., et al., Chronic activation of AMP kinase results in NRF-1 activation and mitochondrial biogenesis. Am J Physiol Endocrinol Metab, 2001. 281(6): p. E1340-6.
[Non-Patent Document 31] Lantier, L., et al., AMPK controls exercise endurance, mitochondrial oxidative capacity, and skeletal muscle integrity. Faseb j, 2014. 28(7): p. 3211-24.
[Non-Patent Document 32] Settembre, C., et al., TFEB controls cellular lipid metabolism through a starvation-induced autoregulatory loop. Nat Cell Biol, 2013. 15(6): p. 647-58.
[Non-Patent Document 33] Jäger, S., et al., AMP-activated protein kinase(AMPK) action in skeletal muscle via direct phosphorylation of PGC-1alpha. Proc Natl Acad Sci U S A, 2007. 104 (29): p. 12017-22.
[Non-Patent Document 34] Chen, L., et al., PGC-1α-Mediated Mitochondrial Quality Control: Molecular Mechanisms and Implications for Heart Failure. Front Cell Dev Biol, 2022. 10: p. 871357.
[Non-Patent Document 35] Tian, L., et al., Pretreatment with Tilianin improves mitochondrial energy metabolism and oxidative stress in rats with myocardial ischemia/reperfusion injury via AMPK/SIRT1/PGC-1 alpha signaling pathway. J Pharmacol Sci, 2019. 139(4): p. 352-360.

### [Detailed Description of the Invention]

### [Technical Problem]

Accordingly, as a result of efforts made to study the physiological function of 1,1-diethoxyethane (1,1-DEE), the inventors of the present invention confirmed that 1,1-DEE acutely inhibits mitochondrial oxidative phosphorylation (OXPHOS) (see Experimental Example 1), and, in the long term, activates AMPK and increases PGC-1α expression, thereby promoting mitochondrial biogenesis (see Experimental Examples 5 and 6). Thus, it was demonstrated that 1,1-DEE can regulate mitochondrial function and energy metabolism by exerting an effect of acutely inhibiting mitochondrial respiration while, at the same time, promoting mitochondrial biogenesis in the long term through AMPK activation, and based on this, the present invention was completed.

Furthermore, the inventors confirmed that 1,1-diethoxyethane (1,1-DEE) according to the present invention not only induces mitochondrial biosynthesis through such an AMP-activated protein kinase (AMPK) pathway-based physiological mechanism, but also exhibits significant therapeutic effects in various disease models. Specifically, it was confirmed that (i) 1,1-diethoxyethane (1,1-DEE) has the effect of extending lifespan without inhibiting development in the Caenorhabditis elegans (C. elegans) model, and (ii) through the effect of transiently and sharply increasing heart rate, it can contribute to the prevention or improvement of cardiovascular diseases (CVD). In addition, (iii) it was confirmed that 1,1-diethoxyethane (1,1-DEE) exhibits an anticancer effect by reducing the survival rate of cancer cells and inducing apoptosis, (iv) it was confirmed that in an ischemic stroke animal model (middle cerebral artery occlusion, MCAO), 1,1-diethoxyethane (1,1-DEE) exhibits effects such as reduction of mortality, promotion of recovery, improvement of cognitive function, and reduction of the brain damage area, and also (v) it was confirmed that 1,1-diethoxyethane (1,1-DEE) can exhibit an effect of improving spatial cognition and memory not only in recovery of cognitive function after ischemic brain injury but also in normal mice.

Accordingly, the present invention aims to provide a mitochondrial biosynthesis promoter comprising 1,1-diethoxyethane (1,1-DEE) as an active ingredient.

The present invention aims to provide a pharmaceutical composition for the prevention or treatment of diseases requiring promotion of mitochondrial biosynthesis comprising 1,1-diethoxyethane (1,1-DEE) as an active ingredient. The present invention also aims to provide a pharmaceutical composition for the prevention or treatment of aging-related diseases, prevention or treatment of cardiovascular diseases, prevention or treatment of cancer, prevention or treatment of stroke, or prevention or treatment of dementia comprising 1,1-diethoxyethane (1,1-DEE) as an active ingredient.

The present invention aims to provide a cosmetic composition for the prevention or improvement of diseases requiring promotion of mitochondrial biosynthesis, comprising 1,1-diethoxyethane (1,1-DEE) as an active ingredient. The present invention also aims to provide a cosmetic composition for delaying aging or extending lifespan, for the prevention or improvement of cardiovascular diseases, for the prevention or improvement of cancer, for the prevention or improvement of stroke, or for the prevention or improvement of dementia, cognitive dysfunction, or memory decline, comprising 1,1-diethoxyethane (1,1-DEE) as an active ingredient.

The present invention aims to provide a food composition for the prevention or improvement of diseases requiring promotion of mitochondrial biosynthesis, comprising 1,1-diethoxyethane (1,1-DEE) as an active ingredient. The present invention also aims to provide a food composition for delaying aging or extending lifespan, for the prevention or improvement of cardiovascular diseases, for the prevention or improvement of cancer, for the prevention or improvement of stroke, or for the prevention or improvement of dementia, cognitive dysfunction, or memory decline, comprising 1,1-diethoxyethane (1,1-DEE) as an active ingredient.

The present invention aims to provide a feed composition for the prevention or improvement of diseases requiring promotion of mitochondrial biosynthesis, comprising 1,1-diethoxyethane (1,1-DEE) as an active ingredient. The present invention also aims to provide a feed composition for delaying aging or extending lifespan, for the prevention or improvement of cardiovascular diseases, for the prevention or improvement of cancer, for the prevention or improvement of stroke, or for the prevention or improvement of dementia, cognitive dysfunction, or memory decline, comprising 1,1-diethoxyethane (1,1-DEE) as an active ingredient.

However, the technical problem to be solved by the present invention is not limited to the problems mentioned above, and other problems not specifically mentioned herein will be clearly understood by those skilled in the art from the following description.

### [Technical Solution]

The present invention discloses a mitochondrial biosynthesis promoter comprising 1,1-diethoxyethane (1,1-DEE) as an active ingredient and the use thereof, for example, a composition for the prevention, treatment, or improvement of a disease requiring promotion of mitochondrial biosynthesis, including a pharmaceutical composition, cosmetic composition, food composition, or feed composition.

The present invention also discloses a composition comprising 1,1-diethoxyethane (1,1-DEE) as an active ingredient for the prevention or treatment of aging-related diseases, and for delaying aging or extending lifespan.

The present invention also discloses a composition comprising 1,1-diethoxyethane (1,1-DEE) as an active ingredient for the prevention, improvement, or treatment of cardiovascular diseases.

The present invention also discloses a composition comprising 1,1-diethoxyethane (1,1-DEE) as an active ingredient for the prevention, improvement, or treatment of cancer and cancer-derived cachexia.

The present invention also discloses a composition comprising 1,1-diethoxyethane (1,1-DEE) as an active ingredient for the prevention, improvement, or treatment of stroke.

The present invention also discloses a composition comprising 1,1-diethoxyethane (1,1-DEE) as an active ingredient for the prevention, improvement, or treatment of dementia.

The present invention also discloses a composition comprising 1,1-diethoxyethane (1,1-DEE) as an active ingredient for the prevention, improvement, or treatment of cognitive dysfunction or memory impairment.

In the present invention, the 1,1-diethoxyethane (1,1-DEE) has the molecular formula C₆H₁₄O₂ and is represented by the following Structural Formula 1. It is also known as acetaldehyde diethyl acetal or ethylidene diethyl ether.

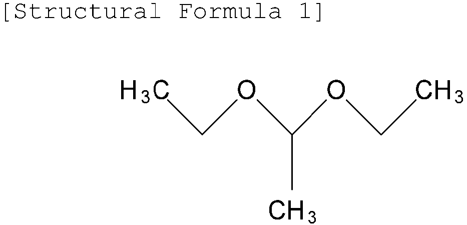

According to one embodiment, a mitochondrial biosynthesis promoter comprising 1,1-diethoxyethane (1,1-DEE) as an active ingredient, is disclosed.

In the present invention, the 1,1-diethoxyethane (1,1-DEE) may promote mitochondrial biosynthesis by increasing the expression of PGC-1α (peroxisome proliferator-activated receptor gamma coactivator-1 alpha).

In the present invention, the 1,1-diethoxyethane (1,1-DEE) may also promote mitochondrial biosynthesis by enhancing the transcriptional activities of Nrf1 and Nrf2, which in turn stimulate the expression of Tfam.

In the present invention, the 1,1-diethoxyethane may also promote an aerobic respiration.

According to another embodiment, a pharmaceutical composition for the prevention or treatment of a disease requiring promotion of mitochondrial biosynthesis, comprising 1,1-diethoxyethane (1,1-DEE) as an active ingredient, is disclosed.

In the present invention, the disease requiring promotion of mitochondrial biogenesis may be selected from the group consisting of ischemic brain diseases, ischemic heart diseases, pulmonary embolism, ischemic-reperfusion injury, stroke, ischemic stroke, migraine, anemia, Parkinson's disease (PD), dementia, Alzheimer's disease (AD), Huntington's disease, Frontotemporal Dementia, amyotrophic lateral sclerosis (ALS), Leigh syndrome, mitochondrial myopathy, mitochondrial encephalomyopathy, Lactic Acidosis, and Stroke-like episodes (MELAS), cirrhosis, Fanconi syndrome, atypical phenylketonuria, asthma, chronic obstructive pulmonary disease (COPD), diabetes, atherosclerosis, heart failure, hypertension, metabolic dysfunction, metabolic dysfunction-associated steatotic liver disease (MASLD), insulin resistance, hypercholesterolemia, optic nerve diseases, Duchenne muscular dystrophy, myopathies, obesity, depression, Osteoarthritis, , cancer, cancer cachexia, inflammatory diseases, age-related diseases, and cardiovascular diseases, cancer, cognitive ability or memory.

In the present invention, the pharmaceutical composition may be administered by one or more administration routes selected from the group consisting of oral administration, intravenous injection, subcutaneous injection, intramuscular injection, intraperitoneal injection, epithelial administration, topical administration, intravaginal administration, pulmonary administration, rectal administration, sublingual administration, buccal administration, transdermal administration, ocular administration, inhalation, intracavernous injection, intrathecal injection, epidural injection, and rectal administration.

According to another embodiment, a cosmetic composition for the prevention or improvement of diseases requiring mitochondrial biosynthesis promotion, comprising 1,1-diethoxyethane (1,1-DEE) as an active ingredient, is disclosed.

In the present invention, the cosmetic composition may be formulated into one or more dosage forms selected from the group consisting of solution, topical ointment, cream, foam, nourishing toner, softening toner, perfume, pack, softening lotion, emulsion, makeup base, essence, soap, liquid cleanser, bath agent, sunscreen cream, sun oil, suspension, emulsion, paste, gel, lotion, powder, soap, surfactant-containing cleansing, oil, powder foundation, emulsion foundation, wax foundation, patch, and spray.

According to another embodiment, a food composition for the prevention or improvement of diseases requiring mitochondrial biosynthesis promotion, comprising 1,1-diethoxyethane (1,1-DEE) as an active ingredient, is disclosed.

In the present invention, the food may include meat, sausage, bread, chocolate, candy, snacks, cookies, pizza, ramen, other noodles, chewing gum, ice cream and dairy products, various soups, beverages, tea, coffee beverages, stamina drinks, alcoholic beverages, or vitamin complexes.

According to another embodiment, a feed composition for the prevention or improvement of diseases requiring mitochondrial biosynthesis promotion, comprising 1,1-diethoxyethane (1,1-DEE) as an active ingredient, is disclosed.

In the present invention, the feed may include powdered feed, solid feed, moist pellet feed, dry pellet feed, extruder pellet (EP) feed, or raw feed.

### [Effects of the Invention]

According to the present invention, 1,1-diethoxyethane (1,1-DEE) can rapidly and reversibly activate AMP-activated protein kinase (AMPK) through ATP depletion and reactive oxygen species (ROS) generation. The activated AMPK induces acetyl-CoA carboxylase (ACC) phosphorylation to inhibit fatty acid synthesis and promote β-oxidation, and regulates glycolysis through phosphorylation of 6-phosphofructo-2-kinase/fructose-2,6-bisphosphatase 2 (PFKFB2). Furthermore, AMPK activation induces an increase in peroxisome proliferator-activated receptor gamma coactivator 1-alpha (PGC-1α) expression, thereby enhancing mitochondrial biosynthesis and energy metabolism, and can promote the expression of mitochondria-related genes and structural formation via the Nrf1/Nrf2-Tfam axis.

Based on this physiological mechanism that induces mitochondrial biosynthesis and metabolic function enhancement, 1,1-DEE exhibited significant therapeutic effects in various disease models.

First, regarding the effect on aging delay and lifespan extension, 1,1-DEE exhibited the ability to extend lifespan in a Caenorhabditis elegans (C. elegans) model without inhibiting development. These results suggest that 1,1-DEE can be utilized as a pharmacological composition, cosmetic composition, food composition, functional health food, or feed composition effective for delaying aging or extending lifespan.

Second, regarding cardiovascular diseases, 1,1-DEE exhibited a transient and rapid increase in heart rate, suggesting its effective use for the prevention, treatment, or improvement of cardiovascular diseases. In addition, the compositions of the present invention can be applied to functional foods or animal feed, which may contribute, for example, to the health management of individuals requiring heart rate regulation or the regulation of abnormal heart rates in livestock and companion animals. Therefore, the present invention provides a safe and effective method for increasing heart rate using 1,1-DEE, which represents a promising technology for the prevention and treatment of cardiovascular diseases requiring enhanced cardiac activity.

Third, regarding cancer and cachexia, 1,1-DEE significantly reduced cancer cell viability and induced apoptosis, thereby exhibiting anticancer effects. In particular, it was confirmed that activation of the AMPK pathway provides a novel anticancer mechanism based on energy metabolism regulation. In vivo experiments further demonstrated therapeutic effects, including tumor growth suppression, prevention of cancer cachexia, and improved survival. Moreover, unlike 1,2-DEE, 1,1-DEE showed selective efficacy, making it a promising anticancer candidate in terms of both safety and effectiveness. Accordingly, the compositions of the present invention can be usefully applied as pharmacological compositions, food compositions, cosmetic compositions, or feed compositions for the prevention, treatment, or improvement of cancer and cancer-derived cachexia.

Fourth, regarding ischemic stroke, 1,1-DEE demonstrated various therapeutic effects in the mouse middle cerebral artery occlusion (MCAO) model, including reduced mortality, prevention of weight loss, enhanced recovery, improved spatial cognition and memory, and reduced brain lesion areas. In particular, the Morris water maze test showed a tendency to locate the platform more rapidly during early learning phases, and MRI analysis confirmed neuroprotective effects. These results suggest that 1,1-DEE may be an effective therapeutic agent for neuroprotection and cognitive function recovery in ischemic stroke.

Fifth, regarding cognitive function and memory impairment, 1,1-DEE improved cognitive abilities not only in stroke models but also in normal mice. Mice treated with 1,1-DEE in the stroke model located the platform faster, followed more direct paths, and traveled significantly shorter distances. In normal mice, repeated administration also showed a tendency to enhance learning speed and memory, particularly with significantly reduced escape times in post-tests. These results indicate that 1,1-DEE is effective in preventing or improving cognitive function and memory decline.

However, the scope of the present invention is not limited by the above-described effects.

### [Brief Description of Drawings]

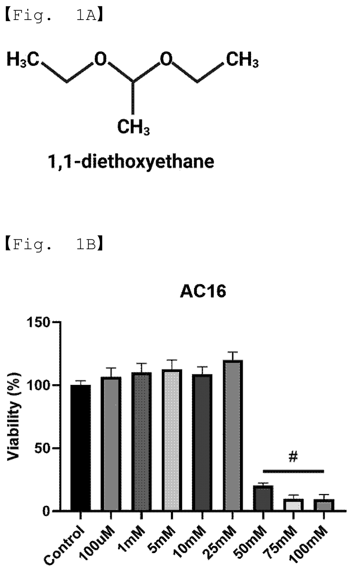

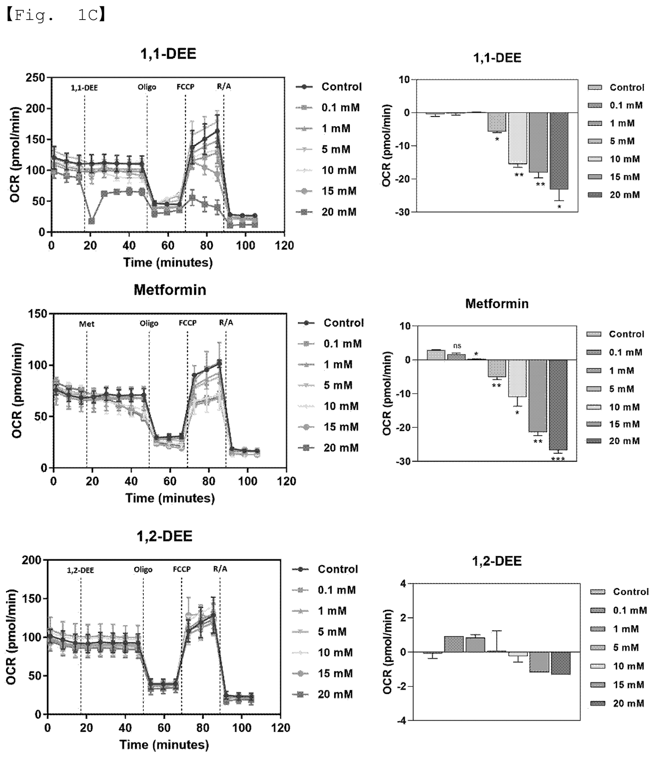

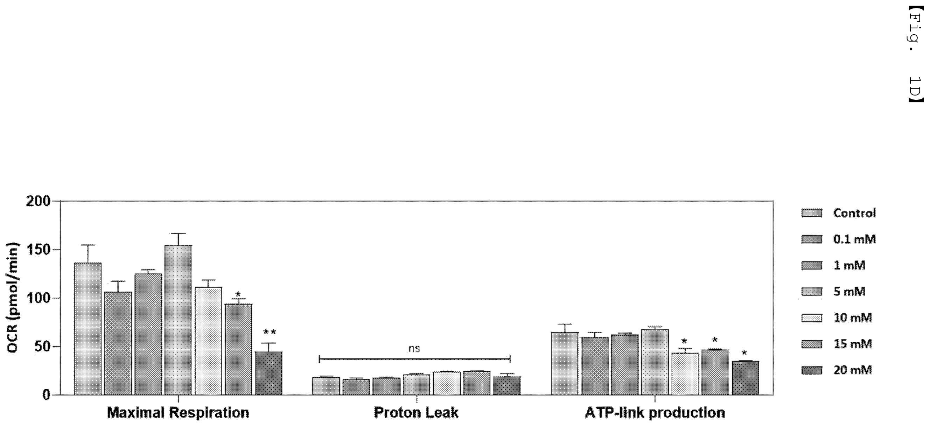

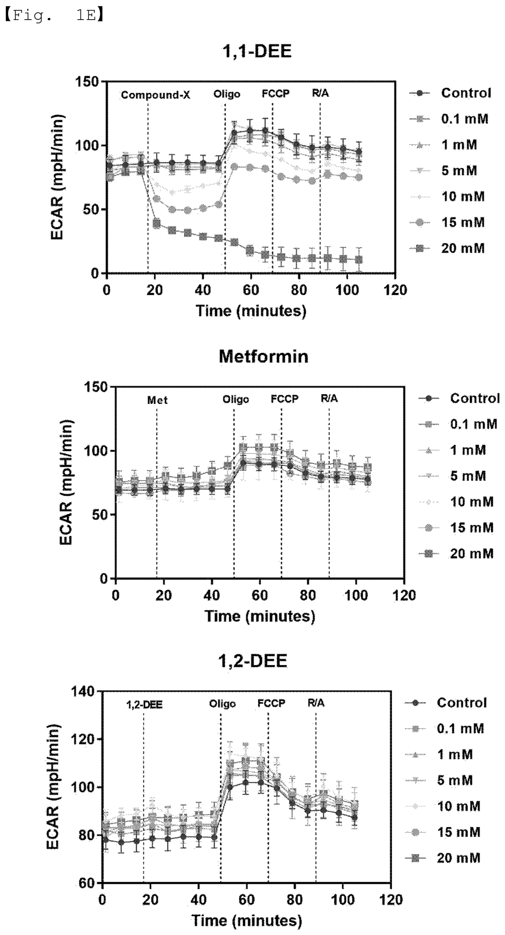

Figure 1 shows the effects of acute exposure to 1,1-DEE on oxygen consumption rate (OCR) and reversible inhibition of glycolysis in AC16 cells: (A) 2D structure of 1,1-DEE. (B) Cell viability assay of AC16 cells cultured for 24 hours with the indicated concentrations of 1,1-DEE using EZ-Cytox reagent. (C) Mitochondrial respiration was evaluated using the Agilent Seahorse XF96 Cell Mito Stress Test Kit after acute exposure to 1,1-DEE, metformin, or 1,2-DEE for 30 minutes. OCR was measured following sequential addition of various concentrations of 1,1-DEE, 1.5 µM oligomycin (Oligo), 1.5 µM FCCP, and 0.5 µM rotenone/antimycin (R/A). (D) Proton leak, ATP production, maximal respiration, and spare respiratory capacity were measured using the Seahorse XF96 Cell Mito Stress Test Kit. (E) Extracellular acidification rate (ECAR) was measured under the conditions described in (C).

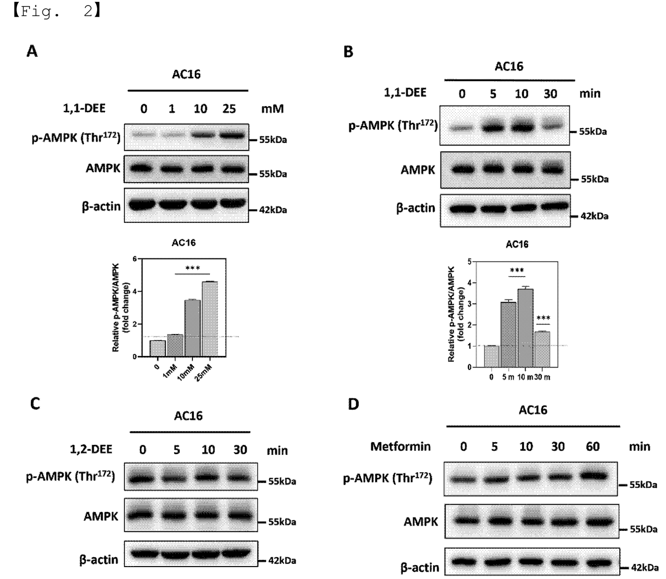

Figure 2 shows the concentration- and time-dependent activation of AMPK upon acute exposure to 1,1-DEE. Western blot analysis of AC16 cells was performed to evaluate phosphorylation of AMPK under the following conditions: (A) Cultured for 10 minutes with various concentrations of 1,1-DEE. (B) Cultured for 30 minutes with 15 mM 1,1-DEE. (C) Cultured for 30 minutes with 15 mM 1,2-DEE. (D) Cultured for 60 minutes with 15 mM metformin.

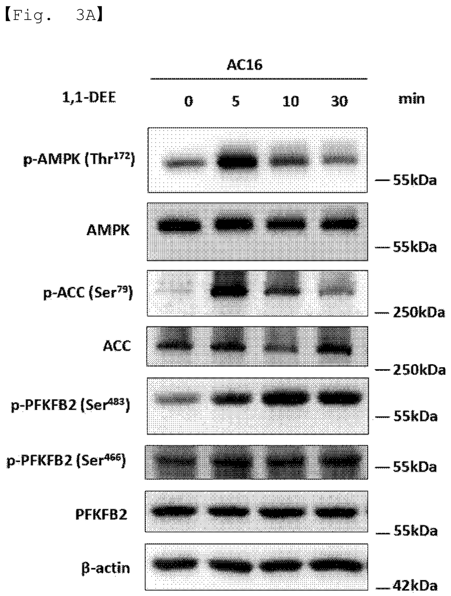

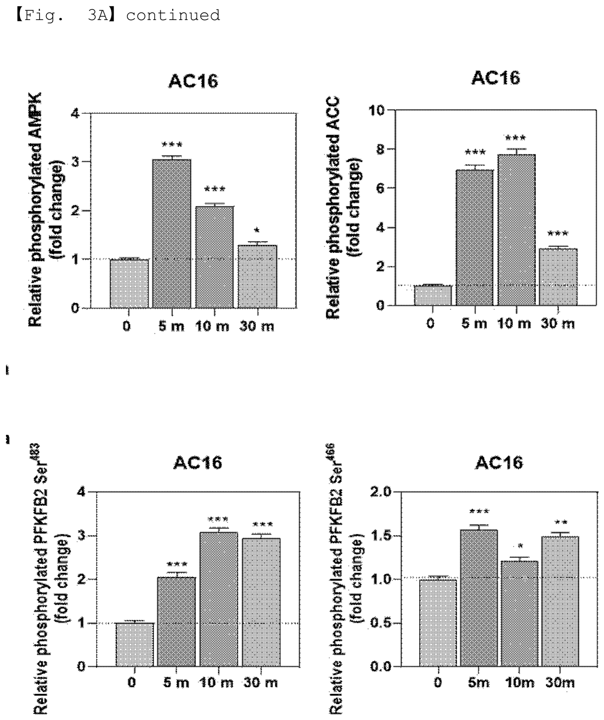

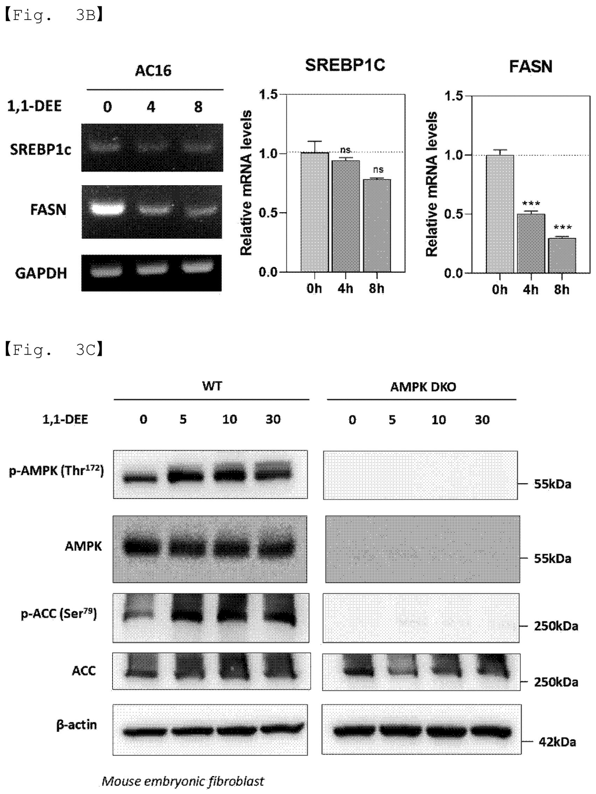

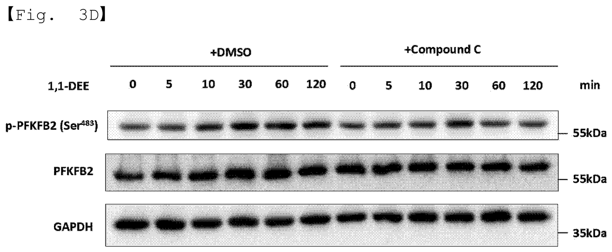

Figure 3 shows the effects of AMPK activation on fatty acid oxidation and glycolysis upon acute exposure to 1,1-DEE: (A) AC16 cells were exposed to 15 mM 1,1-DEE for 30 minutes. Western blot analysis was performed to evaluate phosphorylation of AMPK, ACC, and PFKFB2. (B) AC16 cells were exposed to 15 mM 1,1-DEE for 8 hours. mRNA expression levels of SREBP-1c and FASN were assessed using semiquantitative PCR and qRT-PCR. (C) Wild-type (WT) and AMPK double knockout (DKO) MEF cells were exposed to 15 mM 1,1-DEE for 30 minutes. (D) AC16 cells were pretreated with 10 µM Compound C for 1 hour, followed by incubation with 15 mM 1,1-DEE for 120 minutes. Western blot analysis was performed to evaluate phosphorylation of AMPK effectors including ACC and PFKFB2.

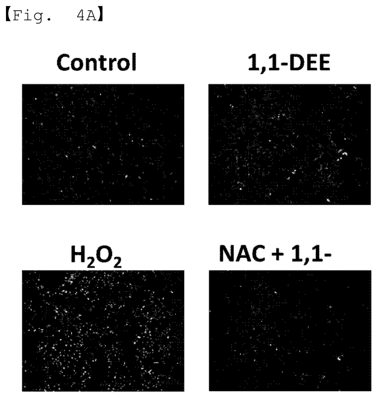

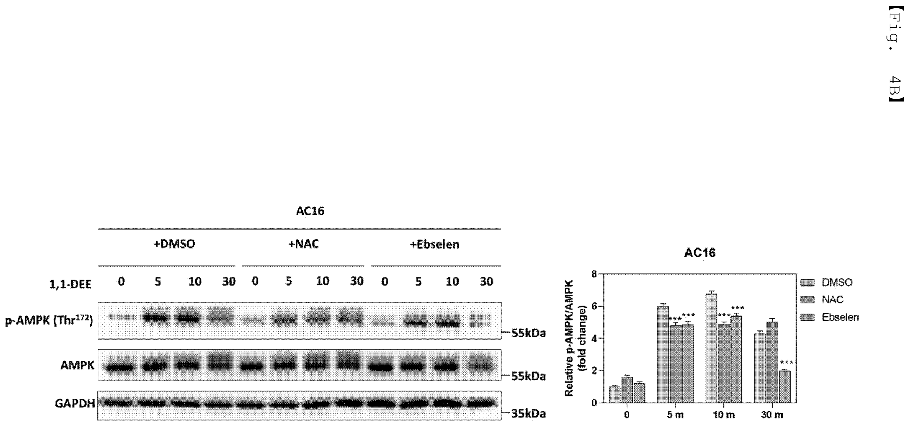

Figure 4 shows that AMPK activation may be partially mediated by 1,1-DEE-induced ROS generation. AC16 cells were pretreated with 10 mM NAC for 2 hours or 10 µM ebselen for 1 hour, followed by exposure to 15 mM 1,1-DEE for 30 minutes. ROS generation was observed by immunofluorescence staining using DCFH-DA, and Western blot analysis was performed to assess AMPK activation via phosphorylation at the Thr172 residue.

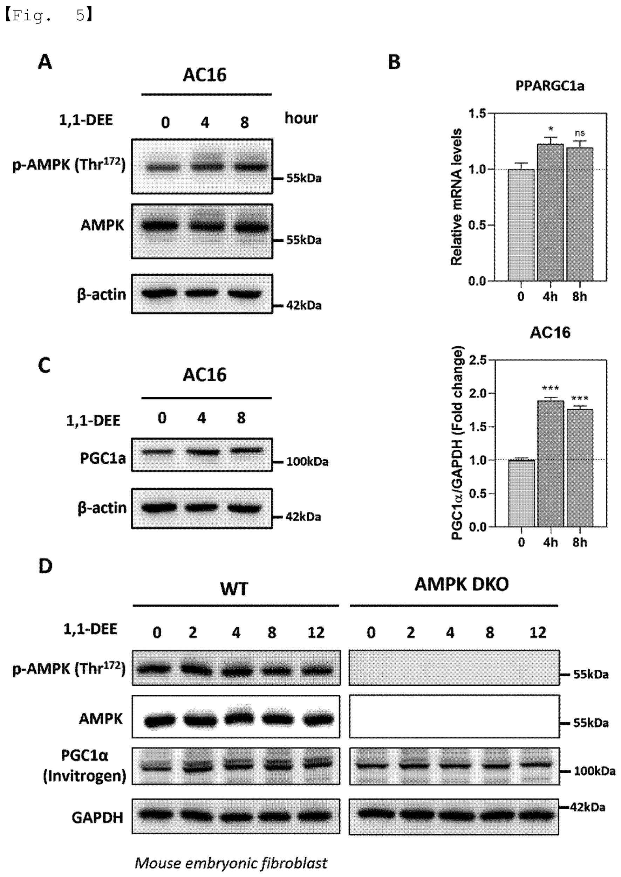

Figure 5 shows that long-term exposure to 1,1-DEE can increase PGC-1α expression via AMPK activation. (A-C) AC16 cells were cultured with 15 mM 1,1-DEE for up to 8 hours: (A) AMPK phosphorylation was evaluated by Western blot analysis. (B) PPARGC1A mRNA expression levels were analyzed using RT-PCR. (C) PGC-1α protein expression levels were confirmed by Western blot analysis. (D) MEF cells were cultured with 15 mM 1,1-DEE for up to 12 hours, and AMPK phosphorylation and PGC-1α expression were evaluated by Western blot analysis.

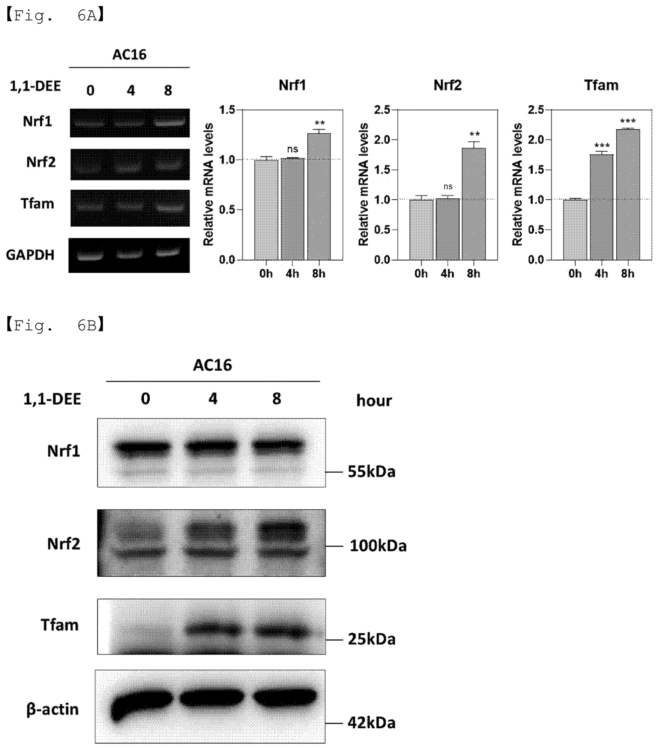

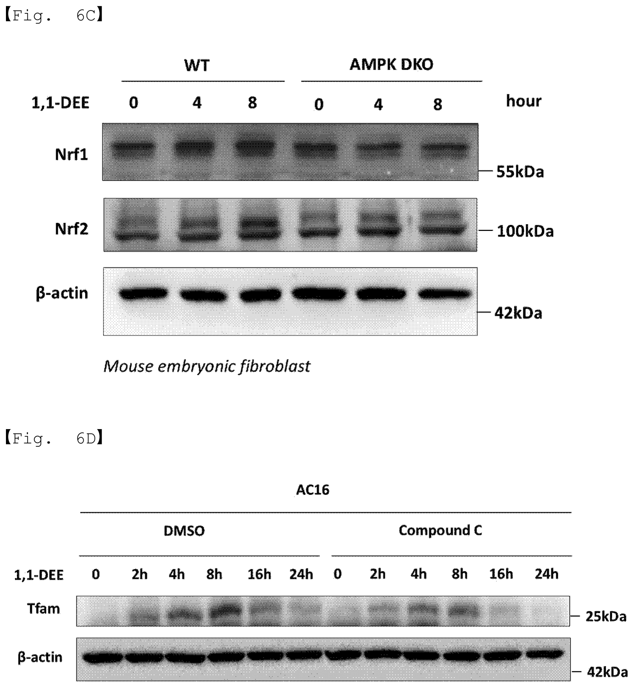

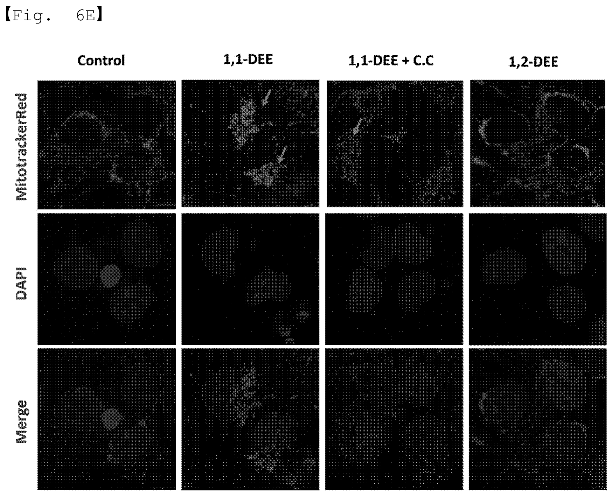

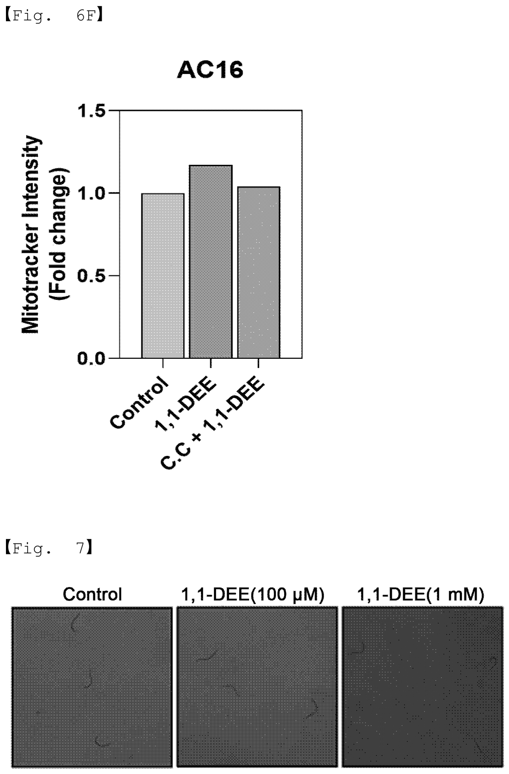

Figure 6 illustrates that PGC-1α-mediated AMPK activation can promote mitochondrial biosynthesis. (A-B) AC16 cells were cultured with 15 mM 1,1-DEE for up to 8 hours: (A) mRNA expression levels of Nrf1, Nrf2, and Tfam were assessed by real-time PCR. (B) Protein expression levels of Nrf1, Nrf2, and Tfam were analyzed by Western blot. (C) WT and AMPK DKO MEF cells were cultured with 15 mM 1,1-DEE for up to 8 hours, and protein expression levels of Nrf1, Nrf2, and Tfam were confirmed by Western blot analysis. (D) AC16 cells were pretreated with 10 µM Compound C for 4 hours and then exposed to 15 mM 1,1-DEE for 24 hours; Tfam protein expression levels were evaluated by Western blot analysis. (E) AC16 cells were pretreated with 10 µM Compound C for 4 hours, exposed to 15 mM 1,1-DEE for 8 hours, and stained with 200 nM MitoTracker Red CMXRos for 30 minutes. Mitochondrial biosynthesis was evaluated using confocal microscopy at 40× magnification. (F) Under the same conditions as (E) , flow cytometry was performed on AC16 cells.

Figure 7 shows the effect of 1,1-DEE (A) according to the present invention on the development of Caenorhabditis elegans at concentrations of 100 µM and 1 mM.

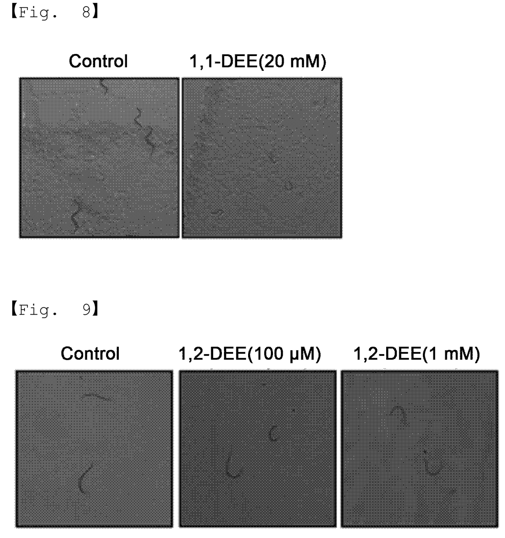

Figure 8 shows the effect of 1,1-DEE (A) according to the present invention on the development of Caenorhabditis elegans at a concentration of 20 mM.

Figure 9 shows the effect of the 1,2-DEE isoform of 1,1-DEE (A) according to the present invention on the development of Caenorhabditis elegans at concentrations of 100 µM and 1 mM.

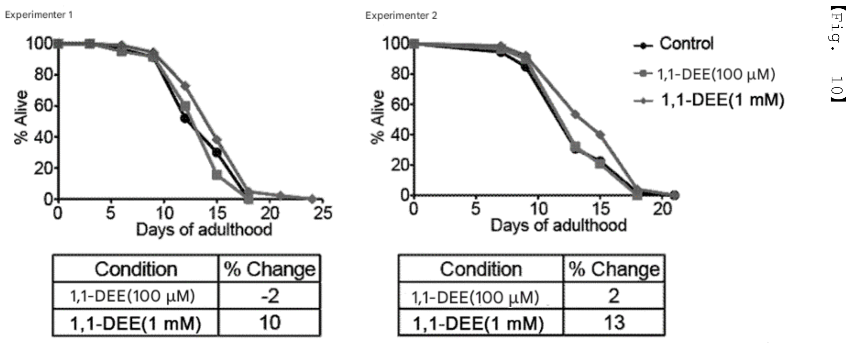

Figure 10 shows the effect of 1,1-DEE (A) according to the present invention on lifespan extension of Caenorhabditis elegans at concentrations of 100 µM and 1 mM.

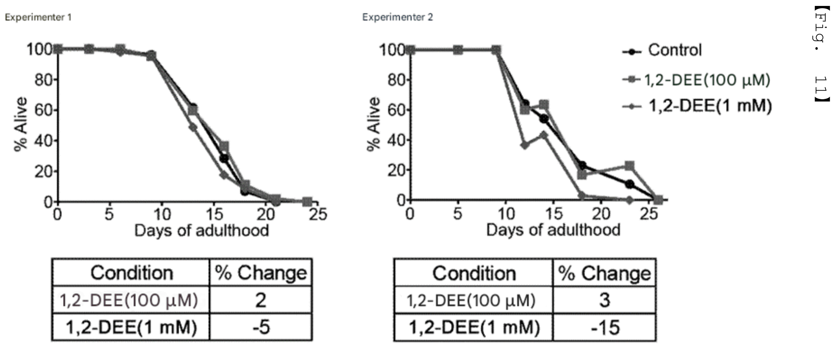

Figure 11 shows the effect of the 1,2-DEE isoform of 1,1-DEE (A) according to the present invention on lifespan extension of Caenorhabditis elegans at concentrations of 100 µM and 1 mM

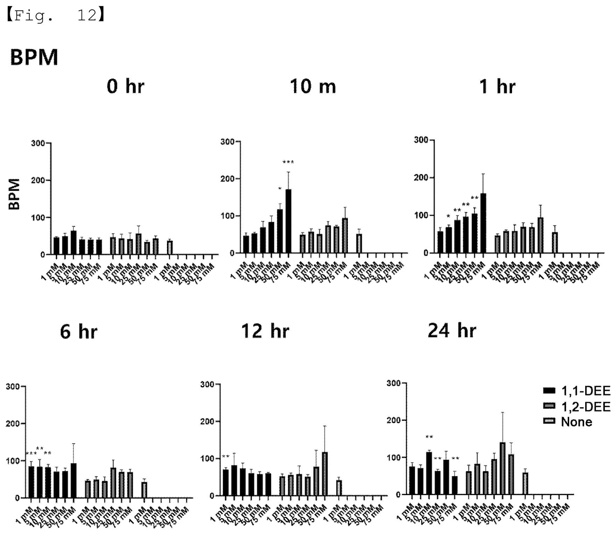

Figure 12 shows the changes in beating rate (BPM: beats per minute) of EHT over time before and after treatment with various concentrations of 1,1-DEE and 1,2-DEE.

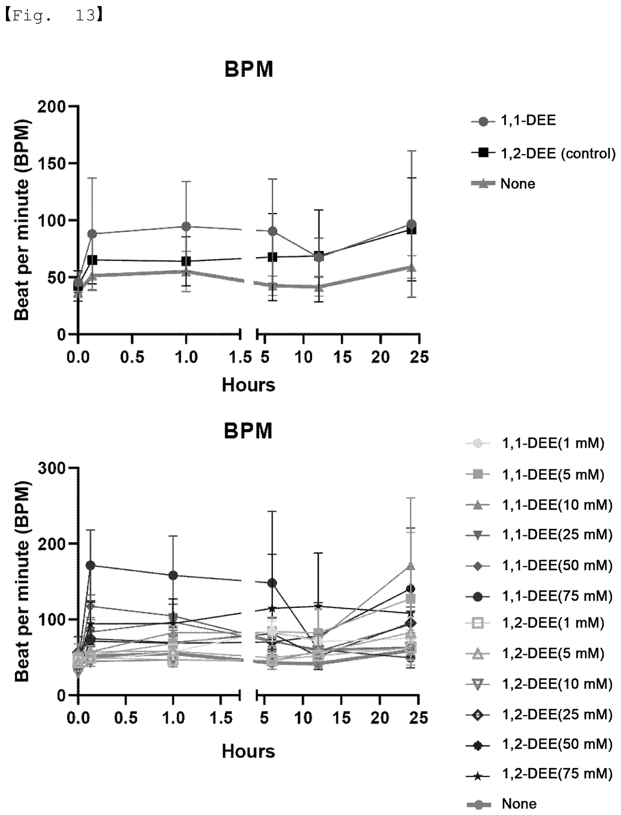

Figure 13 shows the changes in beating rate (BPM: beats per minute) of EHT over time before and after treatment with various concentrations of 1,1-DEE and 1,2-DEE (Top: 26 mM of 1,1-DEE and 1,2-DEE).

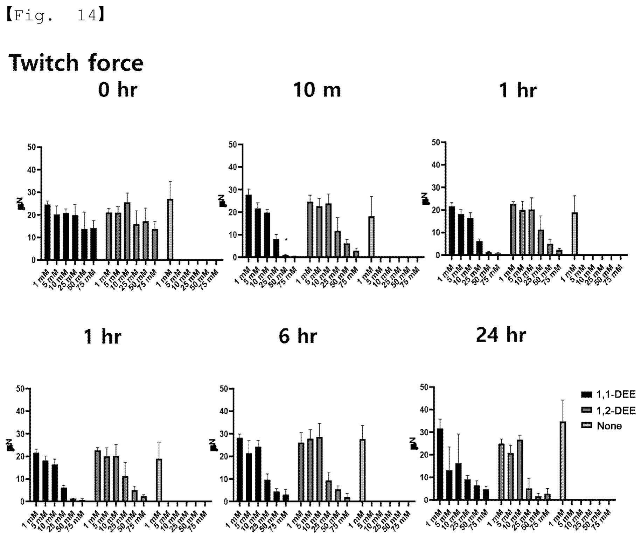

Figure 14 shows the changes in twitch force of EHT over time before and after treatment with various concentrations of 1,1-DEE and 1,2-DEE.

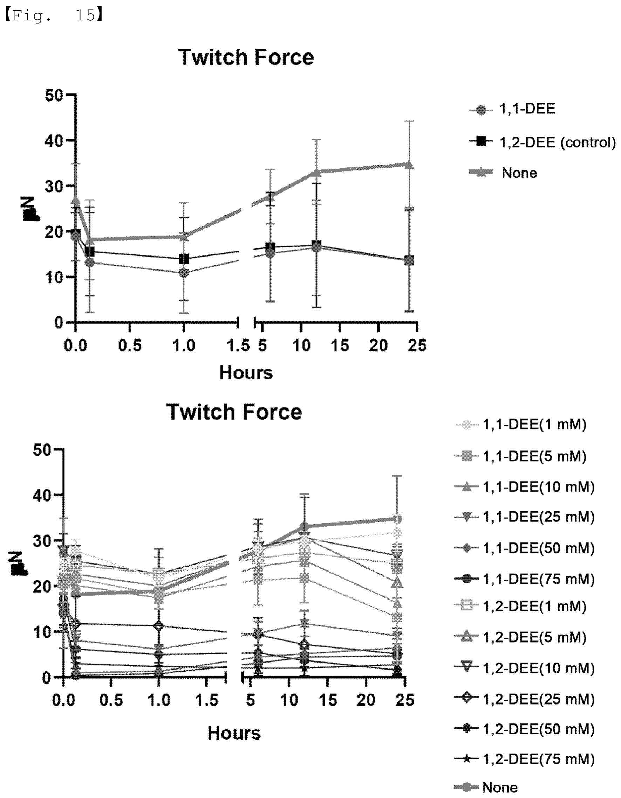

Figure 15 shows the changes in twitch force of EHT over time before and after treatment with various concentrations of 1,1-DEE and 1,2-DEE (Top: 26 mM of 1,1-DEE and 1,2-DEE).

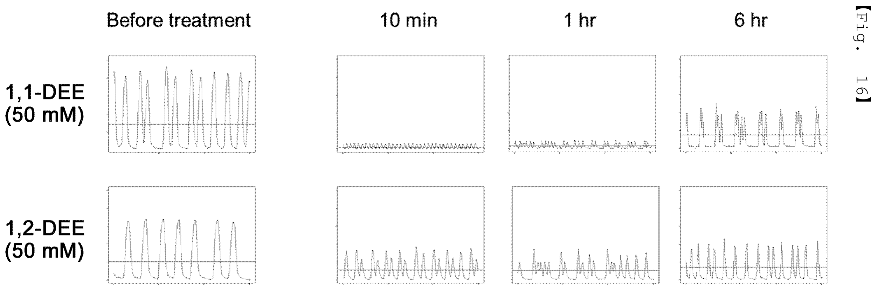

Figure 16 shows representative images of changes in EHT beating upon treatment with 1,1-DEE and 1,2-DEE (50 mM).

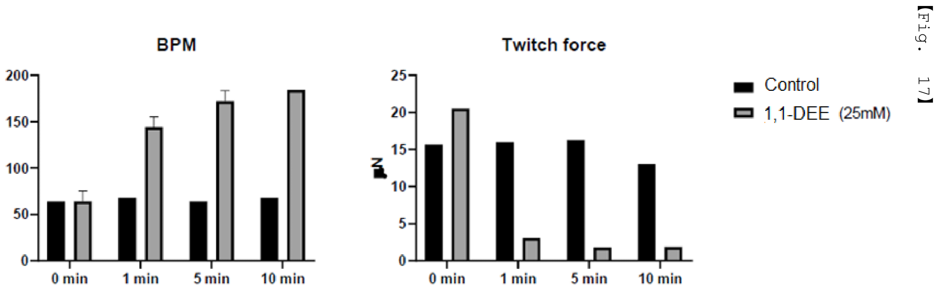

Figure 17 shows the changes in EHT beating rate and twitch force over a 10-minute period immediately after treatment with 1,1-DEE at a concentration of 25 mM.

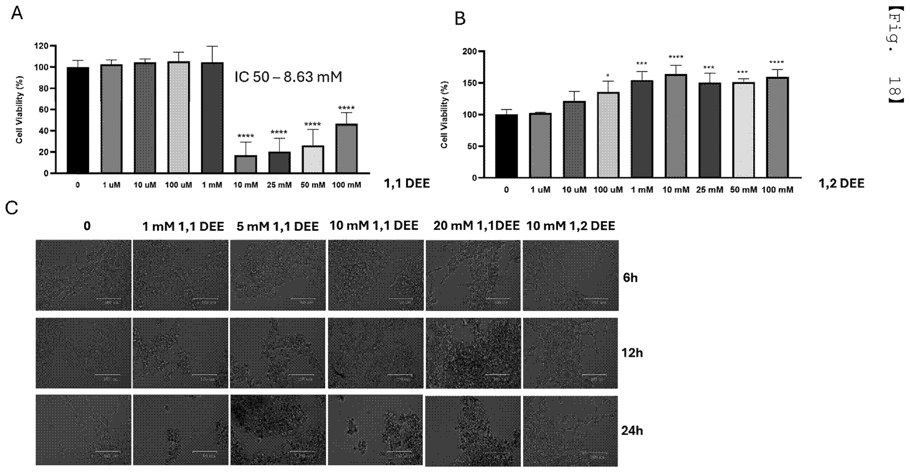

Figure 18 shows the cell viability and morphological changes of SHSY-5Y cells upon treatment with 1,1-diethoxyethane (1,1-DEE) and 1,2-diethoxyethane (1,2-DEE) depending on concentration and exposure time. (A) Cell viability measured by MTT assay after 24-hour treatment with 1,1-DEE or 1,2-DEE, with each experiment performed in triplicate. (B) Morphological changes of SHSY-5Y cells observed under 20× inverted microscopy over time (6, 12, 24 hours) following treatment with varying concentrations (1, 5, 10, 20 mM) of 1,1-DEE or 1,2-DEE.

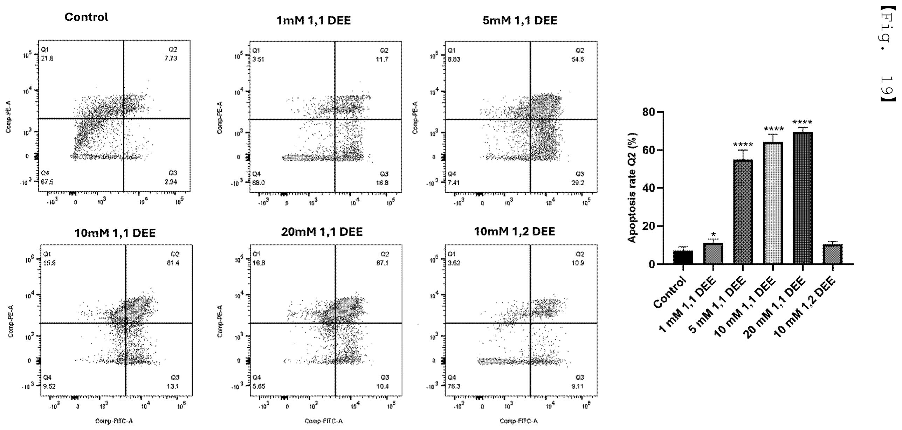

Figure 19 shows the analysis of 1,1-DEE-induced cell death in SHSY-5Y cells using flow cytometry. After 24 hours of treatment, the proportion of apoptotic cells increased significantly with increasing concentrations of 1,1-DEE. Data are presented as mean ± standard deviation (SD) from three independent experiments.

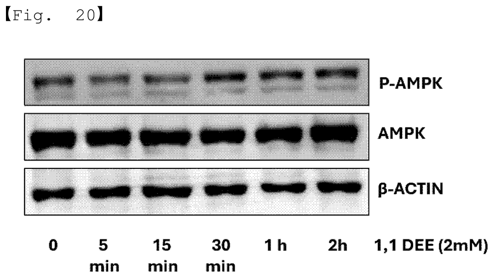

Figure 20 shows the time-dependent activation of AMPK induced by 1,1-DEE in SHSY-5Y cells. Cells were treated with 1,1-DEE for 0-120 minutes, and the expression levels of total AMPK and phosphorylated AMPK proteins were measured by Western blot analysis.

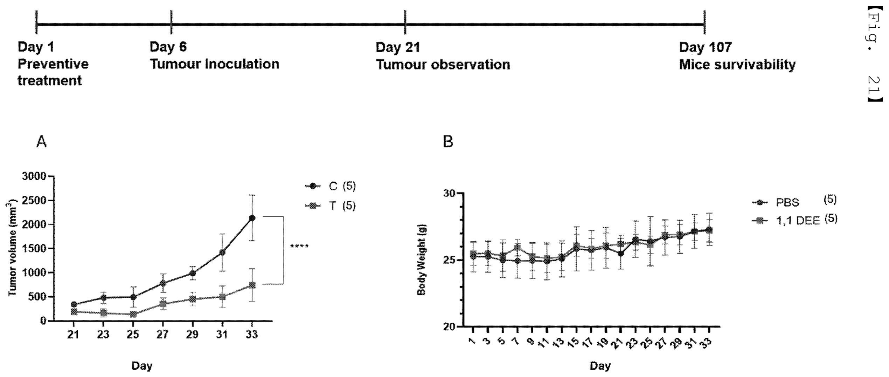

Figure 21 illustrates tumor progression and the inhibitory effect of 1,1-DEE in a xenograft mouse model using SHSY-5Y cells. (A) shows tumor volume changes in the 1,1-DEE treatment group compared to the control group, and (B) presents changes in mouse body weight to assess the overall systemic impact.

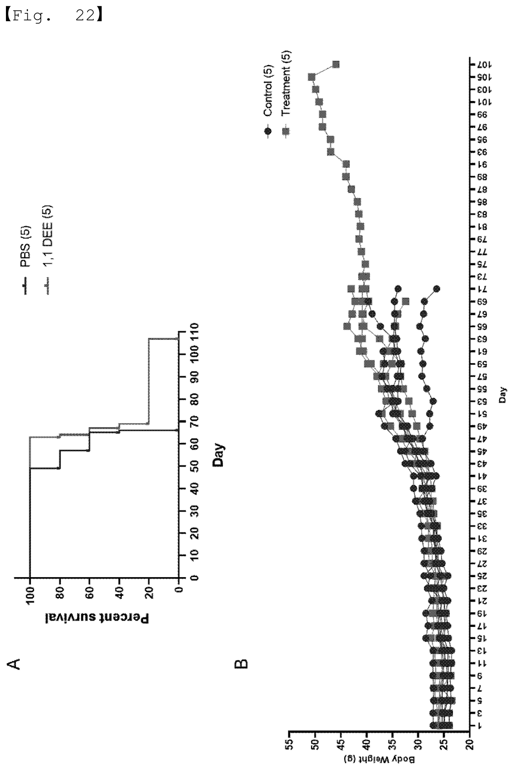

Figure 22 shows the survival rate and body weight changes following preventive administration of 1,1-DEE in a xenograft mouse model. (A) depicts the differences in survival rates between the treatment and control groups, and (B) shows body weight changes from the start of administration to the end of the survival period.

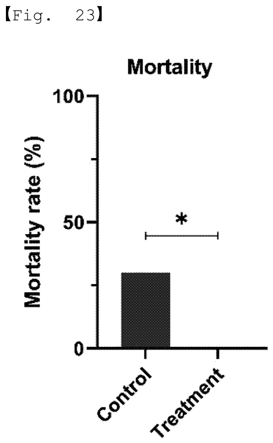

Figure 23 shows the protective effect of 1,1-DEE on mortality in ischemic stroke. In the control group, the mortality rate after middle cerebral artery occlusion (MCAO) was 30%, whereas in the 1,1-DEE treatment group, the mortality rate was 0%. Data are presented as mean ± standard error (SE), with statistical significance indicated (p < 0.05, relative risk [RR] = 0.3684, 95% confidence interval [CI]: 0.1915-0.9505).

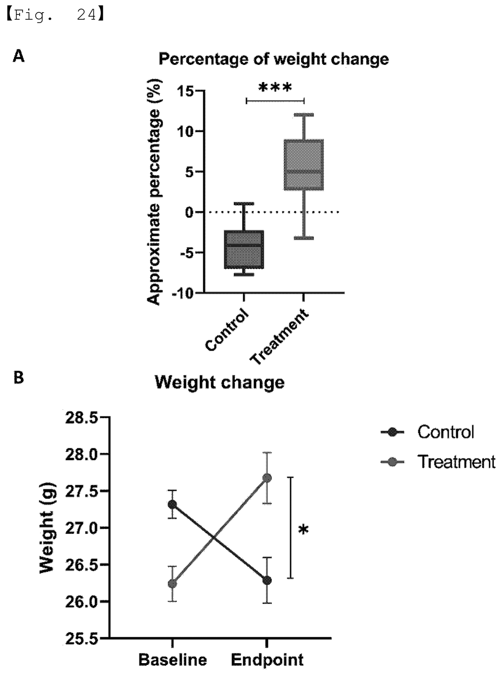

Figure 24 shows the beneficial effect of 1,1-DEE on post-stroke body weight changes. Body weights of the experimental and control groups were measured every 6 days from baseline to the end of the study. (A) The control group exhibited an average weight loss of 3.77% relative to baseline, whereas the treatment group showed an average weight gain of 5.46% (t-test, p < 0.001). (B) While the control group showed continuous weight loss throughout the experiment, the treatment group showed steady weight gain. At the end of the study, the mean body weight of the treatment group was significantly higher than that of the control group (27.68 g vs. 26.29 g, t-test, p < 0.05). All data are presented as mean ± standard error.

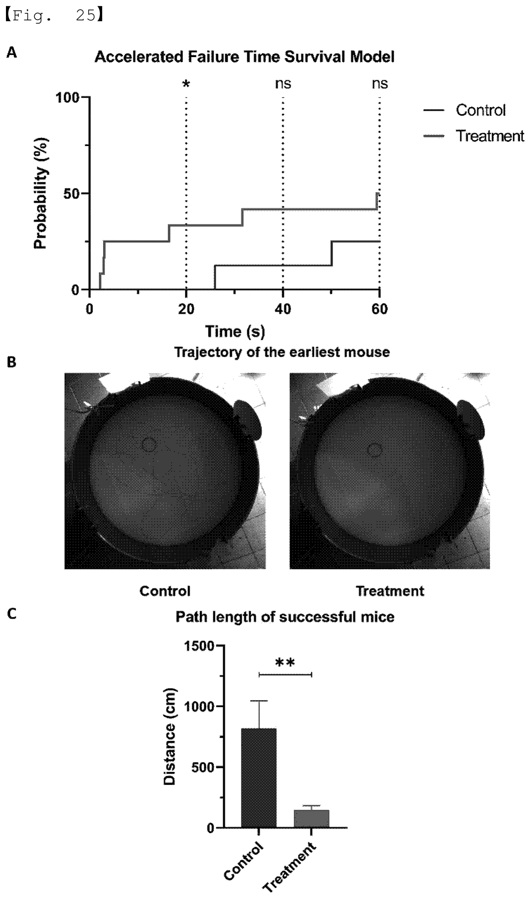

Figure 25 shows the effect of 1,1-DEE on spatial cognition and memory enhancement. (A) The cumulative proportion of mice finding the hidden platform is presented as a time-to-event graph and analyzed using the accelerated failure time (AFT) survival model. At the 20-second time point, the success rate of the treatment group was significantly higher than that of the control group (33% vs. 0%, p < 0.05). However, no statistical significance was observed at the 40-second and 60-second time points. (B) Representative swimming path analysis shows that 1,1-DEE-treated mice followed direct and efficient paths to the platform, whereas control mice exhibited irregular and disoriented paths. (C) Comparison of swimming distances among mice that successfully reached the platform revealed that the treatment group reached the platform via significantly shorter paths than the control group (146.3 cm vs. 820.8 cm, t-test, p < 0.01). All data are presented as mean ± standard error (SE).

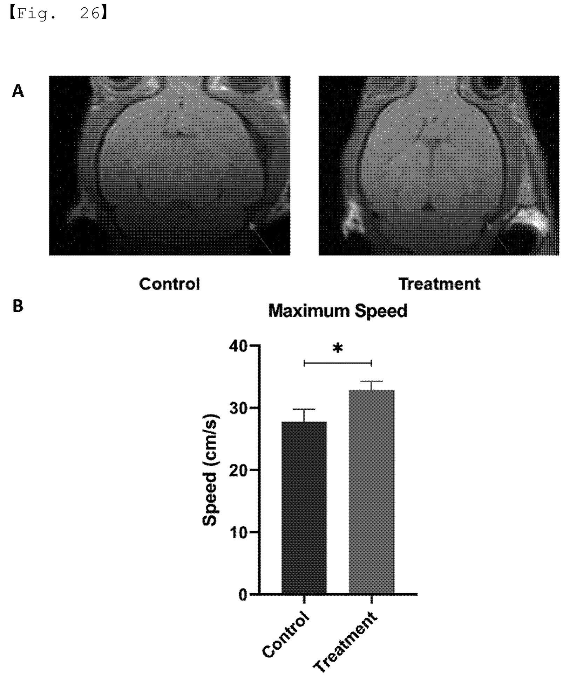

Figure 26 shows that 1,1-DEE can alleviate ischemic stroke and improve post-stroke motor function. (A) Representative brain magnetic resonance imaging (MRI) results for each experimental group are presented. Mice treated with 1,1-DEE exhibited relatively reduced damage from middle cerebral artery occlusion (MCAO). (B) Comparison of maximum swimming speeds measured during the Morris Water Maze (MWM) test. The treatment group showed a significantly higher maximum swimming speed than the control group (33.83 cm/s vs. 27.77 cm/s, t-test, p < 0.05). All data are presented as mean ± standard error (SE).

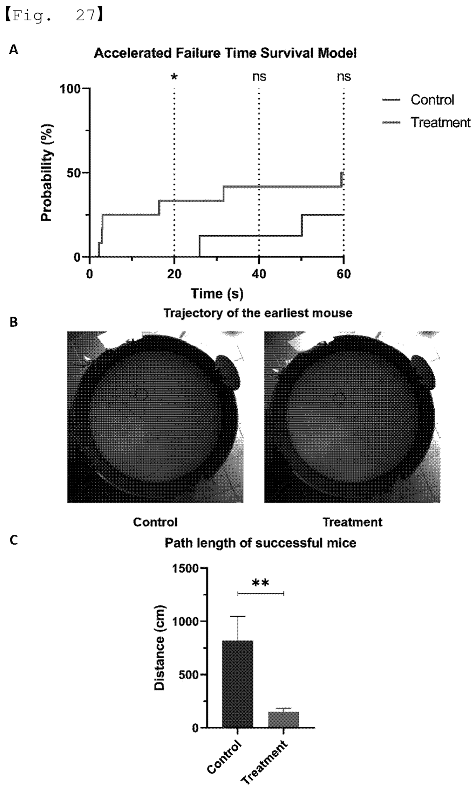

Figure 27 shows that 1,1-DEE can improve spatial cognition and memory in mice after stroke. (A) The time-to-event curve represents the cumulative proportion of mice that located the hidden platform, analyzed using an accelerated failure time (AFT) survival model. Overall, the 1,1-DEE treatment group exhibited a higher probability of reaching the platform compared to the control group. In particular, at the initial 20-second time point, the success rate of the treatment group was 33%, significantly higher than the control group (0%) (p < 0.05). No statistically significant differences were observed at 40 and 60 seconds. (B) Mice treated with 1,1-DEE showed direct swimming paths toward the platform, whereas control mice exhibited irregular and random paths. (C) Comparison of swim path lengths among mice that successfully located the platform showed that the 1,1-DEE treatment group traveled a significantly shorter distance (average 146.3 cm) compared to the control group (average 820.8 cm) (p < 0.01, t-test). All data are presented as mean ± standard error (SE).

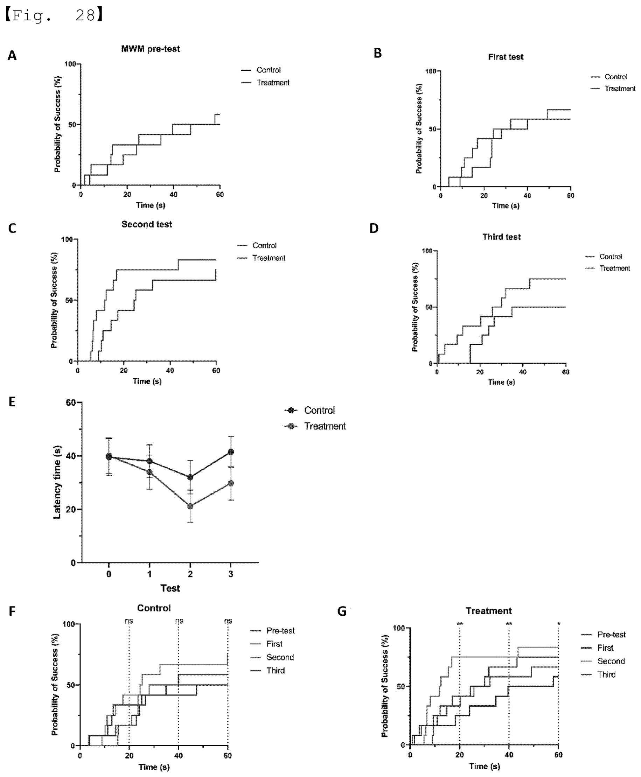

Figure 28 shows that 1,1-DEE can enhance learning and memory in normal mice. (A-D) Time-to-event curves represent the cumulative proportion of mice reaching the platform across different testing sessions: pre-test (A), post-first administration (B), post-second administration (C), and post-third administration (D). Although differences between the 1,1-DEE treatment group and control group were not statistically significant in each session (p > 0.05), the 1,1-DEE-treated mice generally reached the platform faster after repeated administrations. (E) Comparison of average escape times across the pre-test and three subsequent test sessions showed that both groups had similar baseline performance. However, the 1,1-DEE treatment group exhibited a gradual reduction in escape time with repeated dosing, whereas the control group showed no significant change. The difference between groups was not statistically significant (p > 0.05). (F) The control group maintained consistent performance across all sessions without significant changes in the time-to-event curves (p > 0.05). (G) In contrast, the 1,1-DEE treatment group demonstrated significant improvements in performance across test sessions. Specifically, during the second test, escape latency was significantly reduced compared to the pre-test (p < 0.01), and further significant improvement was observed in the third test (p < 0.05).

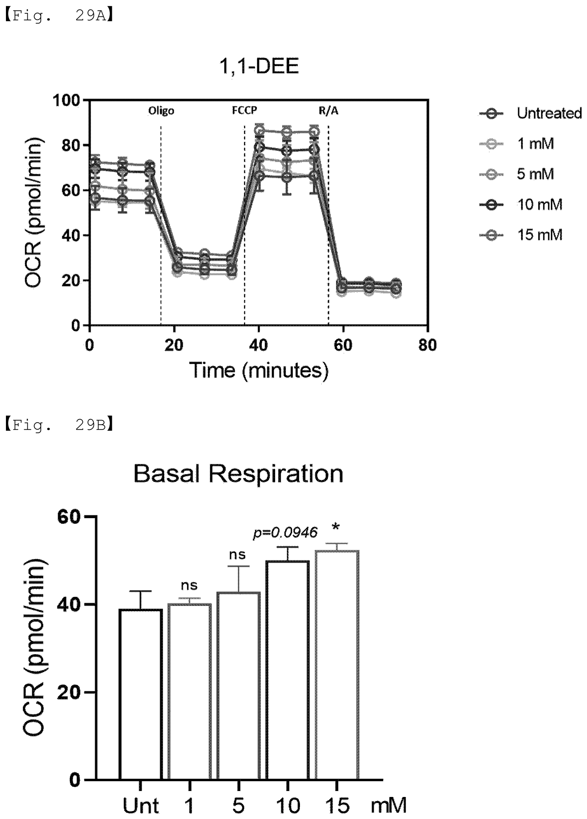

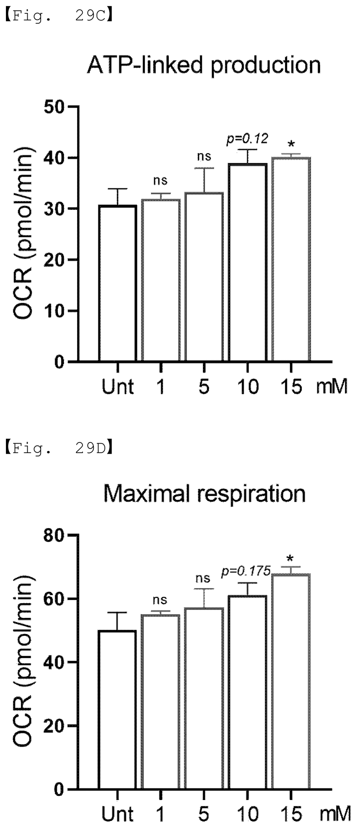

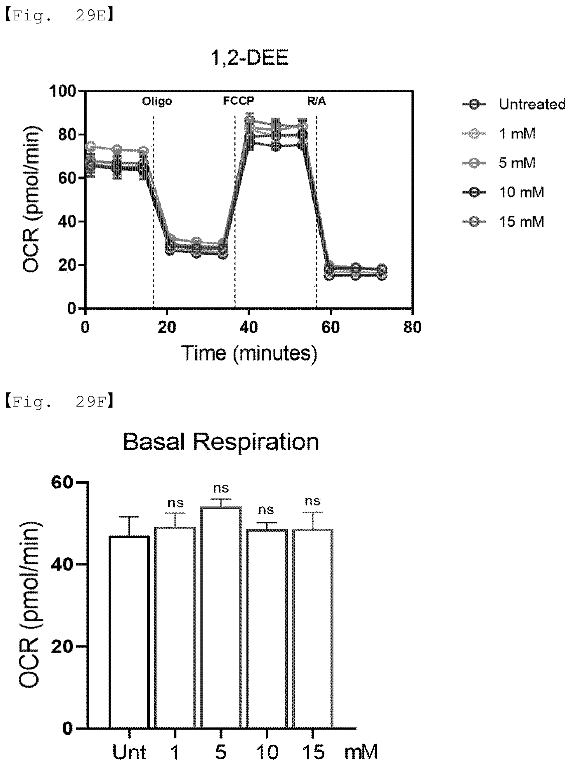

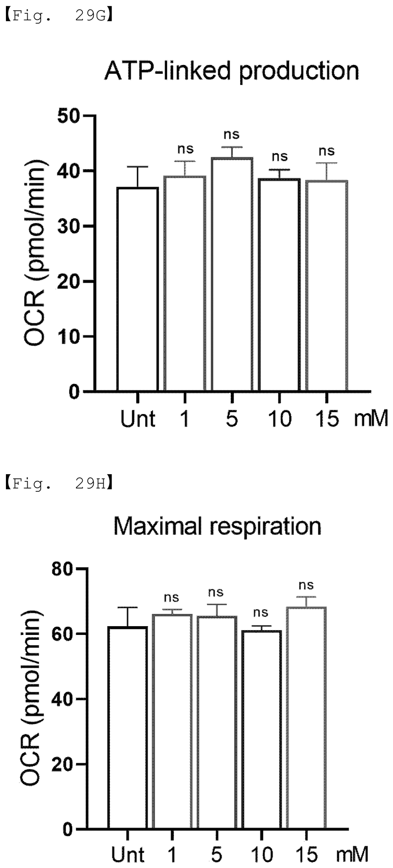

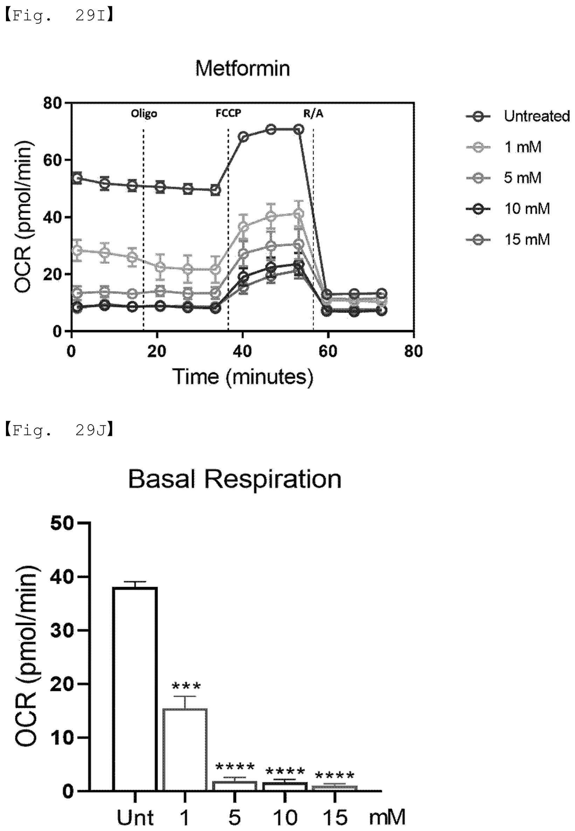

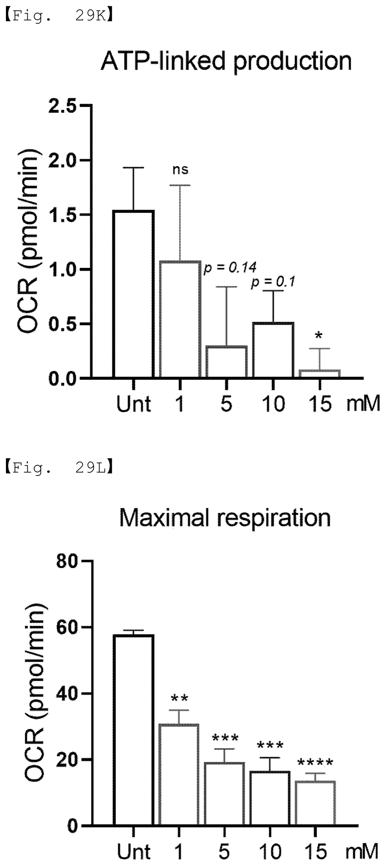

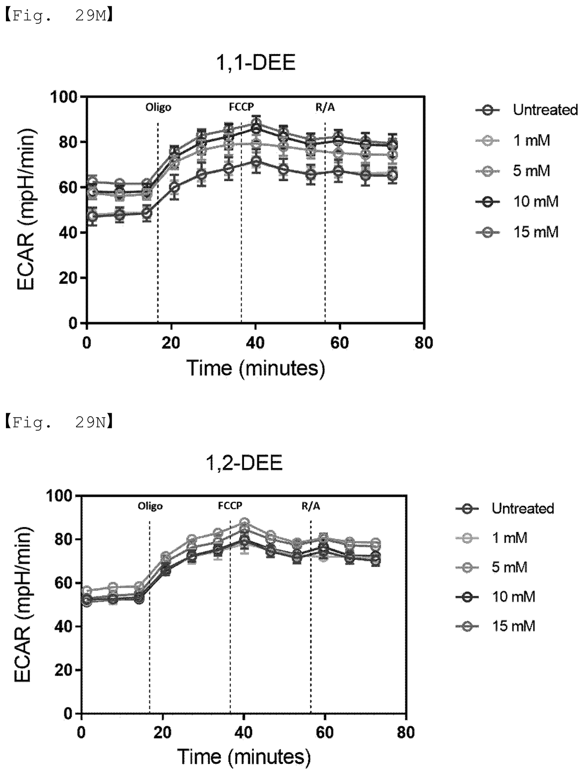

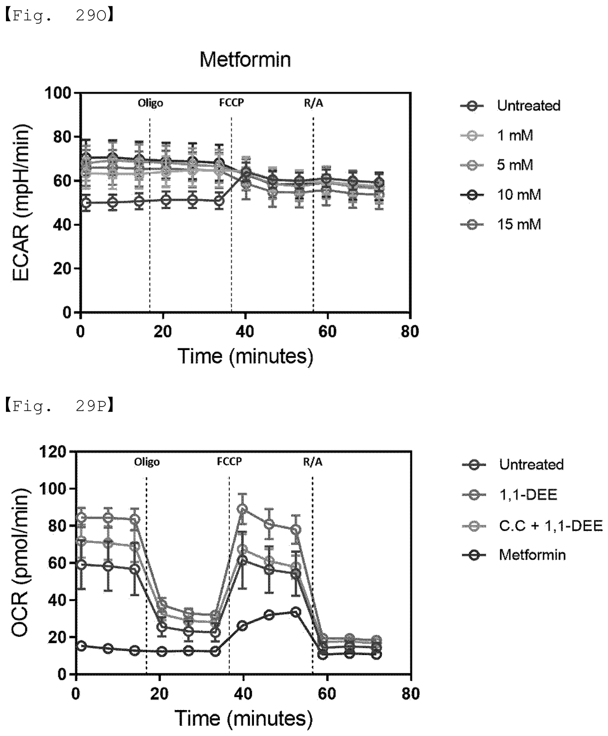

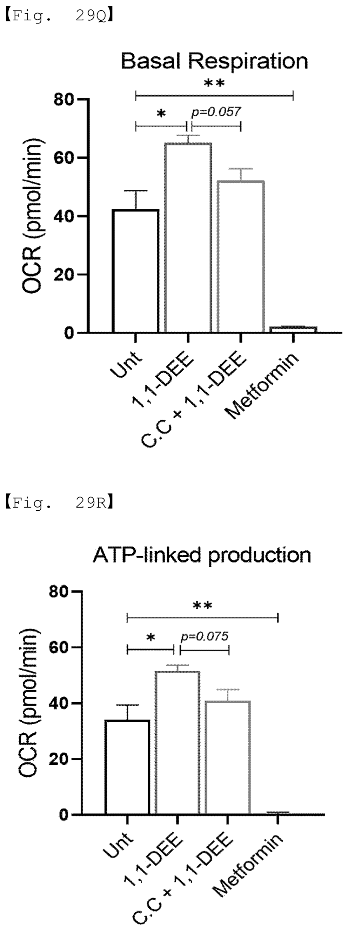

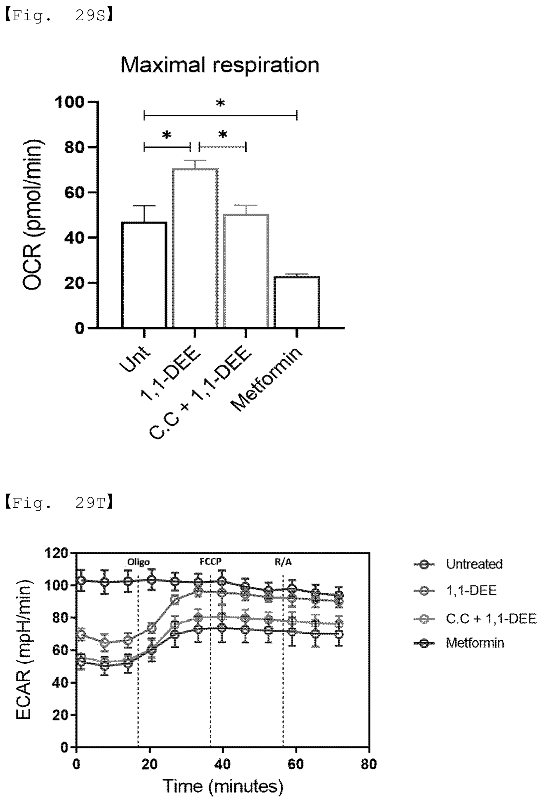

Figure 29 illustrates the effects of long-term 1,1-DEE treatment on mitochondrial respiration and glycolysis via AMPK activation. (A-O) AC16 cells were treated with various concentrations of 1,1-DEE, 1,2-DEE, and metformin for 8 hours. Mitochondrial respiration was assessed using the Agilent Seahorse XF96 Cell Mito Stress Test Kit. Measurements included: Oxygen consumption rate (OCR): panels A, E, I; Basal respiration: panels B, F, J; ATP-linked production: panels C, G, K; Maximal respiration: panels D, H, L; Glycolysis was evaluated after treatment with 1,1-DEE (M), 1,2-DEE (N), and metformin (O). (P-T) AC16 cells were treated under three conditions for 8 hours: 1,1-DEE (15 mM), Compound C (10 µM, 4-hour pretreatment) + 1,1-DEE (15 mM), Metformin (15 mM). Measured parameters included: Mitochondrial OCR (P), Basal respiration (Q), ATP-linked production (R), Maximal respiration (S), Extracellular acidification rate (ECAR, T), Data represent mean ± SEM from three independent experiments for 1,1-DEE, and 2-3 independent experiments for 1,2-DEE and metformin. Statistical significance was calculated using multiple t-tests (nsp > 0.05, *p ≤ 0.05, **p ≤ 0.01, ***p ≤ 0.001, ****p ≤ 0.0001).

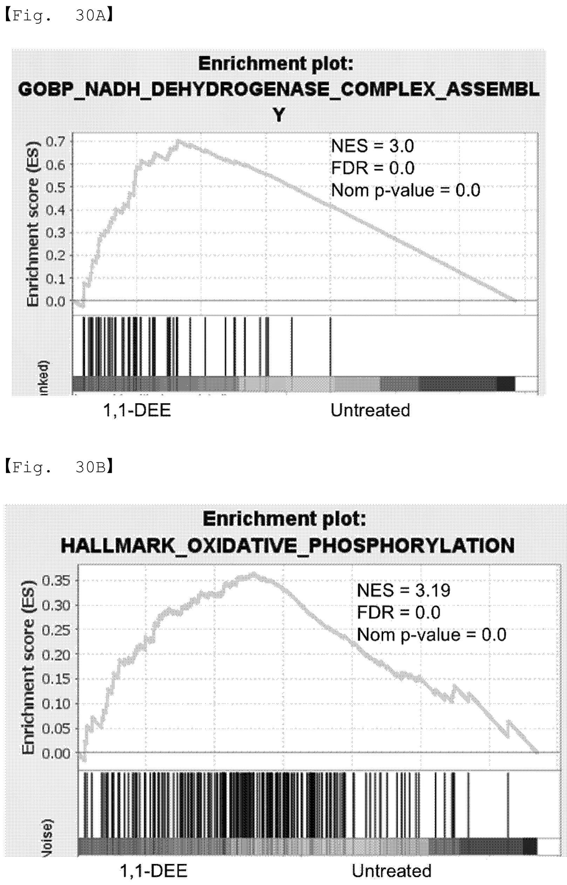

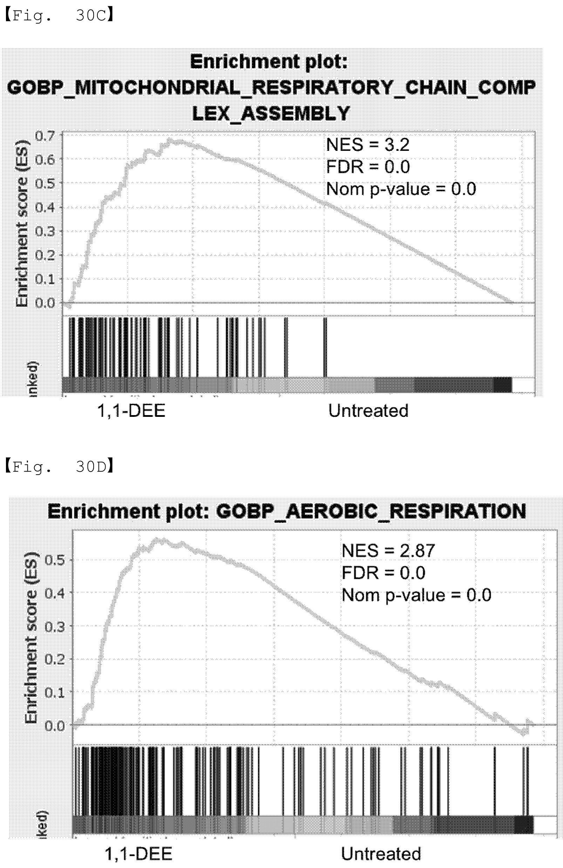

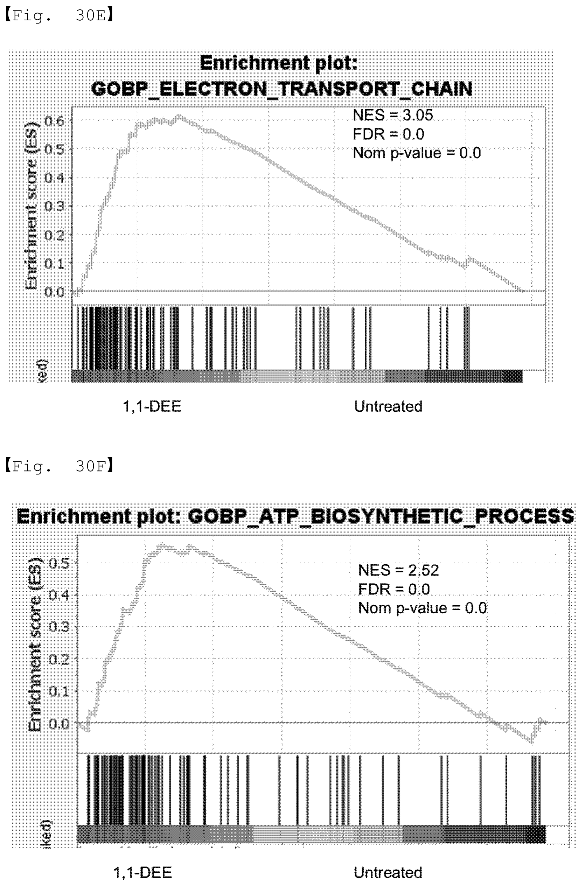

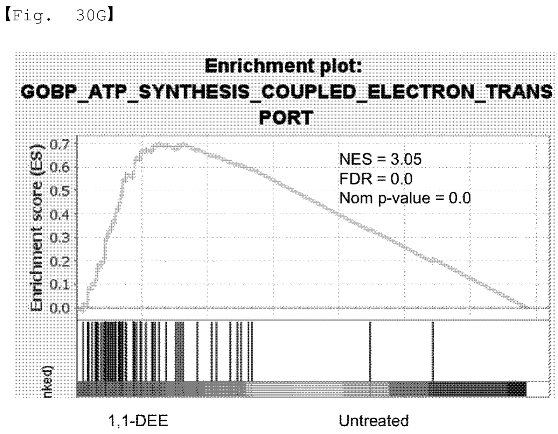

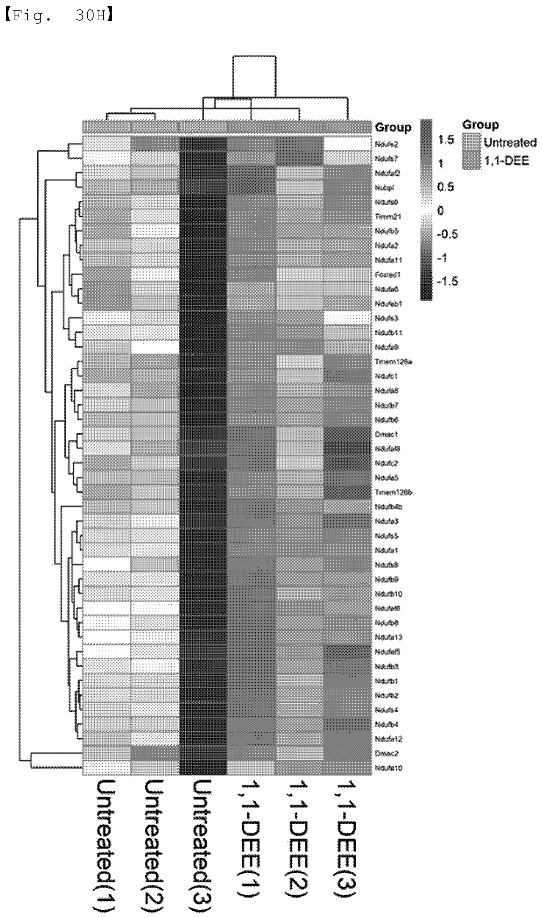

Figure 30 shows the evaluation of mitochondrial function improvement in C57BL/6J mice following 1,1-DEE treatment. Eight-week-old C57BL/6J mice were administered 1,1-DEE for 8 weeks (n = 3). RNA-seq analysis was performed using the livers of the mice. (A-G) GSEA preranked analysis was conducted to evaluate biological processes (GOBP) enriched in the 1,1-DEE-treated group. The evaluated processes included "GOBP NADH dehydrogenase complex assembly" (A), "GOBP oxidative phosphorylation" (B), "GOBP mitochondrial respiratory chain complex assembly" (C), "GOBP aerobic respiration" (D), "GOBP aerobic electron transport chain" (E), "GOBP ATP biosynthetic process" (F), and "GOBP electron transport coupled to ATP synthesis" (G), which were not enriched in the untreated group. (H) The heatmap shows the differences in the expression of key genes between the untreated group and the 1,1-DEE-treated group.

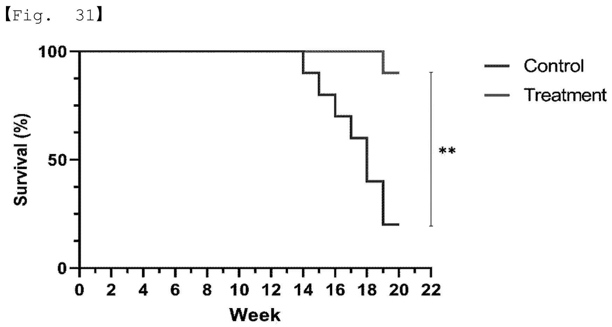

Figure 31 shows the survival curves of rats injected with 1,1-DEE (300 µl) or normal saline (NS, 300 µl) according to the present invention.

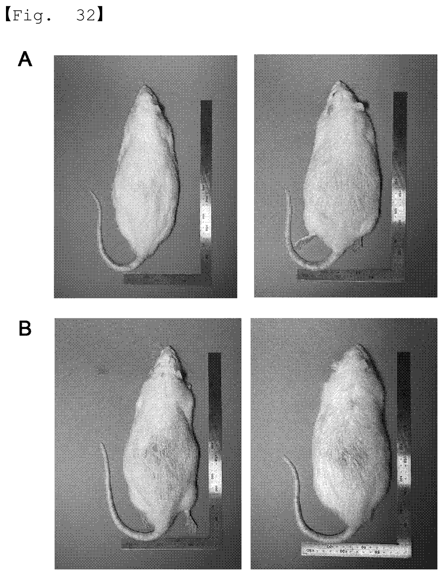

Figure 32 presents photographs to assess health indicators (such as fur condition, body weight, and activity) of rats injected with 1,1-DEE (300 µl) or normal saline (NS, 300 µl) according to the present invention. (A) Rats administered 1,1-DEE (left: 688 g, right: 796 g). (B) Rats administered normal saline (NS) (left: 620 g, right: 781 g).

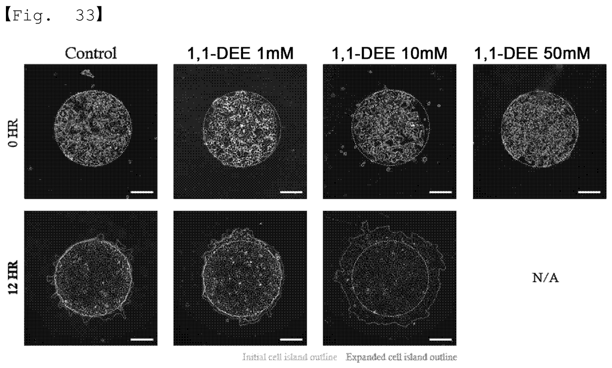

Figure 33 shows the comparison of cell migration patterns at 0 HR (immediately after stencil removal) and 12 HR post-treatment for groups treated with different concentrations of 1,1-DEE (1 mM, 10 mM, 50 mM) according to the present invention.

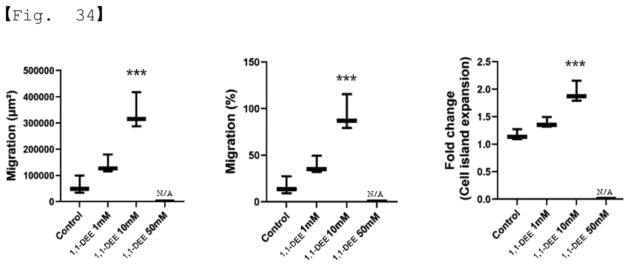

Figure 34 shows the quantification results of migration (µm²), migration percentage (%), and cell island expansion (fold change) for groups treated with different concentrations of 1,1-DEE (1 mM, 10 mM, 50 mM) according to the present invention.

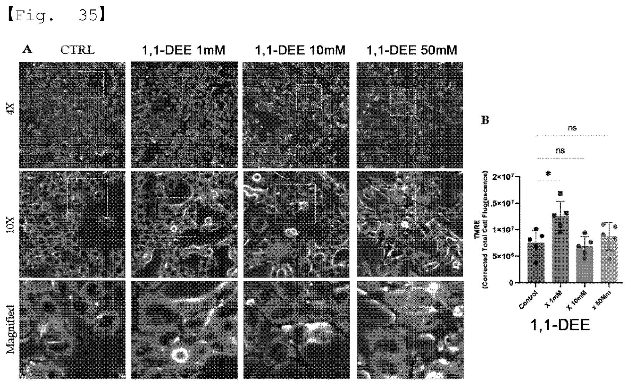

Figure 35 shows the results of TMRE metabolic dye staining (A) for groups treated with different concentrations of 1,1-DEE (1 mM, 10 mM, 50 mM) according to the present invention, and the corresponding quantification of CTCF (Corrected Total Cell Fluorescence) from these images (B) .

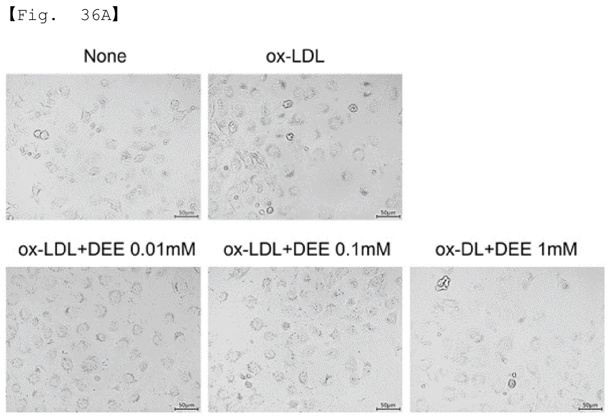

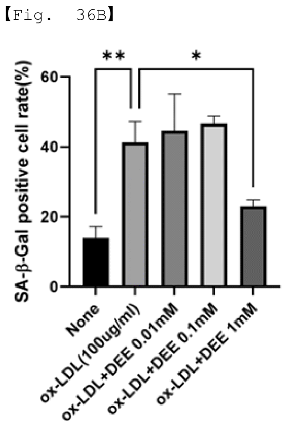

Figure 36 shows the results of SA-β-gal staining performed to evaluate cellular senescence in EA.hy926 endothelial cells pretreated with 1,1-DEE for 8 hours followed by exposure to ox-LDL for 48 hours. Panel (A) presents representative microscopic images acquired at 200× magnification, and Panel (B) illustrates the quantified percentage of SA-β-gal-positive cells. The data are expressed as the mean ± SD from at least three independent experiments, with statistical significance indicated as *P < 0.05 and P < 0.01.

### [DETAILED DESCRIPTION OF THE EXEMPLARY EMBODIMENTS]

Hereinafter, a mitochondrial biosynthesis promoter containing 1,1-diethoxydiethyl (1,1-DEE) as an active ingredient and its use are described in detail. However, this is presented merely as one example of the invention, and the scope of the invention is not limited thereby. It will be apparent to those skilled in the art that various modifications of the embodiments are possible within the scope of the claims of the invention. Unless otherwise specifically stated in the present specification, the terms "comprising" or "containing" refer to the inclusion of certain components (or constituents) without particular limitation, and should not be construed as excluding the presence of other components (or constituents).

As used herein, the term "treatment" refers to any form of therapy or prevention administered to a subject who has a disease or is at risk of developing a disease, and provides effects such as improvement of the subject's condition, delay in disease progression, delay in symptom onset, or slowing of symptom progression. Accordingly, the term "treatment" encompasses prophylactic treatment to prevent the occurrence of symptoms. In addition, the terms "treatment" and "prevention" are not intended to imply the complete cure or elimination of symptoms.

As used herein, the term "improvement" may refer to any action that alleviates a condition or reduces at least the severity of symptoms or parameters related to treatment.

As used herein, the term "subject" refers to an animal, including, but not limited to, cattle, monkeys, horses, sheep, pigs, chickens, turkeys, quails, cats, dogs, mice, rats, rabbits, or guinea pigs. For example, the subject may be a mammal, and particularly, a human.

As used herein, the terms "mitochondrial biosynthesis" and "mitochondrial biogenesis" are used interchangeably. Although "biogenesis" is the technically preferred term for the formation of new mitochondria as organelles, in the context of this specification, both terms are intended to encompass the induction of mitochondrial formation and functional enhancement.

### 1. Mitochondrial Biosynthesis Promoter

The present invention provides a mitochondrial biosynthesis promoter comprising 1,1-diethoxyethane (1,1-DEE) as an active ingredient.

In the mitochondrial biosynthesis promoter according to the present invention, 1,1-diethoxyethane may increase the expression of peroxisome proliferator-activated receptor gamma coactivator-1 alpha (PGC-1α) to promote mitochondrial biosynthesis.

In the mitochondrial biosynthesis promoter according to the present invention, 1,1-diethoxyethane may increase the transcriptional activities of nuclear respiratory factor 1 (Nrf1) and nuclear respiratory factor 2 (Nrf2), which in turn stimulate the expression of mitochondrial transcription factor A (Tfam) to promote mitochondrial biosynthesis.

In the mitochondrial biosynthesis promoter according to the present invention, 1,1-diethoxyethane may promote an aerobic respiration.

In the mitochondrial biosynthesis promoter according to the present invention, the mitochondrial biosynthesis promoter is used for ischemic brain diseases, ischemic heart diseases, pulmonary embolism, ischemic-reperfusion injury, stroke, ischemic stroke, migraine, anemia, Parkinson's disease (PD), dementia, Alzheimer's disease (AD), Huntington's disease, Frontotemporal Dementia, amyotrophic lateral sclerosis (ALS), Leigh syndrome, mitochondrial myopathy, mitochondrial encephalomyopathy, Lactic Acidosis, and Stroke-like episodes (MELAS), cirrhosis, Fanconi syndrome, atypical phenylketonuria, asthma, chronic obstructive pulmonary disease (COPD), diabetes, atherosclerosis, heart failure, hypertension, metabolic dysfunction, metabolic dysfunction-associated steatotic liver disease (MASLD), insulin resistance, hypercholesterolemia, optic nerve diseases, Duchenne muscular dystrophy, myopathies, obesity, depression, Osteoarthritis, , cancer, cancer cachexia, inflammatory diseases, age-related diseases, and cardiovascular diseases, cancer, cognitive ability or memory. According to one exemplary embodiment, the mitochondrial biosynthesis promoter may also be used as a hypoglycemic agent, a cholesterol-lowering agent or a fatty acid-lowering agent.

In the mitochondrial biosynthesis promoter according to the present invention, the mitochondrial biosynthesis promoter may be used for the preparation of a composition for preventing or treating age-related diseases, and for the preparation of a composition for delaying aging or extending lifespan.

In the mitochondrial biosynthesis promoter according to the present invention, the mitochondrial biosynthesis promoter may be used for the preparation of a composition for preventing, improving, or treating cardiovascular diseases.

In the mitochondrial biosynthesis promoter according to the present invention, the mitochondrial biosynthesis promoter may be used for the preparation of a composition for preventing, improving, or treating cancer and cancer-associated cachexia.

In the mitochondrial biosynthesis promoter according to the present invention, the mitochondrial biosynthesis promoter may be used for the preparation of a composition for preventing, improving, or treating stroke.

In the mitochondrial biosynthesis promoter according to the present invention, the mitochondrial biosynthesis promoter may be used for the preparation of a composition for preventing, improving, or treating dementia.

In the mitochondrial biosynthesis promoter according to the present invention, the mitochondrial biosynthesis promoter may be used for the preparation of a composition for preventing, improving, or treating cognitive decline or memory impairment.

In the mitochondrial biosynthesis promoter according to the present invention, the mitochondrial biosynthesis promoter may be used for the preparation of a composition for enhancing immune function, improving physical fitness, increasing physical endurance, raising energy levels, promoting vitality, enhancing physical resilience, or supporting sustained physical activity.

In the mitochondrial biosynthesis promoter according to the present invention, the mitochondrial biosynthesis promoter may also be used as a promoter for (stem) cell differentiation and maturation.

In the mitochondrial biosynthesis promoter according to the present invention, the concentration of 1,1-diethoxyethane may range from 1 mM to 25 mM.

### 2. Use of the Mitochondrial Biosynthesis Promoter

The present invention provides a composition for preventing, treating, or improving diseases that require mitochondrial biosynthesis promotion, comprising 1,1-diethoxyethane (1,1-DEE) as an active ingredient. Such a composition may be formulated as a pharmaceutical composition, a cosmetic composition, a food composition, or a feed composition.

### 2.1. Diseases Requiring Mitochondrial Biosynthesis Promotion

According to one exemplary embodiment, the present invention provides a composition for preventing, improving, or treating diseases that require mitochondrial biosynthesis promotion, comprising 1,1-diethoxyethane (1,1-DEE) as an active ingredient.

In the composition according to the present invention, diseases that require mitochondrial biosynthesis promotion may be one or more diseases selected from the group consisting of ischemic brain diseases, ischemic heart diseases, pulmonary embolism, ischemic-reperfusion injury, stroke, ischemic stroke, migraine, anemia, Parkinson's disease (PD), dementia, Alzheimer's disease (AD), Huntington's disease, Frontotemporal Dementia, amyotrophic lateral sclerosis (ALS), Leigh syndrome, mitochondrial myopathy, mitochondrial encephalomyopathy, Lactic Acidosis, and Stroke-like episodes (MELAS), cirrhosis, Fanconi syndrome, atypical phenylketonuria, asthma, chronic obstructive pulmonary disease (COPD), diabetes, atherosclerosis, heart failure, hypertension, metabolic dysfunction, metabolic dysfunction-associated steatotic liver disease (MASLD), insulin resistance, hypercholesterolemia, optic nerve diseases, Duchenne muscular dystrophy, myopathies, obesity, depression, Osteoarthritis, , cancer, cancer cachexia, inflammatory diseases, age-related diseases, and cardiovascular diseases, cancer, cognitive ability or memory

### (1) Delay of Aging or Extension of Lifespan

As used herein, the term *"aging"* refers to a phenomenon accompanied by various physiological changes, such as decreased cellular differentiation and proliferation, degeneration of cellular function, reduced homeostasis of organs and organ systems, and increased susceptibility to external stress and diseases as time passes after the birth of an organism. The term *"age-related disease"* refers to a disease that occurs more frequently as aging progresses. In the present invention, delaying the aging of an organism means reducing the rate of aging that naturally occurs over time compared with a control group, i.e., an organism that has not been treated or administered with the composition according to one embodiment of the present invention, thereby causing aging to proceed more slowly.

As used herein, the term *"extension of lifespan"* refers to prolonging the lifespan of an organism beyond its natural lifespan. For example, the *"extension of lifespan"* may refer to prolonging the lifespan of an organism that is determined by innate or acquired factors such as genetic factors. Specifically, the *"extension of lifespan"* may involve appropriately controlling lifespan-regulating factors arising from genetic factors of an organism, such as the expression level of related genes or the production level of related proteins, to extend the lifespan of the organism. It also includes extending the lifespan by appropriately controlling lifespan-regulating factors arising from acquired factors such as environmental influences. For example, such extension of lifespan may refer to prolonging the natural time of death of a living organism. In the present invention, extending the lifespan of an organism means enabling the organism to survive for a longer period of time compared with a control group, i.e., an organism that has not been treated or administered with the composition according to one embodiment of the present invention.

According to one exemplary embodiment, a composition for preventing or treating age-related diseases and for delaying aging or extending lifespan, comprising 1,1-diethoxyethane (1,1-DEE) as an active ingredient, is disclosed.

In the present invention, the age-related disease may be one or more diseases selected from the group consisting of Alzheimer's disease, diabetes, Parkinson's disease, Huntington's disease, degenerative joint disease, stroke, angina pectoris, osteoporosis, myocardial infarction, muscular dystrophy, amyotrophic lateral sclerosis (ALS), sarcopenia, liver cirrhosis, chronic kidney disease, macular degeneration, heart failure, senile depression, degenerative disc disease, spinal stenosis, metabolic syndrome, and pulmonary function decline.

In the present invention, the age-related disease may be a cognitive dysfunction disease caused by aging, and the cognitive function may specifically include one or more selected from the group consisting of perception, memory, attention, speech comprehension, speech generation, reading comprehension, creation of imagery, learning, and reasoning.

In the present invention, when the disease requiring mitochondrial biosynthesis promotion is an age-related disease, 1,1-diethoxyethane (1,1-DEE) may be added at a concentration of 100 µM or more and less than 20 mM. Preferably, the 1,1-diethoxyethane may be added at a concentration of 1 mM or more and 10 mM or less. When the concentration of 1,1-diethoxyethane is less than 100 µM, effective results may not be obtained, and when it is 20 mM or higher, development may be inhibited.

In one example, 1,1-diethoxyethane (1,1-DEE) according to the present invention exhibited a lifespan-extending effect in a *Caenorhabditis elegans* model without inhibiting development. Therefore, 1,1-diethoxyethane (1,1-DEE) according to the present invention is expected to be useful in pharmaceutical compositions, cosmetic compositions, food compositions, health functional food compositions, or feed compositions for delaying aging or extending lifespan.

### (2) Cardiovascular Diseases

According to one exemplary embodiment, a composition for preventing, improving, or treating cardiovascular diseases comprising 1,1-diethoxyethane (1,1-DEE) as an active ingredient is disclosed.

In the present invention, the pharmaceutical composition may decrease the contractile force (twitch force) of cardiomyocytes.

In the present invention, the pharmaceutical composition may temporarily and rapidly increase heart rate. For example, the pharmaceutical composition may rapidly increase heart rate for 10 to 60 minutes after administration.

In the present invention, the cardiovascular disease may be one or more diseases selected from the group consisting of hypertension, arrhythmia, heart failure, cardiac arrest, myocardial infarction, atherosclerosis, angina pectoris, stroke, cerebral hemorrhage, hemangioma, coronary artery disease, aortic disease, cerebrovascular disease, ischemic disease, pulmonary embolism, peripheral vascular disease, and cardiovascular diseases requiring cardiac stimulation. In the pharmaceutical composition according to the present invention, the cardiovascular diseases requiring cardiac stimulation may be one or more diseases selected from the group consisting of bradycardia, bradyarrhythmia, bradycardic angina, heart failure, neurogenic syncope, hypotensive shock, and hypothyroidism.

In the present invention, the bradycardia may be one or more selected from the group consisting of transient bradycardia, vasovagal bradycardia, beta-blocker overdose-induced bradycardia, bradycardia during anesthesia induction, bradycardia post-cardiac arrest, and bradycardia due to neurogenic shock, but is not limited thereto.

In the present invention, the bradyarrhythmia may include sick sinus syndrome (SSS) or atrioventricular block (AV block), but is not limited thereto.

In the present invention, the hypotensive shock may include cardiogenic shock or septic shock, but is not limited thereto.

In one example, since 1,1-diethoxyethane (1,1-DEE) according to the present invention can temporarily and rapidly increase heart rate, it may be effectively used for the prevention, treatment, or improvement of cardiovascular diseases. The composition of the present invention may be applied not only for pharmaceutical purposes but also as a functional food or animal feed, thereby offering diverse application possibilities. For example, when used as a functional food, it may assist in health management for individuals requiring heart rate regulation. When applied as an animal feed, it may contribute to the regulation of abnormal heart rate in livestock or companion animals. Therefore, the present invention provides a novel therapeutic approach utilizing 1,1-DEE to safely and effectively increase heart rate, and offers an innovative technology that can contribute to the prevention and treatment of cardiovascular diseases requiring cardiac stimulation.

### (3) Cancer

According to one exemplary embodiment, a composition for preventing, improving, or treating cancer comprising 1,1-diethoxyethane (1,1-DEE) as an active ingredient is disclosed

In the present invention, the cancer may be selected from the group consisting of pancreatic cancer, lung cancer, mesothelioma, stomach cancer, esophageal cancer, liver cancer, biliary tract cancer, bladder cancer, head and neck cancer, oral cancer, nasopharyngeal cancer, adult brain cancer, colon cancer, rectum cancer, colorectal cancer, prostate cancer, ovarian cancer, cervical cancer, uterine cancer, testicular cancer, lymphoma, leukemia, skin cancer, breast cancer, kidney cancer, neuroblastoma, Merkel cell carcinoma, myelodysplastic syndrome, myelofibrosis, and multiple myeloma.

In the present invention, 1,1-diethoxyethane may activate adenosine monophosphate-activated protein kinase (AMPK) .

In the present invention, 1,1-diethoxyethane may induce apoptosis.

In the present invention, 1,1-diethoxyethane may prevent or treat cachexia derived from cancer.

In one example, 1,1-diethoxyethane (1,1-DEE) according to the present invention may exhibit anticancer effects by significantly reducing cancer cell viability and inducing apoptosis, and may provide a novel anticancer mechanism based on the regulation of energy metabolism through activation of the AMPK pathway. Furthermore, in vivo experiments confirmed the tumor growth inhibitory effect, and additional therapeutic benefits such as prevention of cancer-associated cachexia and improvement of survival rate were also observed. In addition, unlike 1,2-diethoxyethane (1,2-DEE), 1,1-DEE selectively exhibits its effects, and thus can be utilized as an excellent anticancer candidate substance in terms of both safety and efficacy. Therefore, the composition comprising 1,1-diethoxyethane (1,1-DEE) according to the present invention is expected to be useful as a pharmaceutical composition, food composition, cosmetic composition, or feed composition for preventing, treating, or improving cancer and cancer-associated cachexia.

### (4) Stroke

As used herein, the term "stroke" refers to a condition characterized by a sudden onset of localized neurological deficits caused by abnormalities in cerebral blood flow. Such stroke may include cerebral infarction, in which neuronal death is induced due to blockage of cerebral blood vessels by thrombi or the like, and cerebral hemorrhage, which occurs due to rupture of cerebral blood vessels. Specific symptoms of stroke may include sudden headache and vomiting, hemiplegia or partial paralysis of the body, sensory loss or numbness in certain body parts, language disorders (aphasia or dysarthria), facial nerve palsy, and ataxia. In the present invention, stroke may also refer to what is commonly known as apoplexy.

According to one exemplary embodiment, a composition for preventing, improving, or treating stroke comprising 1,1-diethoxyethane (1,1-DEE) as an active ingredient is disclosed.

In the present invention, the stroke may be an ischemic stroke or a hemorrhagic stroke.

In the present invention, the composition may promote recovery of motor function impaired by stroke or may reduce the infarct size.

In one example, 1,1-diethoxyethane (1,1-DEE) according to the present invention exhibited therapeutic effects in an ischemic stroke (mouse MCAO model) by improving survival rate through reduced mortality, enhancing general condition through body weight recovery, improving spatial cognition and memory, and reducing brain injury through neuroprotection. These results suggest that 1,1-diethoxyethane (1,1-DEE) may be an effective therapeutic agent for neuroprotection and cognitive recovery in ischemic stroke.

### (5) Dementia

According to one exemplary embodiment, a composition for preventing, improving, or treating dementia comprising 1,1-diethoxyethane (1,1-DEE) as an active ingredient is disclosed.

In the present invention, the dementia may be vascular dementia, Alzheimer's disease, dementia with Lewy bodies, or frontotemporal dementia (FTD).

In one example, 1,1-diethoxyethane (1,1-DEE) according to the present invention exhibited effects such as an increased success rate in reaching the platform, improved cognitive function during the early stages of learning, acquisition of more efficient swimming paths, and reduced swimming distance in a mouse model of ischemic stroke (MCAO-induced model). Notably, considering that the improvement in cognitive function was induced independently of motor ability and that MRI analysis confirmed the reduction of brain lesion areas, 1,1-DEE suggests potential as an effective therapeutic agent for cognitive recovery and neuroprotection following ischemic brain injury.

### (6) Cognitive Ability or Memory

According to one exemplary embodiment, a composition for preventing, improving, or treating cognitive or memory decline, or a composition for enhancing cognitive ability or memory, comprising 1,1-diethoxyethane (1,1-DEE) as an active ingredient, is disclosed.

In one example, 1,1-diethoxyethane (1,1-DEE) according to the present invention, when repeatedly administered to normal mouse models, induced an increased success rate in reaching the platform and a reduced average latency time in the Morris Water Maze test, thereby demonstrating improvements in spatial cognition and memory. In particular, although there was no difference in initial performance between groups, a significant improvement was observed after repeated administration, suggesting that 1,1-DEE may serve as an agent capable of enhancing cognitive function even in normal individuals.

### 2.2. Embodiments of the Composition

### (1) Pharmaceutical Composition

In the pharmaceutical composition according to the present invention, the composition may be administered via oral administration, intravenous injection, subcutaneous injection, intramuscular injection, intraperitoneal injection, epithelial administration, topical administration, vaginal administration, pulmonary administration, rectal administration, sublingual administration, buccal administration, transdermal administration, ocular administration, inhalation, intracavernosal injection, intrathecal injection, epidural injection, or rectal administration, among others. For oral administration, for example, the pharmaceutical composition may be formulated as a tablet or may be coated or formulated to protect the active ingredient from degradation in the stomach. In addition, the composition may be administered by any device that allows the active substance to be delivered to target cells. The route of administration may vary depending on the general condition and age of the subject, the nature of the condition to be treated, and the choice of active ingredient.

In the pharmaceutical composition according to the present invention, the pharmaceutical composition may be carried on a delivery vehicle. The delivery vehicle may include, but is not limited to, one or more selected from the group consisting of viral particles, vesicles, nanoparticles, microparticles, liposomes, transposons, micelles, antibodies, and exosomes.

In the pharmaceutical composition according to the present invention, the suitable dosage of the pharmaceutical composition may vary depending on factors such as the method of formulation, mode of administration, age, body weight, sex, pathological condition, food intake, time of administration, route of administration, rate of excretion, and responsiveness of the patient. A person skilled in the art can readily determine and prescribe an effective dose for the desired therapeutic or preventive effect. For example, the pharmaceutical composition may be administered as a single dose or in multiple doses, and may be administered one to four times per day. In one example, the pharmaceutical composition may be administered in an amount of 0.01 mg/kg to 100 mg/kg, preferably 0.02 mg/kg to 90 mg/kg, and more preferably 0.03 mg/kg to 80 mg/kg, based on an adult human subject.

In the pharmaceutical composition according to the present invention, the pharmaceutical composition may be prepared in a unit dosage form or contained within a multidose container by formulation using pharmaceutically acceptable carriers and/or excipients according to methods easily implementable by those skilled in the art. The formulation may be in the form of a solution, suspension, or emulsion in an oil or aqueous medium, or in the form of an extract, powder, granule, tablet, or capsule, and may further include a dispersing agent or stabilizer. In addition, the pharmaceutical composition may be administered in the form of a suppository, spray, ointment, cream, gel, inhalant, or transdermal patch. Furthermore, the pharmaceutical composition may be formulated for administration to mammals, and more preferably, for administration to humans.

In the pharmaceutical composition according to the present invention, the pharmaceutically acceptable carrier may be solid or liquid, and may include one or more selected from the group consisting of excipients, antioxidants, buffers, bacteriostatic agents, dispersants, adsorbents, surfactants, binders, preservatives, disintegrants, sweeteners, flavoring agents, glidants, release controllers, humectants, stabilizers, suspending agents, and lubricants. Additionally, the pharmaceutically acceptable carrier may be selected from saline, sterile water, Ringer's solution, buffered saline, dextrose solution, maltodextrin solution, glycerol, ethanol, or mixtures thereof.

In one embodiment, a suitable filler may include sugars (e.g., dextrose, sucrose, maltose, and lactose), starches (e.g., corn starch), sugar alcohols (e.g., mannitol, sorbitol, maltitol, erythritol, and xylitol), starch hydrolysates (e.g., dextrin and maltodextrin), cellulose or cellulose derivatives (e.g., microcrystalline cellulose), or mixtures thereof, but is not limited thereto.

In one embodiment, a suitable binder may include povidone, copovidone, methylcellulose, hydroxyethylcellulose, hydroxypropylmethylcellulose, hydroxypropylcellulose, gelatin, gums, sucrose, starch, or mixtures thereof, but is not limited thereto.

In one embodiment, a suitable preservative may include benzoic acid, sodium benzoate, benzyl alcohol, butylated hydroxyanisole, butylated hydroxytoluene, chlorobutanol, gallates, hydroxybenzoates, EDTA, or mixtures thereof, but is not limited thereto.

In one embodiment, a suitable disintegrant may include sodium starch glycolate, cross-linked polyvinylpyrrolidone, cross-linked carboxymethylcellulose, starch, microcrystalline cellulose, or mixtures thereof, but is not limited thereto.

In one embodiment, a suitable sweetener may include sucralose, saccharin, sodium, potassium, or calcium saccharin, acesulfame potassium, sodium cyclamate, mannitol, fructose, sucrose, maltose, or mixtures thereof, but is not limited thereto.

In one embodiment, a suitable glidant may include silica, colloidal silicon dioxide, or talc, but is not limited thereto.

In one embodiment, a suitable lubricant may include long-chain fatty acids and their salts, such as magnesium stearate and stearic acid, talc, glyceryl wax, or mixtures thereof, but is not limited thereto.

### (2) Cosmetic Composition

In the cosmetic composition according to the present invention, the cosmetic composition may be used for skin regeneration, wrinkle improvement, or skin moisturization

In the cosmetic composition according to the present invention, the cosmetic composition may further comprise a dermatologically acceptable carrier. The dermatologically acceptable carrier may include purified water, oil, wax, fatty acid, fatty alcohol, fatty acid ester, surfactant, humectant, thickener, antioxidant, viscosity stabilizer, chelating agent, buffer, preservative, and lower alcohol, but is not limited thereto. The type and concentration thereof may vary and can be modified by those skilled in the art within the scope of the present invention.

In the cosmetic composition according to the present invention, the cosmetic composition may further comprise, in addition to the active ingredient of the present invention, functional substances such as whitening agents, moisturizers, anti-inflammatory agents, antibacterial agents, antifungal agents, vitamins, ultraviolet (UV) blockers, antibiotics, anti-acne agents, perfumes, or dyes, which can be included in the cosmetic composition of the present invention in amounts conventionally used in the cosmetic field. To enhance its functional effects, the cosmetic composition of the present invention may further contain one or more moisturizing active ingredients exhibiting the same or similar function as the composition of the present invention.

In the cosmetic composition according to the present invention, the cosmetic composition may be prepared in the form of a conventional emulsion formulation or solubilized formulation. Examples of cosmetics in the form of an emulsion formulation include nourishing lotions, creams, and essences, and an example of a cosmetic in the form of a solubilized formulation includes softening lotion. The cosmetic composition may be prepared in the form of a topically or systemically applicable adjunct conventionally used in the art by further comprising a dermatologically acceptable carrier or vehicle in addition to the active ingredient of the present invention. Suitable cosmetic formulations include, for example, a solution, gel, solid or paste anhydrous product, an emulsion obtained by dispersing an oil phase in an aqueous phase, suspension, microemulsion, microcapsule, microparticulate form, ionic (liposomal) or non-ionic vesicular dispersions, cream, skin lotion, powder, ointment, spray, or conceal stick. In addition, the cosmetic composition may also be prepared in the form of a foam or an aerosol composition further comprising a compressed propellant.

In the cosmetic composition according to the present invention, the cosmetic composition may be formulated in one or more forms selected from the group consisting of a solution, topical ointment, cream, foam, nourishing lotion, softening lotion, perfume, pack, softening water, emulsion, makeup base, essence, soap, liquid cleanser, bath additive, sunscreen cream, sun oil, suspension, emulsion, paste, gel, lotion, powder, soap, surfactant-containing cleanser, oil, powder foundation, emulsion foundation, wax foundation, patch, and spray.

### (3) Food Composition

As used herein, the term "food" refers to a natural product or processed product containing one or more nutrients, and preferably refers to a form that has undergone a certain degree of processing and is in a state suitable for direct consumption. In its conventional meaning, the term "food" may encompass foods, food additives, functional foods, and beverages.

As used herein, the terms "functional food" or "health-functional food" refer to a group of foods or food compositions to which added value has been imparted by physical, biochemical, biotechnological, or other methods, so that the functions of the respective food act or are expressed for a specific purpose. Such foods are designed and processed to sufficiently exhibit in vivo regulatory functions related to, for example, biological defense rhythm regulation, disease prevention, and recovery. Specifically, the functional food may be a health-functional food. The functional food may further comprise food-acceptable auxiliary additives and may additionally include suitable carriers, excipients, and diluents conventionally used in the production of functional foods. The types of the health supplements are not limited thereto, but may include, for example, powder, granule, tablet, capsule, or beverage forms.

In the food composition according to the present invention, the food composition may be used for improving fatigue or enhancing exercise performance. For example, the food composition may be used to prevent or ameliorate muscle-related conditions caused by physical fatigue, muscle fatigue, myalgia, reduced muscle function, muscle wasting, or muscle degeneration, as well as decreased agility or reduced endurance, but is not limited thereto.

In the food composition according to the present invention, the food may be selected from the group consisting of meat, sausage, bread, chocolate, candy, snacks, confectioneries, pizza, ramen, other noodles, gum, dairy products including ice cream, various soups, beverages, tea, coffee drinks, stamina drinks, alcoholic beverages, or vitamin complexes.

In the food composition according to the present invention, the food composition may further comprise various nutritional supplements, vitamins, electrolytes, flavoring agents, coloring agents, pectic acid and salts thereof, alginic acid and salts thereof, organic acids, protective colloid thickeners, pH adjusters, stabilizers, preservatives, glycerin, alcohol, carbonating agents used in carbonated beverages, and the like. In addition, the composition of the present invention may further comprise pulp for preparing natural fruit juices, fruit juice beverages, or vegetable beverages. These ingredients may be used independently or in combination.

In the food composition according to the present invention, the term "functional food" or "health-functional food" refers to a group of foods or food compositions to which added value has been imparted by physical, biochemical, biotechnological, or other methods, such that the functions of the respective food act or are expressed for a specific purpose. Such foods are designed and processed to sufficiently exhibit in vivo regulatory functions related to biological defense rhythm regulation, disease prevention, and recovery. Specifically, the functional food may be a health-functional food. The functional food may further comprise food-acceptable auxiliary additives and may additionally include suitable carriers, excipients, and diluents conventionally used in the production of functional foods.

### (4) Feed Composition

In the feed composition according to the present invention, the feed comprises nutrients required by animals, such as energy, protein, lipids, vitamins, and minerals, and may be plant-based feed including grains, root crops, food processing by-products, algae, fibers, oils, starches, seed meals, and grain by-products, or animal-based feed including proteins, minerals, oils, mineral substances, and single-cell proteins, but is not limited thereto.

In the feed composition according to the present invention, the feed may be in the form of powdered feed, solid feed, moist pellet feed, dry pellet feed, extruder pellet (EP) feed, or raw feed, but is not limited thereto.

In the feed composition according to the present invention, the feed composition may further comprise binders, emulsifiers, preservatives, or the like, which are added to prevent quality deterioration, and may further comprise feed additives. To enhance efficacy, the feed may additionally comprise amino acids, vitamins, enzymes, flavoring agents, non-protein nitrogen compounds, silicates, buffers, extracts, oligosaccharides, and the like. The feed composition may further comprise feed premixes or other additives, but is not limited thereto.

### 3. Method for Treating Diseases Requiring Mitochondrial Biosynthesis Promoters

The present invention provides a method for preventing or treating diseases requiring mitochondrial biosynthesis promotion, comprising administering to a subject a pharmaceutical composition comprising 1,1-diethoxyethane (1,1-DEE) as an active ingredient according to item 1. Mitochondrial Biosynthesis Promoter. According to an exemplary embodiment, the present invention provides a method for preventing or treating aging-related diseases, cardiovascular diseases, cancer, stroke, dementia, or decline in cognitive function or memory, comprising administering to a subject a pharmaceutical composition comprising 1,1-diethoxyethane (1,1-DEE) as an active ingredient according to item 1. Mitochondrial Biosynthesis Promoter.

In the method according to the present invention, the subject may be a human, cow, monkey, horse, sheep, pig, chicken, turkey, quail, cat, dog, mouse, rat, rabbit, or guinea pig, but is not limited thereto.

In the method according to the present invention, the route, dosage, and frequency of administration of the pharmaceutical composition may be varied according to the condition of the patient and the presence or absence of side effects, and the optimal route, dosage, and frequency of administration can be selected by a person of ordinary skill in the art within an appropriate range. In the present invention, the preferred dosage of the pharmaceutical composition may be in the range of 0.001 mg/kg to 100 mg/kg per day for an adult, depending on the condition, body weight, sex, age, severity of the patient, and route of administration. The administration may be performed once per day or divided into multiple administrations. Such dosage ranges should not be construed as limiting the scope of the present invention in any respect.

The present invention relates, *inter alia,* to the provision of mitochondrial biosynthesis promoter which comprises/is 1,1-diethoxyethane (1,1-DEE). Accordingly, the present invention relates to the provision of 1,1-DEE as a means to induce mitochondrial biosynthesis, specifically, mitochondrial biosynthesis in a cell.

Furthermore, the present invention relates to the provision of a pharmaceutical composition comprising the mitochondrial biosynthesis promoter according to the present invention (i.e. 1,1-DEE). The present invention also relates to the medical use of 1,1-DEE and the pharmaceutical composition comprising the mitochondrial biosynthesis promoter according to the present invention (i.e. 1,1-DEE), for the purposes disclosed herein.

According to the present invention and as shown in the appended non-limiting Examples, 1,1-DEE and/or the pharmaceutical composition comprising 1,1-DEE induce mitochondrial biosynthesis, in particular by:
i. increasing the expression of PGC-1α (peroxisome proliferator-activated receptor gamma coactivator-1 alpha); and/or
ii. enhancing the transcriptional activities of Nrf1 and Nrf2, which in turn stimulate the expression of Tfam.

Further, and as also shown in the appended non-limiting examples, 1,1-DEE promotes an aerobic respiration, i.e. aerobic respiration in mitochondria, such as mitochondria within a cell.

The mitochondrial biosynthesis promoter, i.e. 1,1-DEE, according to the present invention is of particular use in medical interventions directed at treatment of diseases that may be treated, ameliorated and/or prevented by inducing mitochondrial biosynthesis.

Accordingly, the present invention relates to the following pharmaceutical / medical use items, whereby a composition comprising the mitochondrial biosynthesis promoter according to the present invention is to be used:
1. A pharmaceutical composition comprising 1,1-diethoxyethane (1,1,-DEE)or use in the treatment of diseases that requires promotion of mitochondrial biosynthesis.
   As shown in the appended, non-limiting examples and disclosed hereinabove, 1,1-DEE promotes mitochondrial biosynthesis, in particular mitochondrial biosynthesis in a cell. The cell may be a human cell. Accordingly, the appended examples show that 1,1-DEE is capable of promoting mitochondrial biosynthesis, thereby ameliorating diseases, disorders and /or symptoms associated with reduced mitochondrial activity; e.g. aerobic respiration.
2. A pharmaceutical composition comprising 1,1-diethoxyethane (1,1,-DEE) for use in the treatment of a disease selected from the group consisting of ischemic brain diseases, ischemic heart diseases, pulmonary embolism, ischemic-reperfusion injury, stroke, ischemic stroke, migraine, anemia, Parkinson's disease (PD), dementia, Alzheimer's disease (AD), Huntington's disease, Frontotemporal Dementia, amyotrophic lateral sclerosis (ALS), Leigh syndrome, mitochondrial myopathy, mitochondrial encephalomyopathy, Lactic Acidosis, and Stroke-like episodes (MELAS), cirrhosis, Fanconi syndrome, atypical phenylketonuria, asthma, chronic obstructive pulmonary disease (COPD), diabetes, atherosclerosis, heart failure, hypertension, metabolic dysfunction, metabolic dysfunction-associated steatotic liver disease (MASLD), insulin resistance, hypercholesterolemia, optic nerve diseases, Duchenne muscular dystrophy, myopathies, obesity, depression, Osteoarthritis, , cancer, cancer cachexia, inflammatory diseases, age-related diseases, and cardiovascular diseases, cancer, cognitive ability or memory.
3. The pharmaceutical composition for according to item 1, wherein the disease that requires promotion of mitochondrial biosynthesis is selected from the group consisting of ischemic brain diseases, ischemic heart diseases, pulmonary embolism, ischemic-reperfusion injury, stroke, ischemic stroke, migraine, anemia, Parkinson's disease (PD), dementia, Alzheimer's disease (AD), Huntington's disease, Frontotemporal Dementia, amyotrophic lateral sclerosis (ALS), Leigh syndrome, mitochondrial myopathy, mitochondrial encephalomyopathy, Lactic Acidosis, and Stroke-like episodes (MELAS), cirrhosis, Fanconi syndrome, atypical phenylketonuria, asthma, chronic obstructive pulmonary disease (COPD), diabetes, atherosclerosis, heart failure, hypertension, metabolic dysfunction, metabolic dysfunction-associated steatotic liver disease (MASLD), insulin resistance, hypercholesterolemia, optic nerve diseases, Duchenne muscular dystrophy, myopathies, obesity, depression, Osteoarthritis, , cancer, cancer cachexia, inflammatory diseases, age-related diseases, and cardiovascular diseases, cancer, cognitive ability or memory.
4. A pharmaceutical composition comprising 1,1,-DEE for use in the treatment of a disease selected from the group consisting of a cardiovascular disease, stroke, cancer and a neurodegenerative disease, wherein the disease is further characterized by requiring promotion of mitochondrial biosynthesis.

The disease may be a cardiovascular disease selected from the group consisting of hypertension, arrhythmia, heart failure, cardiac arrest, myocardial infarction, atherosclerosis, angina pectoris, stroke, cerebral hemorrhage, hemangioma, coronary artery disease, aortic disease, cerebrovascular disease, ischemic disease, pulmonary embolism, peripheral vascular disease, and cardiovascular diseases requiring cardiac stimulation.
As shown in the appended non-limiting examples, 1,1-DEE or a pharmaceutical composition comprising 1,1-DEE is useful in the treatment of cardiovascular diseases, such as stroke. According to the present invention, said stroke may be ischemic stroke. Accordingly, the present invention relates to the use of 1,1-DEE or a pharmaceutical composition comprising 1,1-DEE in the treatment of stroke, such as ischemic stroke.

Further, the appended non-limiting examples show that treatment with 1,1-DEE or a pharmaceutical composition comprising 1,1-DEE improves the recovery of motor function after ischemic stroke in a subject. It is understood that such improvement may be determined as compared to a suitable reference, e.g. a subject which has also suffered a stroke but has not been treated with 1,1-DEE or the pharmaceutical composition comprising 1,1-DEE according to the present invention.

Additionally, 1,1-DEE or the pharmaceutical composition comprising 1,1-DEE according to the present invention are useful in the treatment of neurodegenerative disease, for example Parkinson's disease (PD), dementia, Alzheimer's disease (AD), Huntington's disease, Frontotemporal Dementia, amyotrophic lateral sclerosis (ALS), Leigh syndrome, mitochondrial myopathy, mitochondrial encephalomyopathy, Lactic Acidosis, and Stroke-like episodes (MELAS).
Accordingly, the present invention also relates to the use of 1,1-DEE or the pharmaceutical composition comprising 1,1-DEE according to the present invention in the treatment of a neurodegenerative, such as Parkinson's disease (PD), dementia, Alzheimer's disease (AD), Huntington's disease, Frontotemporal Dementia, amyotrophic lateral sclerosis (ALS), Leigh syndrome, mitochondrial myopathy, mitochondrial encephalomyopathy, Lactic Acidosis, and Stroke-like episodes (MELAS).

Furthermore, 1,1-DEE or the pharmaceutical composition comprising 1,1-DEE according to the present invention may be used in the treatment of cancer, such as pancreatic cancer, lung cancer, mesothelioma, stomach cancer, esophageal cancer, liver cancer, biliary tract cancer, bladder cancer, head and neck cancer, oral cancer, nasopharyngeal cancer, adult brain cancer, colon cancer, rectum cancer, colorectal cancer, prostate cancer, ovarian cancer, cervical cancer, uterine cancer, testicular cancer, lymphoma, leukemia, skin cancer, breast cancer, kidney cancer, neuroblastoma, Merkel cell carcinoma, myelodysplastic syndrome, myelofibrosis, and multiple myeloma.

The cancer may be neuroblastoma or breast cancer.

Furthermore, and as shown in the appended non-limiting examples, 1,1-DEE or the pharmaceutical composition comprising 1,1-DEE according to the present invention is further useful in the treatment of cancer-induced cachexia.
5. The pharmaceutical composition for use according to any one of items 1 to 4, wherein the composition induces mitochondrial biosynthesis and/aerobic respiration, particularly aerobic respiration in mitochondria, such as mitochondria within a cell, by at least of one:
i. increasing the expression of PGC-1α (peroxisome proliferator-activated receptor gamma coactivator-1 alpha); and/or
ii. enhancing the transcriptional activities of Nrf1 and Nrf2, which in turn stimulate the expression of Tfam.
The pharmaceutical composition for use according to item 5, wherein the composition comprises 1,1-DEE.

The skilled artisan is aware of means and methods to measure/determine features such as expression of PGC-1α, increased expression of PGC-1α, transcriptional activities of Nrf1 and Nrf2, and expression of Tfam. Suitable techniques to assess these parameters are abundantly known in the art. Experimental guidance is, *inter alia* provided in the appended Examples.

As further items, the present invention also provides for food, feed and cosmetic compositions comprising the mitochondrial biosynthesis promoter (i.e. 1,1-DEE) according to the present invention. Such compositions may be generated by adding the mitochondrial biosynthesis promoter according to the present invention (i.e. 1,1-DEE) to the respective composition. Such compositions are of particular use for improving non-medical conditions associated with a lack of mitochondria or a reduction of mitochondrial activity. For example, compositions comprising 1,1-DEE may be useful in the context of anti-aging, lifespan extension, or enhancement of physical vitality.

Hereinafter, various examples are provided to facilitate understanding of the invention. The following examples are provided solely to aid in the understanding of the invention and are not intended to limit the scope of the invention to these examples.

### <Materials and Methods>

### 1. Materials

1,1-DEE (A902), 2',7'-dichlorofluorescein diacetate (DCFH-DA, #35845), N-acetyl-L-cysteine (A9165), and hydrogen peroxide (H₂O₂, #88579) were purchased from Sigma-Aldrich (St. Louis, MO, USA). 1,2-DEE was purchased from Tokyo Chemical Industry (Tokyo, Japan). The EZ-Cytox assay kit (EZ-3000) was obtained from DoGenBio (Geumcheon, Seoul, Republic of Korea). MitoTracker^{®} Red CMXRos (#9082) and protease/phosphatase inhibitor cocktail (100×, #5872) were purchased from Cell Signaling Technology (CST, Danvers, MA, USA).

The following antibodies were used: AMPK (#2532, 1:1000), phospho-AMPK (Thr172) (#2535, 1:1000), ACC (#3676, 1:1000), phospho-ACC (Ser79) (#11818, 1:1000), PFKFB2 (#13029, 1:1000), phospho-PFKFB2 (Ser483) (#13064, 1:1000), PGC-1α (#2178, 1:1000), Nrf1 (nuclear respiratory factor 1) (#46743, 1:1000), Nrf2 (#12721, 1:1000), and β-actin (#4970, 1:1000) antibodies (CST). In addition, PGC-1α (#PA5-72948, 1:1000) and mitochondrial transcription factor A (Tfam, #MA5-16148, 1:1000) antibodies were purchased from Invitrogen. GAPDH (LF-PA0212, 1:1000) and anti-rabbit IgG horseradish peroxidase-conjugated antibody (LF-SA8002, 1:5000) were obtained from AbFrontier (Daejeon, Korea).

### 2. Cell Culture and Treatment

AC16 cells were maintained in Dulbecco's Modified Eagle Medium/Nutrient Mixture F-12 (DMEM/F12, LM002-05) supplemented with fetal bovine serum (FBS, S001-01) and 1× penicillin/streptomycin (P/S, LS 202-02) (Welgene, Gyeongsan, Korea). Cells were passaged at approximately 70% confluency using 1× trypsin-EDTA (#25300-062). For specific experiments, cells were seeded into 6-well plates and allowed to attach overnight. Prior to co-incubation with cells, 1,1-DEE was diluted in serum-free medium.

Wild-type (WT) and AMPKα double knockout (DKO) mouse embryonic fibroblast (MEF) cell lines were provided by Gachon University College of Medicine and were cultured in DMEM (LM001-05) supplemented with 10% (v/v) FBS and 1× P/S (Welgene).

### 3. Cell Viability Assay

The cytotoxicity of 1,1-DEE was evaluated using the EZ-Cytox assay according to the manufacturer's protocol. Cells were seeded into 96-well plates at a density of 17,000 cells/10 µL/well and allowed to attach overnight in an incubator at 37°C with 5% CO₂. The following day, cells were treated with 1,1-DEE and incubated for 24 hours. After 24 hours of incubation, cells were treated with EZ-Cytox reagent (1:10 v/v) and incubated for 30 minutes. The signal was detected at 450 nm using a microplate reader. Cell viability was calculated using the following Equation 1, and the results were analyzed using GraphPad Prism 8.0.1 (San Diego, CA, USA). [Equation 1] $Viability \left(%\right) = \frac{Exp - Blank}{Control - Blank} \times 100$

In Equation 1, "blank" refers to the absorbance of wells containing cell-free medium and EZ-Cytox reagent, "control" refers to the absorbance of wells containing cells and EZ-Cytox reagent but not treated with 1,1-DEE, and "exp" refers to the absorbance of wells containing cells, 1,1-DEE, and EZ-Cytox reagent.

### 4. Assessment of Mitochondrial Oxygen Consumption Rate (OCR) and Extracellular Acidification Rate (ECAR)

Mitochondrial OCR and ECAR were evaluated using the Agilent Seahorse Mito Stress Test Kit (#103015-100, Seahorse Bioscience, Houston, TX, USA) according to the manufacturer's protocol. Prior to the assay, AC16 cells were seeded into 96-well Seahorse cell culture microplates at a density of 1.8 × 10⁴ cells/well and allowed to attach overnight. The sensor cartridge was hydrated by inserting it into 200 µL of Seahorse XF calibration solution (#100840-000) and incubated overnight in a non-CO₂ incubator at 37°C. On the day of the assay, cells were washed and replaced with 180 µL/well of Seahorse XF DMEM (#103575-100) according to a modified protocol. OCR and ECAR were measured using the Seahorse XFe96 Analyzer (Agilent, California, USA) by sequentially injecting 1,1-DEE, oligomycin (1.5 µM), carbonyl cyanide-4-(trifluoromethoxy) phenylhydrazone (FCCP, 1.5 µM), and rotenone/antimycin A (0.5 µM) into the injection ports. The results were analyzed using GraphPad Prism 8.0.1.

### 5. Immunofluorescence Staining and Flow Cytometry Analysis

Cells were seeded into 6-well plates and allowed to attach overnight at 37°C under 5% CO₂. Subsequently, cells were exposed to 1,1-DEE. For immunofluorescence staining, after specific treatments, cells were washed with PBS and incubated with 10 µM DCFH-DA for ROS detection or 200 nM MitoTracker^{®} Red CMXRos for mitochondrial labeling at 37°C under 5% CO₂ for 30 minutes. Following incubation, cells were washed twice with PBS, and images were captured using a fluorescence microscope. For flow cytometry analysis, cells were incubated with DCFH-DA or MitoTracker, collected by trypsinization, and fixed in 70% ethanol at 4°C for 30 minutes. The DCFH-DA signal was detected using a FACS Canto II with excitation and emission wavelengths of 485 nm and 535 nm, respectively, while the MitoTracker Red signal was detected at excitation and emission wavelengths of 579 nm and 599 nm, respectively.

### 6. Western Blot Analysis

Cells were washed twice with Dulbecco's phosphate-buffered saline (DPBS, LM001-01, Welgene) and lysed using a lysis buffer containing 20 mM Tris-HCl (pH 7.5), 150 mM NaCl, 5% glycerol, 0.1% NP-40, and 1× protease/phosphatase inhibitor cocktail. The cell lysates were placed on ice and gently mixed every 5 minutes for 30 minutes. Cellular debris was removed by centrifugation at 13,000 rpm for 10 minutes, and the supernatant was collected. Protein concentrations were determined using a BCA protein assay kit (Pierce, USA). The lysates were mixed with sample buffer containing 60 mM Tris (pH 6.8), 25% glycerol, 2% SDS, 5% 2-mercaptoethanol, 0.5% bromophenol blue, and 2-mercaptoethanol, and heated at 95°C for 5 minutes. Samples were then loaded onto SDS-PAGE gels and transferred to Immobilon PVDF membranes. The membranes were blocked in TBST containing 3% BSA at room temperature for 1 hour and incubated overnight at 4°C with primary antibodies. The following day, membranes were washed three times with TBST and further incubated with appropriate secondary antibodies for 1 hour. Protein expression was detected using Immobilon chemiluminescent HRP substrate (#P90720, Merck Millipore) and visualized with Fusion Solo Vilber Lourmat (Vilber GmbH, Eberhardzell, Germany). Protein quantification was performed using ImageJ software (ImageJ 1.5i, National Institutes of Health, Bethesda, MD, USA).

### 7. RNA Extraction, Semi-Quantitative PCR, and Quantitative Real-Time PCR (qRT-PCR)

Total RNA was extracted using Trizol reagent (#155960626, Ambion, Carlsbad, California, USA). RNA concentrations were measured using a NanoDrop spectrophotometer, and the A260/A280 ratio ranged from 1.8 to 2.0, indicating high purity. Complementary DNA (cDNA) was synthesized using Improm-II^{™} reverse transcriptase (A3802, Promega, Madison, USA) according to the manufacturer's protocol. The resulting cDNA was applied to semi-quantitative PCR using AccuPower^{®} PCR Premix (Bioneer, Daejeon, Korea). Additionally, qRT-PCR was performed using TOPreal^{™} SYBR Green qPCR Premix (#RT500S, Enzynomics, Daejeon, Korea). Primers were purchased from Bioneer and are listed in Table 1.

**[Table 1]**

| Gene | Forward Primer | Reverse Primer |
|---|---|---|
| *PPARGC1A* | AGCCCCATGGATGAAGGGTACT | CAGCCTTGGGGAGGTCTCAT |
| *Nrf1* | AGTCTCCACGGCGCAGG | TCGGGAGAAGAAGGCGAGTC |
| *Nrf2* | ACATCCAGTCAGAAACCAGTG | CCGGGAATATCAGGAACAAGTG |
| *Tfam* | TGATTCACCGCAGGAAAAGC | GTGCGACGTAGAAGATCCTTTC |
| *GAPDH* | GAAGGTGAAGGTCGGAGTC | GAAGATGGTGATGGGATTTC |

### 8. Statistical Analysis

Statistical analyses were performed using GraphPad Prism 8.0.1. Data are presented as the mean ± standard error of the mean (SEM). Statistical significance was determined using two-way analysis of variance (ANOVA), and a p-value ≤ 0.05 was considered statistically significant.

### <Results>

### 1. Evaluation of Transient Inhibition of OCR and ECAR by 1,1-DEE in AC16 Cells

To evaluate the effect of 1,1-DEE (Figure 1A) on cell viability and to determine the concentrations to be used in subsequent experiments, an EZ-Cytox assay was performed. AC16 cells were treated with various concentrations of 1,1-DEE for 24 hours, followed by the addition of EZ-Cytox reagent to assess cell viability. The results showed that concentrations of 25 mM or lower did not induce cytotoxicity (Figure 1B). Under the same conditions, metformin treatment was also found to be safe. Accordingly, 25 mM was selected as the maximum treatment concentration for subsequent experiments.

Mitochondrial respiration was assessed using the Agilent Seahorse XF96 Cell Mito Stress Test Kit. Various concentrations of 1,1-DEE were acutely injected into the cells for 30 minutes, and mitochondrial modulators, including oligomycin, FCCP, and rotenone/antimycin, were sequentially injected to monitor OCR and ECAR.

As a result, basal OCR sharply decreased immediately after 1,1-DEE injection, but a rapid and reversible recovery was subsequently observed. The decrease in OCR occurred at concentrations of 10-20 mM; however, within this concentration range, OCR did not fully recover even after 30 minutes. In contrast, injection of 1,2-DEE did not produce significant changes in OCR, and treatment with metformin at 5-20 mM showed a gradual decrease in OCR after 30 minutes (Figure 1C). Furthermore, mitochondrial ATP-linked respiration, maximal respiration, and spare respiratory capacity were assessed following sequential injections of oligomycin, FCCP, and rotenone/antimycin. Acute injection of 1,1-DEE at 10-20 mM decreased ATP-linked respiration, maximal respiration, and spare respiratory capacity, whereas no significant changes were observed in proton leak (Figure 1D).

ECAR was measured as the rate of lactate release per minute from pyruvate, reflecting the glycolytic flux of the cells. Similar to basal OCR, acute injection of 1,1-DEE resulted in a reversible decrease in ECAR (Figure 1E), whereas treatment with 1,2-DEE or metformin did not produce significant changes in ECAR (Figure 1E).

Collectively, these results suggest that 1,1-DEE rapidly modulates mitochondrial and glycolytic functions, inducing a reversible decrease in both mitochondrial oxidative phosphorylation (OXPHOS) and cellular glycolysis.

### 2. Evaluation of 1,1-DEE-Mediated AMPK Activation

Eukaryotic cells possess a highly conserved homeostatic system to respond to decreases in ATP levels when oxidative phosphorylation (OXPHOS) and glycolysis are impaired by mitochondrial toxins. A central component of this system is AMP-activated protein kinase (AMPK), the key regulator of cellular energy. Upon OXPHOS inhibition, AMPK is fully activated within minutes, promoting catabolic processes while suppressing anabolic pathways.

To determine whether 1,1-DEE affects AMPK activation by phosphorylating the Ser172 residue of AMPK through OXPHOS inhibition, AC16 cells were treated with various concentrations of 1,1-DEE for 10 minutes, and phospho-AMPK expression was analyzed. The results showed that phospho-AMPK expression was increased as early as 10 minutes after 1,1-DEE treatment (Figure 2A).

Furthermore, to examine whether 1,1-DEE transiently induces AMPK activation, cells were exposed to 1,1-DEE for defined time intervals. Phospho-AMPK expression transiently increased at 5 and 10 minutes and then decreased, indicating that acute exposure to 1,1-DEE can reversibly activate AMPK (Figure 2B). In contrast, exposure to 1,2-DEE did not affect AMPK activation over 30 minutes (Figure 2C), whereas metformin treatment gradually increased AMPK phosphorylation after 60 minutes (Figure 2D).

### 3. Mechanistic Investigation of Fatty Acid Oxidation and Glycolytic Regulation via 1,1-DEE-Induced AMPK Activation

AMP-activated protein kinase (AMPK) regulates fatty acid metabolism through the phosphorylation of acetyl-CoA carboxylase (ACC). Phosphorylation of ACC by AMPK inhibits its activity, thereby suppressing fatty acid synthesis. Inhibition of ACC reduces the production of malonyl-CoA, a substrate for fatty acid synthase (FAS), which normally suppresses mitochondrial fatty acid β-oxidation. Thus, AMPK can promote fatty acid oxidation by controlling ACC activity.

AC16 cells were treated with various concentrations of 1,1-DEE for 30 minutes, and the phosphorylation levels of ACC and PFKFB2, downstream targets of AMPK, were analyzed. 1,1-DEE-activated AMPK phosphorylated ACC at Ser79, thereby inhibiting ACC activity, reducing fatty acid synthesis, and promoting mitochondrial fatty acid β-oxidation (Figure 3A). In addition, 1,1-DEE treatment decreased the expression of sterol regulatory element-binding protein-1c (SREBP-1c) and FASN, further supporting the suppression of ACC activity (Figure 3B). AMPK is also known to regulate glycolysis via activation of PFKFB2. Following 1,1-DEE exposure, phosphorylation of PFKFB2 at Ser466 and Ser483 increased, indicating enhanced PFKFB2 activity and glycolytic flux (Figure 3A).

To confirm that ACC and PFKFB2 phosphorylation is AMPK-dependent, wild-type (WT) and AMPK double knockout (DKO) MEF cells were treated with 1,1-DEE for 30 minutes. Consistent with results in AC16 cells, 1,1-DEE treatment in WT MEF cells increased AMPK and ACC phosphorylation, whereas no increase was observed in AMPK DKO MEF cells (Figure 3C). Furthermore, pre-treatment with the AMPK inhibitor compound C reduced phosphorylation at PFKFB2 Ser483, confirming that PFKFB2 activation is dependent on 1,1-DEE-induced AMPK activation (Figure 3D).

### 4. Investigation of ROS-Mediated AMPK Activation Induced by 1,1-DEE

AMPK activation is primarily stimulated by changes in the ADP/ATP and AMP/ATP ratios. However, several studies have reported that AMPK can also be activated by reactive oxygen species (ROS), particularly H₂O₂. To determine whether 1,1-DEE-induced AMPK activation is mediated by ROS, we performed the following experiments.

After treatment with 1,1-DEE, intracellular ROS levels were measured using DCFH-DA and analyzed via live-cell fluorescence imaging. Compared to the control, ROS levels were increased after 10 minutes of 1,1-DEE treatment (Figure 4A). Furthermore, pre-treatment with the ROS scavenger N-acetylcysteine (NAC) reduced ROS generation induced by 1,1-DEE (Figure 4A).

Subsequently, cells were pre-treated with NAC or ebselen prior to 30-minute exposure to 1,1-DEE, and AMPK phosphorylation levels were analyzed via Western blot. The results showed that AMPK phosphorylation was reduced in the presence of ROS scavengers, indicating that ROS mediate 1,1-DEE-induced AMPK activation (Figure 4B).

### 5. Evaluation of PGC-1α Upregulation via AMPK Activation Following Long-Term 1,1-DEE Treatment

In this study, we assessed AMPK activation following long-term exposure to 1,1-DEE. AC16 cells were treated with 15 mM 1,1-DEE for 8 hours, and increased AMPK phosphorylation was observed at 4 and 8 hours, confirming AMPK activation (Figure 5A).

Among AMPK targets, PGC-1α is a key regulator of most genes related to mitochondrial metabolism and is considered a central regulator of mitochondrial biogenesis. Treatment of AC16 cells with 1,1-DEE for 8 hours resulted in increased mRNA expression of PPARGC1A, the gene encoding PGC-1α (Figure 5B). Western blot analysis further confirmed that 1,1-DEE-induced AMPK activation led to elevated PGC-1α expression (Figure 5C).

To determine whether the increase in PGC-1α expression is dependent on AMPK activation, wild-type (WT) and AMPK double-knockout (DKO) MEF cells were treated with 15 mM 1,1-DEE for up to 12 hours. In WT MEF cells, AMPK phosphorylation induced by 1,1-DEE was associated with increased PGC-1α expression starting at 2 hours, whereas no significant changes in PGC-1α expression were observed in AMPK DKO MEF cells (Figure 5D).

### 6. Mechanistic Evaluation of Mitochondrial Biogenesis Regulated by AMPK-Induced PGC-1α

PGC-1α is a key transcriptional regulator of mitochondrial biogenesis, capable of increasing mitochondrial content through the transcriptional machinery. PGC-1α binds to Nrf1 and Nrf2, enhancing the transcription of mitochondrial genes related to the electron transport chain (ETC), while simultaneously promoting the expression of Tfam, which is responsible for mitochondrial transcription and genome replication. This coordinated regulation facilitates the formation of new mitochondria.

To evaluate whether PGC-1α induces mitochondrial biogenesis via the Nrf1/Nrf2-Tfam axis, AC16 cells were treated with 15 mM 1,1-DEE for up to 8 hours. Semi-quantitative PCR and qRT-PCR results showed that Nrf1 and Nrf2 mRNA levels increased after 8 hours of treatment, whereas Tfam expression was significantly elevated starting at 4 hours (Figure 6A). Western blot analysis confirmed significant increases in Nrf2 and Tfam protein levels, while Nrf1 protein levels remained unchanged (Figure 6B).

To determine whether AMPK regulates the upregulation of the Nrf1/Nrf2-Tfam axis, wild-type (WT) and AMPK double-knockout (DKO) MEF cells were treated with 15 mM 1,1-DEE for up to 8 hours. In WT MEF cells, protein levels of Nrf1 and Nrf2 were increased, whereas no changes were observed in AMPK DKO cells (Figure 6C).

Pre-treatment of AC16 cells with the AMPK inhibitor compound C suppressed Tfam protein expression during 1,1-DEE exposure, confirming that mitochondrial biogenesis via the Nrf1/Nrf2/Tfam axis is dependent on 1,1-DEE-induced AMPK activation (Figure 6D).

Flow cytometry analysis using MitoTracker Red showed increased mitochondrial fluorescence upon 1,1-DEE exposure, whereas compound C pre-treatment reduced this signal, indicating diminished mitochondrial content (Figure 6F). Confocal microscopy revealed that 1,1-DEE treatment induced a fragmented mitochondrial morphology (green arrows), which was alleviated by compound C pre-treatment. No such changes were observed following 1,2-DEE treatment (Figure 6E).

### 7. Evaluation of Mitochondrial Respiration and Glycolytic Enhancement Induced by AMPK Activation Following Long-Term 1,1-DEE Treatment

After confirming that AMPK activation promotes mitochondrial fatty acid oxidation and biogenesis signaling, mitochondrial respiratory capacity was assessed using the Seahorse XF96 Mito Stress Test Kit following 8-hour treatment with 1,1-DEE. Treatment with various concentrations of 1,1-DEE led to a time-dependent increase in mitochondrial OCR (Figure 29A), which was reflected by elevated basal respiration (Figure 29B), ATP-linked production (Figure 29C), and maximal respiration (Figure 29D).

During this period, glycolytic activity was also enhanced, as evidenced by a gradual increase in ECAR, indicating activation of glycolysis (Figure 29M). These results suggest that long-term 1,1-DEE treatment promotes both mitochondrial oxidative phosphorylation (OXPHOS) and glycolysis for ATP production. In contrast, 1,2-DEE treatment did not affect mitochondrial OCR or ECAR (Figures 29E-H, 29N).

Metformin treatment caused a gradual decrease in OCR (Figures 29I-L), while ECAR increased, suggesting that cells compensated for inhibited mitochondrial respiration by enhancing glycolytic ATP production (Figure 29O). Pre-treatment with the AMPK inhibitor compound C reduced mitochondrial OCR in 1,1-DEE-treated cells, leading to decreased basal respiration (Figure 29P), ATP-linked production (Figure 29Q), and maximal respiration (Figures 29R, 29S), and also suppressed ECAR (Figure 29T).

Taken together, these data indicate that the enhancement of cellular energy metabolism, including mitochondrial respiration and glycolysis by 1,1-DEE, is mediated via AMPK activation.

Additionally, male C57BL/6 mice were treated with 1,1-DEE for 8 weeks, and liver tissues were subjected to RNA-seq analysis. Pre-ranked GSEA revealed that 1,1-DEE treatment induced enrichment in multiple mitochondrial-related biological processes (GOBP) (Figures 30A-G). Heatmap analysis of key genes in the "GOBP NADH dehydrogenase complex assembly" pathway showed moderate increases in expression of NADH:ubiquinone oxidoreductase genes (e.g., ndufv1, ndufs2) in the 1,1-DEE-treated group compared with untreated controls, suggesting enhanced mitochondrial oxidative capacity and respiration (Figure 30H).

### <Experimental Example>

### Experimental Example 1. Evaluation of the effect of 1,1-DEE on lifespan extension and Suppression of Cellular Senescence

### 1-1. C. elegans Development Assay

In this experiment, wild-type Caenorhabditis elegans (Bristol N2 strain) was used. Bleached eggs were placed in S-basal buffer supplemented with either 1,1-DEE or 1,2-DEE and cultured in liquid until the L1 larval stage at 20°C. During the incubation, the bleached eggs were placed in 50 mL tubes mounted on a rotator and slowly rotated to ensure uniform mixing. Upon reaching the L1 larval stage, the worms were separated from the S-basal buffer by centrifugation and resuspended in liquid nematode growth media (NGM). E. coli OP50, serving as the food source for the worms, was added to the liquid NGM. The worms were then distributed into 96-well plates. Each well contained liquid NGM, OP50, approximately 20 worms, 1,1-DEE or 1,2-DEE (0, 100 µM, 1 mM, 20 mM), and amphotericin B (0.1 µg/mL), for a total volume of 200 µL. The 96-well plates were gently agitated on a shaker at 20°C to prevent settling of the compounds during liquid culture. After 2-3 days of culture, worm development was observed under a microscope to evaluate the effects of 1,1-DEE and 1,2-DEE.

The results showed that treatment with 1,1-DEE at concentrations of 100 µM and 1 mM did not affect C. elegans development, whereas treatment at 20 mM inhibited their development (Figures 7 and 8). Similarly, 1,2-DEE at 100 µM and 1 mM did not cause any developmental defects (Figure 9). Based on these findings, concentrations that did not affect development, 100 µM and 1 mM, were selected for the subsequent lifespan experiments.

### 1-2. C. elegans lifespan Assay

The 96-well plate setup for the lifespan assay was conducted in the same manner as the developmental assay. However, to prevent egg hatching once the worms reached the L4 stage, 5-fluoro-2' -deoxyuridine (FUDR) was added. Each well had a total volume of 200 µL, containing liquid NGM, E. coli OP50, approximately 20 worms, 1,1-DEE or 1,2-DEE (0, 100 µM, 1 mM, 20 mM), amphotericin B (0.1 µg/mL), and FUDR (200 µM). The 96-well plate was gently mixed on a shaker at 20°C to prevent settling of the compounds. Worm survival was assessed every 2-3 days, and worms that did not move in the liquid medium were considered dead. All experiments were independently performed by two experimenters. Lifespan differences were calculated based on the area under the curve (AUC) of the survival curves, following the actual experimental procedures.

The results showed that treatment with 1,1-DEE at 1 mM extended lifespan by 10% and 13% in experimenters 1 and 2, respectively (Figure 10), whereas 1,1-DEE at 100 µM had little effect on lifespan. In contrast, 1,2-DEE at 1 mM reduced lifespan by 5% and 15% in experimenters 1 and 2, respectively, and treatment at 100 µM had no significant effect (Figure 11). These results demonstrate that 1,1-DEE at 1 mM can extend the lifespan of C. elegans without affecting their development.

### 1-3. Rat lifespan assay

To evaluate the lifespan-extending effects of 1,1-DEE in a mammalian model, a lifespan study was conducted using 48-week-old rats. A total of 20 rats were randomly divided into two groups (n=10 per group). The control group received a single tail vein injection of 300 µL of saline, whereas the experimental group received 300 µL of 1,1-DEE solution at a dose of 300 mg/kg via the same route. All injections were performed under isoflurane inhalation anesthesia at 48 weeks of age. The rats were then maintained under identical environmental conditions for 20 weeks, with two rats per cage from the same group, and uniform feeding and lighting conditions were maintained.

In the control group, survival gradually declined starting at week 62: one rat died at week 62, one at week 63, one at week 64, one at week 65, two at week 66, and two at week 67. In contrast, in the 1,1-DEE-treated group, only one rat died by week 67, indicating a marked improvement in survival. Kaplan-Meier survival curve analysis revealed that the 1,1-DEE group showed a statistically significant increase in survival compared to the control group (p < 0.01, **) (Figure 31). At the end of week 68, only 2 of 10 rats survived in the control group, whereas 9 of 10 rats survived in the 1,1-DEE-treated group.

Additionally, general health indicators including fur condition, respiratory rate, and spontaneous activity were periodically assessed. Rats treated with 1,1-DEE exhibited shinier, healthier fur and noticeably increased spontaneous activity compared to the control group (Figure 32). These results suggest that 1,1-DEE not only extends lifespan but may also contribute to an improvement in health-span.

### 1-4. Endothelial Cell Senescence Assay

To evaluate the anti-senescence activity of 1,1-diethoxyethane (1,1-DEE), a cellular aging model using human vascular endothelial EA.hy926 cells was established. The cells were cultured in Dulbecco's modified Eagle medium (DMEM) supplemented with 10% fetal bovine serum and antibiotics at 37°C in a humidified 5% CO₂ incubator. Prior to treatment, the cells were incubated in serum-free DMEM overnight. The cells were then pretreated with various concentrations of 1,1-DEE (0, 0.01, 0.1, and 1 mM) for 8 h, followed by exposure to oxidized LDL (ox-LDL, 100 µg/mL) for 48 h to induce senescence.

Senescence-associated β-galactosidase (SA-β-gal) staining was conducted using a commercial kit according to the manufacturer's protocol. After fixation, the cells were incubated in staining solution at 37°C for 18-24 h. Stained cells were observed under a light microscope at 200× magnification, and the proportion of SA-β-gal-positive cells was quantified based on at least three independent experiments.

Exposure to ox-LDL markedly increased senescence, resulting in approximately a three-fold elevation in SA-β-gal-positive cells compared to the untreated control (41.3% vs. 14%). Pretreatment with 1 mM 1,1-DEE significantly suppressed ox-LDL-induced senescence, reducing the positive cell proportion to 23.0% (p < 0.01), corresponding to a 44.3% decrease. Lower concentrations (0.01 and 0.1 mM) did not exhibit notable effects. The calculated effect size (Cohen's d = 4.1) indicated a strong biological impact of 1,1-DEE at 1 Mm(Figure 36).

These results confirm that 1,1-DEE effectively suppresses endothelial cell senescence induced by dyslipidemic stress. In combination with the lifespan extension observed in C. elegans and rats, these findings suggest that 1,1-DEE exerts both lifespan- and health-span-enhancing effects by mitigating cellular aging processes.

### Experimental Example 2. Evaluation of the Effects of 1,1-DEE on Heart Rate

### 2-1. Formation of Engineered Heart Tissue (EHT)

Induced pluripotent stem cell-derived cardiomyocytes (iPSC-CM) and primary cardiac fibroblasts were mixed at a ratio of 9:1 with 12.5 mg/mL fibrin gel and then loaded into a mold formed with 2% agar. After placing the ends of silicone pillars in contact with the cell and hydrogel mixture, the construct was cured in a 37°C incubator for 30 minutes. The cured hydrogel was removed from the mold and cultured for 8 days in vitamin A-supplemented RPMI1640 cell culture medium. The medium was replaced every 2 days, and 20 µg/mL Aprotinin was added to the cell culture medium from day 1 to day 7 of culture to prevent fibrin degradation.

### 2-2. Measurement of beating (heart rate) following 1,1-DEE and 1,2-DEE treatment

On day 8 of culture, cell culture medium (RPMI1640 - vitamin A) containing 1,1-DEE and 1,2-DEE was prepared at final concentrations of 1 mM, 5 mM, 10 mM, 25 mM, 50 mM, and 75 mM, and the existing medium was replaced with the prepared medium. Before and at 10 minutes, 1 hour, 6 hours, 12 hours, and 24 hours after medium replacement, the movement of the pillars was recorded for 15 seconds using a 4× objective lens. The recorded videos were quantified for pillar movement using custom-developed MATLAB code, and the distance of pillar displacement and the number of movements over 15 seconds were analyzed using the findpeaks function in R. Based on these data, the twitch force generated by the EHT and the BPM (beats per minute) were calculated.

Figures 12 and 13 show the changes in the beating rate (BPM: beats per minute) of EHTs over time before and after treatment with various concentrations of 1,1-DEE and 1,2-DEE.

Figures 14 and 15 show the changes in the twitch force of EHTs over time before and after treatment with various concentrations of 1,1-DEE and 1,2-DEE.

Figure 16 presents representative images of EHT beating following treatment with 50 mM of 1,1-DEE and 1,2-DEE.

Following treatment with 1,1-DEE and 1,2-DEE, it was observed that the beating rate of EHTs increased in a 1,1-DEE concentration-dependent manner, while the twitch force decreased proportionally in the 1,1-DEE-treated group. These changes persisted up to 1 hour after compound treatment, after which no significant differences were observed compared to the 1,2-DEE-treated group. Additionally, an increase in the beating rate was accompanied by a decrease in beating amplitude, which is considered to be related to the reduction in twitch force (see Figures 12-16).

A comprehensive analysis of high- and low-concentration experiments revealed a clear trend of increasing BPM and decreasing twitch force depending on the 1,1-DEE concentration. Notably, the effect on BPM recovered approximately 6 hours post-treatment, whereas the effect on twitch force persisted up to 24 hours.

Furthermore, Figure 17 shows the changes in beating rate and twitch force of EHTs during the 10-minute period immediately after treatment with 25 mM 1,1-DEE. As shown in Figure 17, the BPM began to increase as early as 1 minute post-treatment, with the effect becoming more pronounced over time. In contrast, the twitch force gradually decreased during the same time period, reaching less than approximately 10% of the pre-treatment value at 10 minutes. These results indicate that 1,1-DEE rapidly increases heart rate immediately after treatment while simultaneously suppressing contractile force, supporting its potential as an acute heart rate-promoting composition.

Therefore, these experimental results demonstrate that 1,1-DEE rapidly increases the beating of EHTs from immediately after treatment up to 10 minutes to 1 hour, and that this effect diminishes over time. Accordingly, 1,1-DEE has been shown to have potential as a drug capable of temporarily increasing heart rate.

### Experimental Example 3. Evaluation of the effects of 1,1-DEE on cachexia

### 3-1. Experimental Method

### (1) Cell Viability Assay

Cells were seeded in 96-well plates at a density of 2×10⁴ cells per well (100 µl) in RPMI supplemented with 10% FBS and 1% penicillin-streptomycin. The following day, different concentrations of 1,1-DEE and 1,2-DEE, including serial dilutions, were added to each well (total 120 µl) and incubated for 24 hours. Subsequently, 10 µl of MTT (DoGenBio Co., Ltd.) was added to each well of the plate and incubated at 37°C for 2 hours. Cell viability was analyzed by measuring absorbance at 450 nm using a microplate spectrophotometer (Epoch, Biotek, USA).

### (2) Cell Culture

SHSY-5Y (Human Neuroblastoma) cells were purchased from the Korean Cell Line Bank (Daehak-ro, Jongno-gu, Seoul, South Korea) and cultured in growth medium containing RPMI 1640 supplemented with 10% FBS and 1% penicillin-streptomycin at 37°C in a humidified incubator with 5% CO₂.

### (3) Western Blot

To extract proteins from SHSY-5Y cells (5 × 10⁵/mL) cultured in 60 × 15 mm cell culture dishes (SPL Life Sciences, Gyeonggi-do, Republic of Korea), 150 µL of Pro-PREP^{™} protein extraction solution (iNtRON Biotechnology, Gyeonggi-do, South Korea) was added to each plate. Materials such as polyvinylidene fluoride (PVDF) membranes and Western chemiluminescent HRP substrates were purchased from Millipore Corporation (Billerica, MA, USA).

After separating a total of 30 µg of protein, the samples were resolved using 10% sodium dodecyl sulfate-polyacrylamide gel electrophoresis (SDS-PAGE). The proteins were then transferred onto a PVDF membrane for further analysis. The membrane was blocked for 1-2 hours in TBST containing 5% skim milk and 0.1% Tween-20, followed by overnight incubation at 4°C with primary antibodies (1:1000) in TBST. After three washes with TBST for 10 minutes each, immunoreactive proteins were detected using horseradish peroxidase-conjugated secondary antibodies (1:2000) via chemiluminescence.

Various antibodies from Cell Signaling Technology (Danvers, MA, USA) were used for Western blot experiments, including anti-phospho-AMPK (Cell Signaling, #2535S), anti-AMPK (Cell Signaling, #2532S), and anti-β-actin (Cell Signaling, #5125S) monoclonal antibodies. To assess total protein levels, the blotted membranes were stripped using Restore^{™} Western Blot Stripping Buffer (Thermo Fisher Scientific, Meridian Rd., Rockford, IL, USA) at 56°C for 30 minutes.

### (4) Assessment of Cell Death Using Annexin V/FITC

The apoptotic potential of SHSY-5Y cells was assessed using an Annexin V fluorescence kit (BD Pharmingen, San Diego, USA) according to the manufacturer's protocol. Cells were seeded in 6-well plates at a density of 2.0 × 10⁵ cells per well in RPMI medium containing 10% FBS and cultured for 24 hours. Subsequently, the cells were treated with the desired concentration of 1,1-DEE and incubated at 37°C for 24 hours.

After treatment, the cells were harvested and pelleted, then resuspended in 400 µL of binding buffer. The cells were stained with 5 µL FITC-Annexin V and 10 µL PI provided in the kit. Flow cytometry analysis was performed using a BD FACS Calibur (BD Biosciences, CA, USA), and plots and data were analyzed using FlowJo^{™} software (BD Biosciences, CA, USA).

### (5) Animal Care and Drug Administration

Male NSG mice at 4 weeks of age were purchased from the National Center for Immunotherapy Innovation, Chonnam National University (CNU), and housed at the CNU Laboratory Animal Research Center. Mice were maintained at 23°C and 60% humidity under a 16-hour light/8-hour dark cycle, with ad libitum access to food and water until experimental procedures were conducted. Five animals were housed per cage, and body weight was recorded three times per week. All experimental procedures, including those involving live animals, were conducted in accordance with institutional guidelines of Chonnam National University(CNU IACUC-H-2024-42).

SHSY-5Y cells cultured in RPMI medium containing 10% FBS were suspended in 100 µL of phosphate-buffered saline (PBS) at a concentration of 10⁷ cells and subcutaneously injected into the right flank of each mouse. Mice were divided into control and treatment groups. For prophylactic purposes before tumor induction, 1,1-DEE (110 mg/kg) was administered intraperitoneally (IP) three times on alternate days. The treatment group received alternate continuous injections from day 1 to day 105, whereas the control group received PBS injections. The day following drug administration, the animals were used for survival studies.

### 3-2. Results

### (1) Cytotoxicity evaluation of 1,1-DEE and 1,2-DEE (negative control) on SHSY-5Y cell line

The cell viability of SHSY-5Y cells treated with 1,1-DEE was measured using the MTT assay, as shown in Figure - 18. The results indicated that 1,1-DEE reduced the viability of SHSY-5Y cells at concentrations more than 10 mM (Figure 18A). In contrast, no significant effect was observed in the 1,2-DEE treatment group (Figure 18B).

To further evaluate morphological changes, the effects of time- and dose-dependent treatment with 1,1-DEE on SHSY-5Y cell morphology were observed using an inverted light microscope. The results demonstrated that 1,1-DEE induced distinct morphological changes and reduced cell viability in a time- and dose-dependent manner, whereas such effects were not observed in the 1,2-DEE treatment group (Figure 18C).

### (2) Evaluation of cell death induction by 1,1-DEE

To determine whether 1,1-DEE induces cell death, SHSY-5Y cells were treated with 1,1-DEE for 24 hours, followed by Annexin V and PI staining. The results showed that 1,1-DEE increased the proportion of late apoptotic cells in a concentration-dependent manner, corresponding to the Q2 quadrant (Figure 19).

### (3) Evaluation of AMPK activation induction by 1,1-DEE

To assess the effect of 1,1-DEE on AMPK activation, experiments were conducted using the SHSY-5Y cell line. The results showed that 1,1-DEE activated AMPK in a time-dependent manner, with significant activation observed between 30 minutes and 2 hours. In addition, 1,1-DEE at a concentration of 2 mM induced particularly pronounced concentration-dependent AMPK activation (Figure 20).

### (4) Evaluation of tumor growth inhibition by 1,1-DEE

To determine whether 1,1-DEE suppresses tumor growth in vivo, a subcutaneous tumor model was established using 4-week-old NSG mice. The experimental group received a total of three intraperitoneal injections of 1,1-DEE prior to stable subcutaneous injection of SHSY-5Y cells. Tumor size was measured starting three weeks after tumor inoculation, and tumor growth was monitored while repeating administration of 1,1-DEE at a fixed concentration every two days. The results showed that 1,1-DEE significantly inhibited tumor growth in vivo. As shown in Figure 21(A), the tumor growth curves for each group indicate that, between days 21 and 33 after tumor palpation, tumor volume was markedly reduced in the 1,1-DEE-treated group. In addition, Figure 21(B) presents body weight changes of the mice measured during the experimental period, and survival rate and tumor size measurements were used to evaluate the in vivo antitumor effect.

### (5) Evaluation of survival improvement and prevention of cancer cachexia by 1,1-DEE

To assess whether 1,1-DEE acts prophylactically before cancer induction, 1,1-DEE was administered using a metronomic regimen with three doses at one-day intervals prior to tumor cell injection, and drug administration was continued thereafter. As shown in the Kaplan-Meier survival curve in Figure 22(A), the 1,1-DEE-treated group exhibited significantly higher survival compared to the PBS-treated group.

In addition, Figure 22(B) shows changes in body weight of the mice throughout the survival period, providing an indirect assessment of cancer cachexia occurrence. The results indicated that body weight loss was suppressed in the 1,1-DEE-treated group, supporting its potential preventive effect against cancer cachexia.

### Experimental example 4. Evaluation of the effect of 1,1-DEE on ischemic stroke

### 4-1. Experimental Methods

### (1) Animals

All animal experiments were conducted in accordance with the guidelines for the care and use of laboratory animals at Chonnam National University (CNUHIACUC-25015) . Mice were maintained under a 12-hour light/dark cycle with ad libitum access to food and water. Male C57BL/6 (Black 6) mice, 8 weeks old and weighing 27-28 g, were used (Samtako Bio Korea Co., Ltd, Korea).

### (2) Drugs and Administration

The test compound was prepared by dilution in physiological saline and administered as a single intravenous injection via the tail vein at a dose of 20 mg/kg.

### (3) Induction of Ischemic Stroke

The ischemic stroke model was induced via middle cerebral artery occlusion (MCAO) surgery. Anesthesia was induced by inhalation of isoflurane mixed with oxygen (Hana Pharm, Seoul, Republic of Korea). After a midline cervical incision, the left common carotid artery, external carotid artery, and internal carotid artery were exposed. A monofilament was inserted through the external carotid artery approximately 10 mm to occlude the origin of the middle cerebral artery. Occlusion was maintained for 45 minutes, after which the filament was removed to allow reperfusion. The surgical site was sutured, and the mice were monitored during recovery.

### (4) Brain MRI

The mice were placed in a prone position, and brain images were acquired using MRI equipment.

### (5) Morris Water Maze (MWM)

A water maze with a diameter of 100 cm, height of 50 cm, water depth of 30 cm, water temperature of 24±1°C, and opaque white water was used in an environment with visual cues blocked. The pool was divided into four quadrants in a clockwise manner, and a hidden platform was placed in Zone 1. Mice were allowed to freely explore for two days to acclimate to the environment. During training, each trial lasted 60 seconds; if a mouse failed to find the platform, it was guided to the platform and allowed to stay for 10 seconds. Even if the mouse successfully found the platform, it was allowed to remain for 10 seconds. Each trial started from Zone 3. During the testing period, exploration time, path, speed, and success rate were recorded and analyzed using EthoVision XT 14 software.

### 4-2. Results

### (1) Evaluation of the Effect of 1,1-DEE on Post-Ischemic Stroke Mortality

Mortality was compared between the control group (n=10) and the treatment group (n=12) after MCAO. Body weight was measured one day before MCAO, and the treatment group received 1,1-DEE. MCAO was performed under blinded conditions. MRI was conducted 36 hours after occlusion, and the MWM test was performed 48 hours after occlusion. Three days later, mice were sacrificed following final body weight measurement. The results showed that the control group exhibited a 30% mortality rate, whereas all mice in the 1,1-DEE treatment group survived. Statistical analysis (Chi-square test, p < 0.05) indicated a risk ratio (RR) of 0.3684 (95% CI: 0.1915-0.9505), demonstrating that the risk of death in the treatment group was reduced by 63.16% (Fig. 23).

### (2) Evaluation of the Effect of 1,1-DEE on Post-Stroke Body Weight Changes

Body weight loss after stroke is a common phenomenon caused by systemic inflammation and neurological damage and can lead to sarcopenia and cachexia. Body weight is closely associated with functional recovery. In this study, body weight changes were also assessed. During the 6-day experimental period, the control group showed an average body weight loss of 3.77%, whereas the treatment group exhibited an average body weight gain of 5.46% (t-test, p < 0.001) (Fig. 24A). Final body weight was also significantly higher in the treatment group at 27.68g compared to 26.29g in the control group (t-test, p < 0.05) (Fig. 24B).

### (3) Evaluation of the Effect of 1,1-DEE on Spatial Memory and Cognitive Function

The Morris Water Maze (MWM) is widely used to assess spatial learning and memory in rodents. In this study, analysis was performed using time-to-event survival curves that integrate latency and success rate. Analysis with the accelerated failure time (AFT) model showed that at the final 60-second time point, 50% of the treatment group succeeded, whereas only 25% of the control group succeeded (p > 0.05). However, at the 20-second time point, 33% of the treatment group succeeded, while none of the control group succeeded, indicating a significant difference (p < 0.05) (Fig. 25A). Swimming speed was similar between the two groups (t-test, p > 0.05), suggesting that swimming ability did not influence the results.

Furthermore, analysis of the swimming trajectories showed that the treatment group followed a direct path toward the target, whereas the control group exhibited a random path (Fig. 25B). Comparison of the path lengths for successful mice also revealed that the treatment group had a significantly shorter path than the control group (146.3 cm vs. 820.8 cm, t-test, p < 0.01) (Fig. 25C). This suggests that 1,1-DEE has a positive effect on the acquisition and retention of spatial memory.

Taken together, these results demonstrate that treatment with 1,1-DEE can enhance spatial cognition or memory recall, particularly during the early stages of task learning.

### (4) Evaluation of the Effect of 1,1-DEE on Alleviating Cerebral Ischemic Injury and Post-Stroke Motor Function

Magnetic resonance imaging (MRI) of the brain was performed within 36 hours after middle cerebral artery occlusion (MCAO) in both experimental groups. Representative images showed that mice treated with 1,1-DEE exhibited smaller infarct areas compared to the control group, suggesting a mitigating effect of 1,1-DEE on stroke-induced brain injury (Fig. 26A).

Ischemic stroke induces not only cognitive deficits but also motor dysfunction. Although the Morris Water Maze (MWM) test is not the optimal method for evaluating motor impairment, it was assumed that MCAO-induced stroke could impair swimming ability in mice. Accordingly, the maximum swimming speed observed in each trial was measured. Interestingly, mice treated with 1,1-DEE showed significantly higher maximum swimming speeds compared to controls (33.83 cm/s vs. 27.77 cm/s, t-test, p < 0.05), indicating that 1,1-DEE may promote motor function recovery after ischemic stroke (Fig. 26B).

### Experimental example 5. Evaluation of the Effect of 1,1-DEE on Cognitive Function

### 5-1. Experimental Method

The experimental animals, drug, administration method, and Morris Water Maze (MWM) test were conducted using the same procedures as described in Experimental example 4.

### 5-2. Results

### (1) Evaluation of the Effect of 1,1-DEE on Cognitive Function in an Ischemic Stroke-Induced Mouse Model

Vascular dementia is the second most common type of dementia after Alzheimer's disease, and stroke is a major risk factor for dementia. In this example, an ischemic stroke mouse model induced by middle cerebral artery occlusion (MCAO) was used to evaluate the impact of stroke on cognitive function. The effect of 1,1-DEE in this model was assessed (Fig. 27). Male C57BL/6 mice underwent a Morris Water Maze (MWM) training session, after which they were randomly assigned to the control group (n = 10) or the 1,1-DEE treatment group (n = 12). In the treatment group, 1,1-DEE was administered via intravenous injection 1 day prior to the MCAO surgery. The MCAO surgery was performed under blinded conditions, and 36 hours after surgery, brain infarction was confirmed using MRI (Fig. 26A). The MWM test was conducted 48 hours after surgery, and the mice were sacrificed 3 days after the test. Evaluation of the MWM test was based on latency time and platform reach success rate, and statistical analysis was performed using an accelerated failure time (AFT) model.

As a result, the 1,1-DEE treatment group showed a higher proportion of mice reaching the platform within 60 seconds compared to the control group, although the difference was not statistically significant (p > 0.05). However, at the 20-second time point, 33% of the 1,1-DEE-treated mice successfully reached the platform, whereas none of the control mice did (Fig. 27A), and this difference was statistically significant (p < 0.05). There was no significant difference in swimming speed between the two groups (p > 0.05, data not shown), indicating that the observed differences in cognitive function were not due to differences in motor ability. Analysis of representative swimming paths for each group showed that the 1,1-DEE-treated mice used a direct strategy toward the platform, while the control mice exhibited random paths (Fig. 27B). Comparison of swimming distances among the mice that successfully reached the platform revealed that the average distance in the control group was 820.8 cm, whereas in the 1,1-DEE group it was 146.3 cm, a statistically significant reduction (p < 0.01, Fig. 27C).

These results suggest that 1,1-DEE has a positive effect on cognitive function recovery following ischemic brain injury.

### (2) Evaluation of the Effect of 1,1-DEE on Cognitive Function in Normal Mice

In this study, the effect of 1,1-DEE on cognitive enhancement was evaluated using the Morris Water Maze (MWM) test in normal male C57BL/6 mice. First, a pre-test was conducted to assess the baseline performance of the mice, and based on this, the animals were randomly assigned to a control group (n = 12) and a 1,1-DEE-treated group (n = 12). The control group received PBS, while the treated group was administered 1,1-DEE at a dose of 20 mg/kg via intravenous injection. Drug administration was performed on days 1, 5, and 9, and MWM tests were conducted on days 3, 7, and 11, which corresponded to two days after each respective drug administration (Fig. 28).

Pre-test results showed no statistically significant difference in baseline performance between the two groups (Fig. 28A). Following the first drug administration, the proportion of mice reaching the platform within 20 seconds was 16.7% in the control group and 41.7% in the 1,1-DEE-treated group (Fig. 28B). After the second administration, the difference became more pronounced: 5 out of 9 mice (55.6%) in the control group succeeded, whereas 9 out of 10 mice (90%) in the treated group reached the platform within 20 seconds (Fig. 28C). A similar trend was observed after the third administration (Fig. 28D). Although the cumulative success rates at each time point were not statistically significant (p > 0.05), the treated group consistently performed better. The average latency time (time to reach the platform) was 40.07 s for the control group and 39.52 s for the treated group in the pre-test; 38.04 s and 33.93 s in the first test; 32.00 s and 21.14 s in the second test; and 41.48 s and 29.78 s in the third test (Fig. 28E). While these differences were not statistically significant at individual time points (p > 0.05), accelerated failure time (AFT) analysis across repeated measures revealed that the treated group showed a significant reduction in latency compared to pre-test values (second test: p < 0.01; third test: p < 0.05, Fig. 28G). These results suggest that 1,1-DEE can enhance spatial learning and memory even in normal mice.

### Experimental example 6. Evaluation of the Effects of 1,1-DEE on Cancer

### 6-1. Experimental Methods

MCF7 breast cancer cells were used to evaluate changes in cell motility and mitochondrial function following 1,1-DEE treatment. Cells were patterned into 2D islands on collagen-coated polyacrylamide gels using PDMS stencils. A cell suspension at a concentration of 1.5 × 10⁶ cells/mL (200 µL per stencil) was dispensed onto each stencil and allowed to attach for 3 hours at 37°C in a humidified incubator containing 5% CO₂. After attachment, the PDMS stencils were removed, and the cells were washed with PBS. Culture medium containing 1,1-DEE at concentrations of 1mM, 10mM, or 50mM was then added. Cell motility was quantified using time-lapse imaging and MATLAB analysis. Mitochondrial function was assessed by TMRE fluorescent staining.

### 6-2. Results

As a result, in the cell motility analysis, no movement was observed in the 50mM treatment group due to cell death (Fig. 33), and quantitative analysis of cell motility also showed no significant movement in the 50mM treatment group (Fig. 34). Analysis of TMRE fluorescence signals revealed that mitochondrial function was impaired in the 50mM treatment group, indicating functional damage (Fig. 35). These results suggest that AMPK activation is closely involved in metabolic regulation and cellular function of cancer cells, and that 1,1-DEE can exhibit anticancer effects within an appropriate concentration range.
Ministry / Funding Organization: Chonnam Technopark
Research Program: Hwasun Vaccine Industrial Complex Intellectual Property Capacity Building Support Project
Project Title: IP-R&D
Host Organization: LuxAnima Co., Ltd.

As described above, specific aspects of the present invention have been described in detail. It will be apparent to those skilled in the art that such specific descriptions are merely preferred embodiments, and the scope of the present invention is not limited thereby. Accordingly, the actual scope of the present invention is defined by the appended claims and their equivalents.

## Claims

1. A mitochondrial biosynthesis promoter comprising 1,1-diethoxyethane (1,1-DEE) as an active ingredient.

2. The mitochondrial biosynthesis promoter according to claim 1,
wherein the 1,1-diethoxyethane promotes mitochondrial biosynthesis by increasing the expression of PGC-1α (peroxisome proliferator-activated receptor gamma coactivator-1 alpha).

3. The mitochondrial biosynthesis promoter according to claim 1,
wherein the 1,1-diethoxyethane promotes mitochondrial biosynthesis by enhancing the transcriptional activities of Nrf1 and Nrf2, which in turn stimulate the expression of Tfam.

4. The mitochondrial biosynthesis promoter according to claim 1,
wherein the 1,1-diethoxyethane promotes an aerobic respiration.

5. A pharmaceutical composition for the prevention or treatment of a disease requiring promotion of mitochondrial biosynthesis, comprising 1,1-diethoxyethane (1,1-DEE) as an active ingredient,
wherein the disease requiring promotion of mitochondrial biosynthesis is selected from the group consisting of ischemic brain diseases, ischemic heart diseases, pulmonary embolism, ischemic-reperfusion injury, stroke, ischemic stroke, migraine, anemia, Parkinson's disease (PD), dementia, Alzheimer's disease (AD), Huntington's disease, Frontotemporal Dementia, amyotrophic lateral sclerosis (ALS), Leigh syndrome, mitochondrial myopathy, mitochondrial encephalomyopathy, Lactic Acidosis, and Stroke-like episodes (MELAS), cirrhosis, Fanconi syndrome, atypical phenylketonuria, asthma, chronic obstructive pulmonary disease (COPD), diabetes, atherosclerosis, heart failure, hypertension, metabolic dysfunction, metabolic dysfunction-associated steatotic liver disease (MASLD), insulin resistance, hypercholesterolemia, optic nerve diseases, Duchenne muscular dystrophy, myopathies, obesity, depression, Osteoarthritis, , cancer, cancer cachexia, inflammatory diseases, age-related diseases, and cardiovascular diseases, cancer, cognitive ability or memory.

6. A pharmaceutical composition for the prevention or treatment of age-related diseases and for delaying aging or extending lifespan comprising 1,1-diethoxyethane (1,1-DEE) as an active ingredient.

7. A pharmaceutical composition for the prevention or treatment of cardiovascular diseases comprising 1,1-diethoxyethane (1,1-DEE) as an active ingredient.

8. A pharmaceutical composition for the prevention or treatment of cancer comprising 1,1-diethoxyethane (1,1-DEE) as an active ingredient.

9. A pharmaceutical composition for the prevention or treatment of stroke comprising 1,1-diethoxyethane (1,1-DEE) as an active ingredient.

10. A pharmaceutical composition for the prevention or treatment of decline and impairment of cognitive ability or memory_comprising 1,1-diethoxyethane (1,1-DEE) as an active ingredient.

11. A pharmaceutical composition for the prevention or treatment of dementia comprising 1,1-diethoxyethane (1,1-DEE) as an active ingredient.

12. A cosmetic composition for preventing or improving a disease requiring promotion of mitochondrial biosynthesis, comprising 1,1-diethoxyethane (1,1-DEE) as an active ingredient.

13. A food composition for preventing or improving a disease requiring promotion of mitochondrial biosynthesis, comprising 1,1-diethoxyethane (1,1-DEE) as an active ingredient.

14. A feed composition for preventing or improving a disease requiring promotion of mitochondrial biosynthesis, comprising 1,1-diethoxyethane (1,1-DEE) as an active ingredient.
